# EUROPEAN PATENT APPLICATION

(11) **EP 3 889 262 A1**
(43) Date of publication of application: **06.10.2021**
(21) Application number: 21165535.2
(22) Date of filing: 29.03.2021
(51) Int. Cl.: C12N 15/113

(54) **INHIBITION OF CASPASE-3 BY THE MICRORNA 126-5P**

(30) Priority: 27.03.2020 EP 20166120
(71) Applicant: Ludwig-Maximilians-Universität München, 80539 München (DE)
(72) Inventor: WEBER, Christian, 80337 München (DE); SANTOVITO, Donato, 85256 Vierkirchen (DE)
(74) Representative: Vossius & Partner Patentanwälte Rechtsanwälte mbB

(57) **Abstract**

The present invention relates to a nucleic acid molecule comprising or consisting of (a) the nucleic acid sequence of SEQ ID NO: 1, (b) a nucleic acid sequence being at least 80%, preferably at least 90% and most preferably at least 95% identical to SEQ ID NO: 1, (c) a fragment of the nucleic acid sequence of (a) or (b), wherein said fragment comprises at least 15 nucleotides, preferably at least 17 nucleotides and most preferably at least 19 nucleotides, (d) the nucleic acid sequence or any one of (a) to (c), wherein U is replaced by T, (e) a vector expressing the nucleic acid sequence of any one of (a) to (d), and/or (f) a host comprising the vector of (e) for use in the prevention and/or treatment of disease-related apoptosis.

## Description

The present invention relates to a nucleic acid molecule comprising or consisting of (a) the nucleic acid sequence of SEQ ID NO: 1, (b) a nucleic acid sequence being at least 80%, preferably at least 90% and most preferably at least 95% identical to SEQ ID NO: 1, (c) a fragment of the nucleic acid sequence of (a) or (b), wherein said fragment comprises at least 15 nucleotides, preferably at least 17 nucleotides and most preferably at least 19 nucleotides, (d) the nucleic acid sequence or any one of (a) to (c), wherein U is replaced by T, (e) a vector expressing the nucleic acid sequence of any one of (a) to (d), and/or (f) a host comprising the vector of (e) for use in the prevention and/or treatment of disease-related apoptosis.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Apoptosis is a process of programmed non-inflammatory cell death which is generally characterized by distinct morphological characteristics and energy-dependent biochemical mechanisms (Nagata, Ann Rev Immunol 2018; 36(1), 1-29). Apoptosis occurs following the activation of specific pathways and results in a series of well-defined morphological events. The apoptotic cell initially shows nuclear and cytoplasmic condensation, followed by blebbing of the plasma membrane that results in release of small extracellular vesicles containing cellular components known as apoptotic bodies. Apoptosis is mediated by a specific set of proteases, named caspases, that act in a coordinated pathway which culminate with the activation of caspase-3 and -7 which are responsible for an ordered disruption of the cell without leakage of cellular components and induction of inflammation. All caspases are synthesized as inactive zymogens that need activation which is generally achieved through cleavage of a pro-domain by other caspases and association of two pro-caspase monomers. Dimerization is a critical step for the formation of a proper active site required for the catalytic activity of caspases (MacKenzie and Clark, Adv Exp Med Bio 2012; 747:55-73.)

Apoptosis is considered a vital component of various processes including normal cell turnover, proper development and functioning of the immune system, hormone-dependent atrophy, embryonic development and chemical-induced cell death.

On the other hand, inappropriate apoptosis (either too little or too much) is a factor in many human conditions, including but not limited to neurodegenerative diseases, ischemic-reperfusion damage, autoimmune disorders and many types of cancer.

Therefore an ongoing need exits for developing new treatment option to control disease-related inappropriate apoptosis. This need is addressed by the present invention.

Accordingly the present invention relates is a first aspect to a nucleic acid molecule comprising or consisting of (a) the nucleic acid sequence of SEQ ID NO: 1, (b) a nucleic acid sequence being at least 80%, preferably at least 90% and most preferably at least 95% identical to SEQ ID NO: 1, (c) a fragment of the nucleic acid sequence of (a) or (b), wherein said fragment comprises at least 15 nucleotides, preferably at least 17 nucleotides and most preferably at least 19 nucleotides, (d) the nucleic acid sequence or any one of (a) to (c), wherein U is replaced by T, (e) a vector expressing the nucleic acid sequence of any one of (a) to (d), and/or (f) a host comprising the vector of (e) for use in the prevention and/or treatment of disease-related apoptosis.

In accordance with the present invention the term "nucleic acid molecule" defines a linear molecular chain. The specific nucleic acid molecule in accordance with the invention consists of at least 15 nucleotides. The term "nucleic acid molecule" is interchangeably used herein with the term "polynucleotide".

Nucleic acid molecules in accordance with the present invention include DNA and RNA. The nucleic acid molecules in accordance with the present invention typically have one strand of nucleotide bases; i.e they are single-stranded (ss). In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. "RNA" (ribonucleic acid) means any chain or sequence of the chemical building blocks adenine (A), guanine (G), cytosine (C) and uracil (U), called nucleotide bases, that are linked together on a ribose sugar backbone. Included are also single-stranded hybrid molecules, i.e., DNA-RNA hybrid molecules. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. The nucleic acid molecule of the invention may additionally comprise promoters, enhancers, response elements, signal sequences, polyadenylation sequences, introns, 5'- and 3'- non-coding regions, and the like.

Mature microRNAs (miRNAs) are a class of naturally occurring, small non-coding RNA molecules being generally about 21-25 nucleotides in length. MicroRNAs are partially complementary to one or more messenger RNA (mRNA) molecules, and their canonical function is to downregulate gene expression in a variety of manners, including translational repression, mRNA cleavage, and deadenylation.

MicroRNAs are transcribed by RNA polymerase II as large RNA precursors called pri-miRNAs and comprise of a 5' cap and poly-A tail. The pri-miRNAs are processed in the nucleus by a microprocessor complex, consisting of the RNase III enzyme Drosha, and the double-stranded-RNA-binding protein, Pasha/DGCR8. The resulting pre-miRNAs are aproximately 70-nucleotides in length and are folded into imperfect stem-loop structures. The pre-miRNAs are then exported into the cytoplasm by the karyopherin exportin 5 (Exp5) and Ran-GTP complex. Ran (RAS-related nuclear protein) is a small GTP binding protein belonging to the RAS superfamily that is essential for the translocation of RNA and proteins through the nuclear pore complex. The Ran-GTPase binds Exp5 and forms a nuclear heterotrimer with pre-miRNAs. Once in the cytoplasm, the pre-miRNAs undergo an additional processing step by the RNAse III enzyme Dicer generating the mature miRNA.

Thousands of miRNAs have since been identified in various organisms through random cloning and sequencing or computational prediction. The miRBase, hosted by the Sanger Institute provides miRNA nomenclature, sequence data, annotation and target prediction information.

This database comprises the human hsa-miR-126 (Accession No. MI0000471). Processing of the pre-miR-126 duplex by Dicer leads to the formation of the guide strand (miR-126-3p) and the passenger stand (miR-126-5p). The 5p strand is present in the forward (5'-3') position, while the 3p strand is located in the reverse position. SEQ ID NO: 1 is the mature sequence of the 5p strand (or passenger strand) of the human microRNA 126 (hsa-miR-126-5p, Accession No. MIMAT0000444). Hsa-miR-126-5p is single-stranded and comprises 21 nucleotides.

In accordance with the present invention, the term "percent (%) sequence identity" describes the number of matches ("hits") of identical nucleotides of two or more aligned nucleic acid sequences as compared to the number of nucleotides residues making up the overall length of the template nucleic acid sequences. In other terms, using an alignment, for two or more sequences or subsequences the percentage of amino acid residues or nucleotides that are the same (e.g. 80% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Alignments in connection with the present invention are preferably to be carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

As defined herein above, a nucleotide sequence identity of at least 80%, preferably at least 90% and most preferably at least 95% with SEQ ID NO: 1 is envisaged by the invention. Because SEQ ID NO: 1 consists of 21 nucleotides at least 80%, at least 90% and at least 95% allow for up to 4, up to 2 or 1 mutation(s) of SEQ ID NO: 1, respectively. Each mutation can be a deletion, addition or substitution of a nucleotide within SEQ ID NO: 1 and is preferably a substitution.

As defined herein above, also envisioned are fragments of SEQ ID NO: 1 comprising at least 15 nucleotides, preferably at least 17 nucleotides and most preferably at least 19 nucleotides. Likewise envisioned are fragments of SEQ ID NO: 1 comprising at least 16 nucleotides, preferably at least 18 nucleotides and most preferably at least 20 nucleotides. The at least 15, 16, 17, 18, 19 or 20 are preferably are at least 15, 16, 17, 18, 19 or 20 being identical to SEQ ID NO: 1 and more preferably at least 15, 16, 17, 18, 19 or 20 being identical to a contiguous stretch of at least 15, 16, 17, 18, 19 or 20 in SEQ ID NO: 1.

The nucleic acid molecule according to the invention may be a recombinantly produced or isolated from its natural source. The nucleic acid molecule according to the invention may be any precursor of SEQ ID NO: 1 or any fragment thereof as long as a sequence identity of at least 80% over the entire length of the miRNA of SEQ ID NO: 1 is maintained. Also orthologous or homologous sequences of the miRNA selected from different species including precursor or a functional fragment thereof may be used, wherein these orthologous or homologous sequences also preferably share a sequence identity of at least 80% with the miRNA of SEQ ID NO: 1.

The vector according to the invention is an expression vector encoding the nucleic acid molecule of (a) to (c) in expressible form. An expression vector may be a plasmid that is used to introduce a specific transcript into a target cell. Once the expression vector is inside the cell, the transcript that is encoded by the vector is produced by the cellular-transcription machinery. The plasmid is in general engineered to contain regulatory sequences that act as enhancer and/or promoter regions and lead to efficient transcription of the transcript.

Non-limiting examples of expression vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen). For the formulation of a pharmaceutical composition as will be described herein below a suitable vector is selected in accordance with good manufacturing practice. Such vectors are known in the art, for example, from Ausubel et al, Hum Gene Ther. 2011 Apr; 22(4):489-97 or Allay et al., Hum Gene Ther. May 2011; 22(5): 595-604.

A typical mammalian expression vector contains the promoter element, which mediates the initiation of transcription of RNA, the transcript sequence, and signals required for the termination of transcription and optionally polyadenylation of the transcript. Moreover, elements such as origin of replication, drug resistance gene, regulators (as part of an inducible promoter) may also be included. The lac promoter is a typical inducible promoter, useful for prokaryotic cells, which can be induced using the lactose analogue isopropylthiol-b-D-galactoside. ("IPTG"). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Alternatively, the recombinant transcript can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded transcript. The DHFR (dihydrofolate reductase) marker is useful to develop cell lines that carry several hundred or even several thousand copies of the gene of interest. Another useful selection marker is the enzyme glutamine synthase (GS) (Murphy et al. 1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E*. *coli* and other bacteria. For vector modification techniques, see Sambrook and Russel (2001), Molecular Cloning: A Laboratory Manual, 3 Vol. Generally, vectors can contain one or more origins of replication (ori) and inheritance systems for cloning or expression, one or more markers for selection in the host, e.g., antibiotic resistance, and one or more expression cassettes. Suitable origins of replication (ori) include, for example, the Col E1, the SV40 viral and the M 13 origins of replication.

As mentioned, the transcript sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the nucleotide sequence as defined in item (a) of the above preferred embodiment of the invention is operatively linked to such expression control sequences allowing expression in prokaryotic or eukaryotic cells.

The host may be a prokaryotic or eukaryotic cell. A suitable eukaryotic host may be a mammalian cell (preferably human), an amphibian cell, a fish cell, an insect cell, a fungal cell or a plant cell. Representative examples of bacterial cells are *E*. *coli, Streptomyces* and *Salmonella typhimurium* cells; of fungal cells are yeast cells; and of insect cells are Drosophila 9 and Spodoptera Sf9 cells. It is preferred that the cell is a mammalian cell such as a human cell. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. The cell may be a part of a cell line, preferably a human cell line. Appropriate culture mediums and conditions for the above-described host cells are known in the art. The host is preferably a host cell and more preferably an isolated host cell. The host is also preferably a non-human host.

The nucleic acid molecule in accordance with the present invention is preferably formulated as a pharmaceutical composition. In accordance with the present invention, the term "pharmaceutical composition" relates to a composition for administration to a patient, preferably a human patient.

The pharmaceutical composition of the invention comprises the nucleic acid molecule in accordance with the present invention recited above. It may, optionally, comprise further molecules capable of altering the characteristics of the nucleic acid molecule in accordance with the present invention thereby, for example, stabilizing, modulating and/or activating their function. The composition may be in solid, liquid or gaseous form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier or excipient. By "pharmaceutically acceptable carrier or excipient" is meant a non-toxic solid, semisolid or liquid filler, diluent, encapsulating material or formulation auxiliary of any type (see also Handbook of Pharmaceutical Excipients 6ed. 2010, Published by the Pharmaceutical Press). Examples of suitable pharmaceutical carriers and excipients are well known in the art and include phosphate buffered saline solutions, water, emulsions, such as oil/water emulsions, various types of wetting agents, sterile solutions, organic solvents including DMSO etc. Compositions comprising such carriers or excipients can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.5 mg to 50 mg and most preferably 0.5 mg to 10 mg per day.

Furthermore, said compound being an nucleic acid sequence, such as an miRNA, the total pharmaceutically effective amount of pharmaceutical composition administered will typically be less than about 75 mg per kg of body weight, such as for example less than about 70, 60, 50, 40, 30, 20, 10, 5, 2, 1, 0.5, 0.1, 0.05, 0.01 mg per kg of body weight. More preferably, the amount will be less than 2000 nmol of nucleic acid sequence (e.g., about 4.4 x 10¹⁶ copies) per kg of body weight, such as for example less than 1500, 750, 300, 150, 75, 15, 7.5, 1.5, 0.75, 0.15, 0.075, 0.015, 0.0075 nmol of miRNA agent per kg of body weight.

The length of treatment needed to observe changes and the interval following treatment for responses to occur vary. The particular amounts may be determined by conventional tests which are well known to the person skilled in the art.

The pharmaceutical composition may be administered, for example, orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable carriers or excipients as described herein above.

The nucleic acid molecule according to the invention may be formulated as vesicles, such as liposomes. Liposomes have attracted great interest because of their specificity and the duration of action they offer from the standpoint of drug delivery. Liposomal delivery systems have been used to effectively deliver nucleic acids, such as miRNA *in vivo* into cells (Zimmermann et al. (2006), Nature, 441:111-114). Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are phagocytosed by macrophages and other cells in vivo.

The subject to be treated in accordance with the invention is a mammal, preferably human.

The fragments of SEQ ID NO: 1 as well as the nucleotide sequences with at least 80%, preferably at least 90% and most preferably at least 95% identity to SEQ ID NO: 1 according to the present invention retain the activity of SEQ ID NO: 1 to bind to caspase-3 in an endothelial cell, thereby being capable of inhibiting apoptosis of the endothelial cell.

Endothelial cells form a one-cell thick walled layer called endothelium that lines all of human blood vessels such as arteries, arterioles, venules, veins and capillaries. Smooth muscle cells layer beneath the endothelial cells. The exception to this is the capillaries where endothelium makes up the entire blood vessel wall. The primary function of endothelium is the maintenance of vessel wall permeability and regulating blood flow.

The endothelium acts as a barrier between the blood and the rest of the body tissue while being selectively permeable for certain chemicals and white blood cells to move across from blood to tissue or for waste and carbon-dioxide to move from tissue to blood. This property of endothelial cells is especially investigated in the blood-brain-barrier system. Moreover, endothelial cells generate an anti-thrombotic surface that facilitates transit of plasma and cellular constituents throughout the vasculature. The endothelium is also responsible for maintaining homeostasis and formation of new blood vessels (process referred to as angiogenesis). Endothelial cells are also active participants in and regulators of the inflammatory processes.

As discussed, apoptosis is a form of cell death in which a programmed sequence of events leads to the elimination of cells generally without releasing harmful substances into the surrounding area. When apoptosis works overly well, too many cells are killed which, in turn, may cause severe tissue damage. This scenario is referred to herein a "disease-related apoptosis". Disease-related apoptosis occurs, for example, in diseases such as strokes and neurodegenerative disorders such as Alzheimer's, Huntington's, and Parkinson's diseases.

Apoptosis can be initiated through one of two pathways. In the intrinsic pathway the cell kills itself because it senses cell stress, while in the extrinsic pathway the cell kills itself because of signals from other cells. Weak external signals may also activate the intrinsic pathway of apoptosis. Both pathways induce cell death by activating caspases, which are enzymes that degrade proteins. The two pathways both activate initiator caspases, which activate executioner caspases, which then kill the cell by degrading proteins indiscriminately. Caspases are proteins that are highly conserved, cysteine-dependent aspartate-specific proteases. Caspase 2, 8, 9, 10, 11, and 12 are initiator caspases, whereas caspase 3, 6, and 7 are effector caspases.

Human caspase-3 (also known as CPP32/Yama/apopain) is encoded by the CASP3 gene. CASP3 orthologs have been identified in numerous mammals. This protein cleaves and activates caspases 6 and 7; and the protein itself is processed and activated by caspases 8, 9, and 10. It is the predominant caspase involved in the cleavage of amyloid-beta 4A precursor protein, which is associated with neuronal death in Alzheimer's disease.

Caspase-3 is formed from a 32 kDa zymogen (pro-caspase-3) that is cleaved into 17 kDa and 12 kDa subunits. When the procaspase is cleaved at a particular residue, the active heterotetramer can then be formed by hydrophobic interactions, causing four anti-parallel beta-sheets from p17 and two from p12 to come together to make a heterodimer, which in turn interacts with another heterodimer to form the full 12-stranded beta-sheet structure surrounded by alpha-helices that is unique to caspases. When the heterodimers align head-to-tail with each other, an active site is positioned at each end of the molecule formed by residues from both participating subunits, though the necessary Cys-163 and His-121 residues are found on the p17 (larger) subunit.

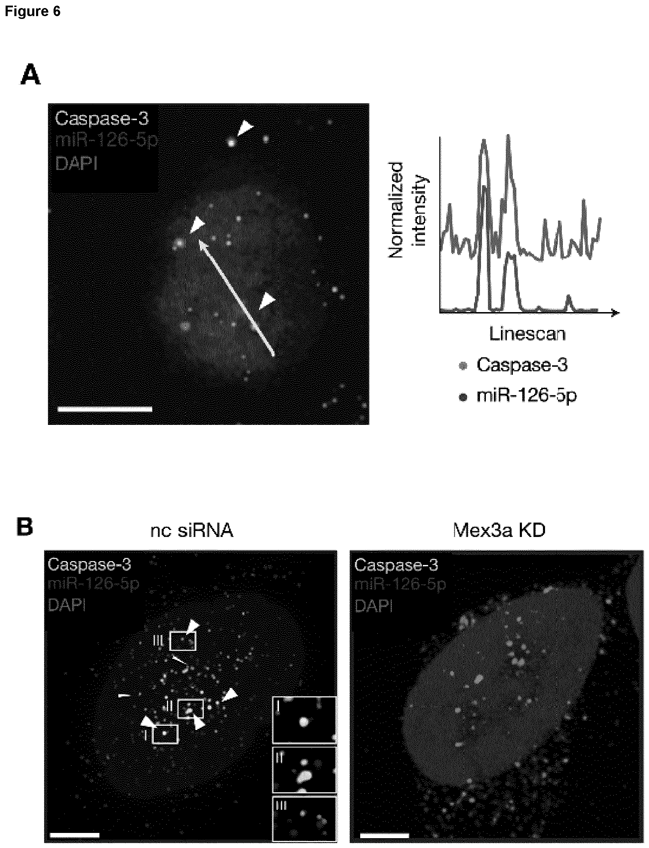

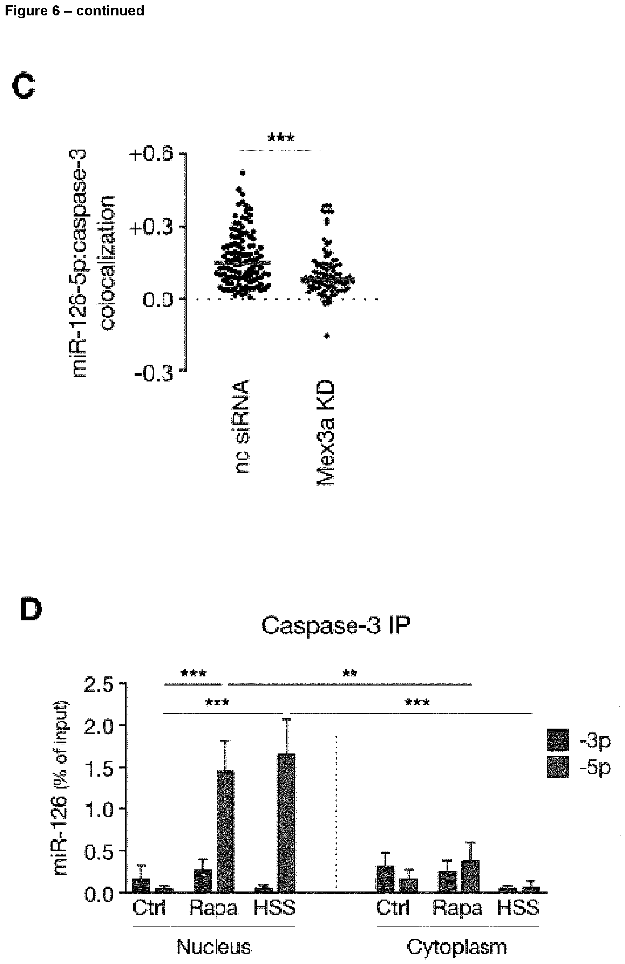

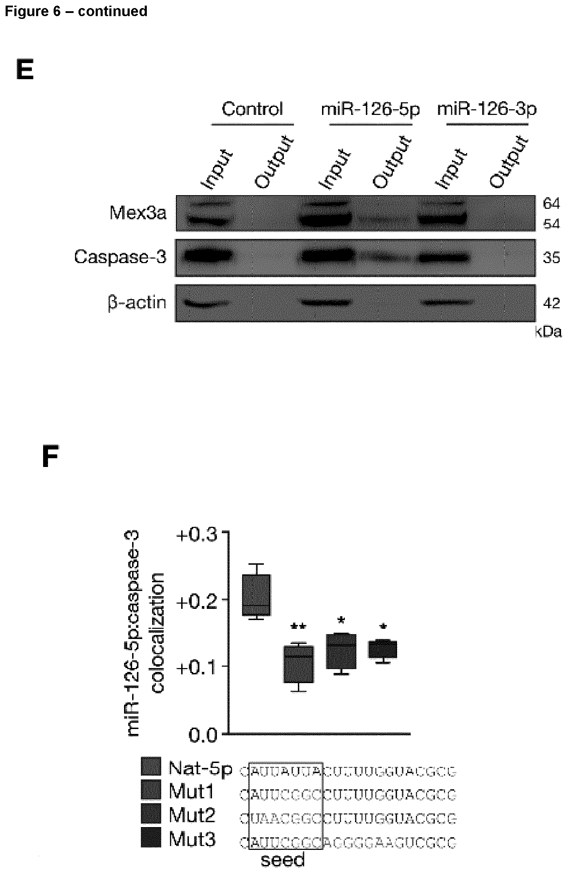

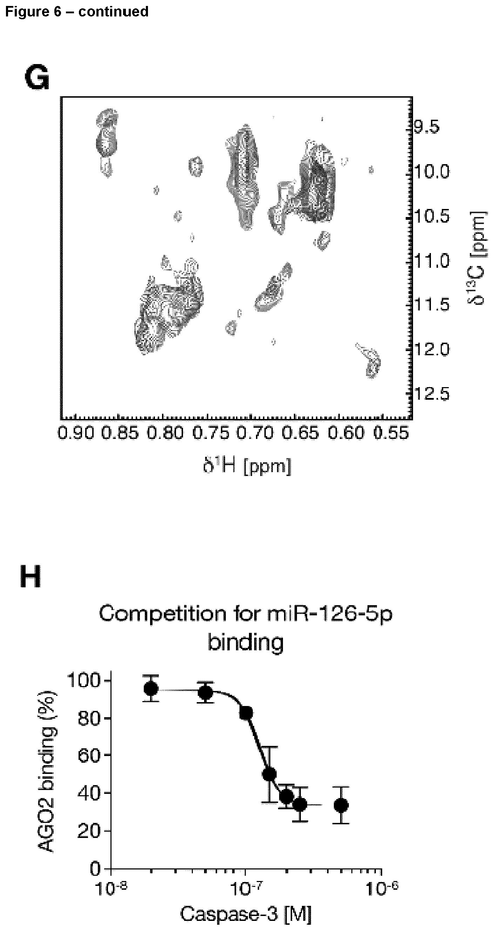

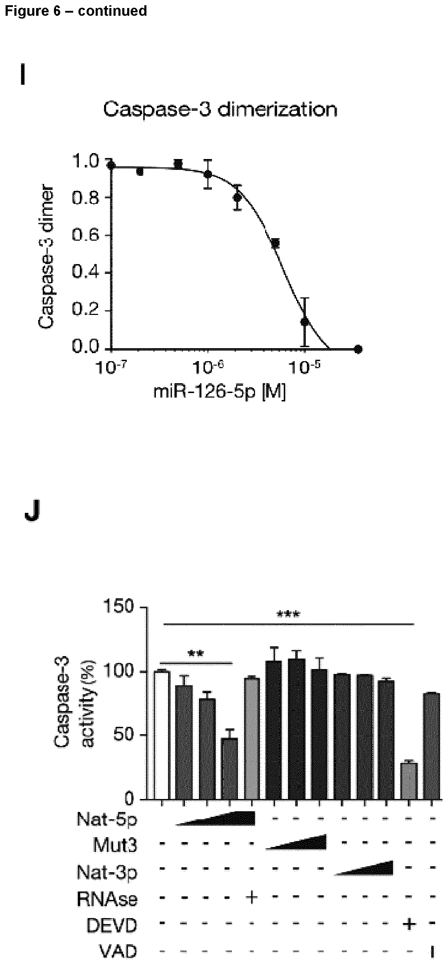

As can be taken from the appended examples, in connection with the present invention it was surprisingly found that mR-126-5p is capable of binding to caspase-3 protein within endothelial cells thereby preventing the formation of the discussed heterodimer in the endothelial cells. Hence, it was found that miR126-5p is capable of acting as an aptamer or in an aptamer-like way, since it binds to the specific target molecule caspase-3. In more detail, it is shown that miR-126-5p sustains endothelial integrity in the context of high shear stress and autophagy. Bound to Ago2, miR-126-5p forms a complex with Mex3a, which occurs on the surface of autophagic vesicles and guides its transport into the nucleus. Mutational studies and biophysical measurements demonstrate that Mex3a binds to the central U- and G-rich regions of miR-126-5p with nanomolar affinity via its two KH domains. In the nucleus, miR-126-5p dissociates from Ago2 and binds to caspase-3 in an aptamer-like fashion with its seed sequence. By this binding mR126-5p inhibits the protein cleavage activity of caspase-3 thereby stopping the apoptotic pathway. The downstream activation of caspases-6 and 7 is blocked. It was in particular surprisingly found that only miR126-5p prevents apoptosis while the duplex of miR126 and well as miR126-3p are not capable of preventing apoptosis. In the examples herein below a direct binding of miR126-5p to caspase-3 is demonstrated (Fig. 6).

In this connection it is of note that the remarkable abundance of miR-126-5p in endothelial cells compared to other cell types strongly indicates that miR-126-5p is a regulator of apoptosis only or at least very preferentially in endothelial cells. The anti-apoptotic effect of miR-126-5p outside of the RISC is important for endothelial integrity under conditions of high shear stress promoting autophagy: ablation of Mex3a or ATG5 *in vivo* attenuates nuclear import of miR-126-5p, aggravates endothelial apoptosis and exacerbates atherosclerosis. This is underlined by the findings of Wang et al. (2017), Oncology Research, 25:463-470, wherein it is reported that miR126-5p promotes cell apoptosis of cervical cancer by canonical targeting of Bcl2l2 transcript. The examples herein below show that the mode of action of miR126-5p in endothelial cells is rather the contrary as in cervical cancer cells. In endothelial cells, miR-126-5p inhibits apoptosis by targeting caspase-3.

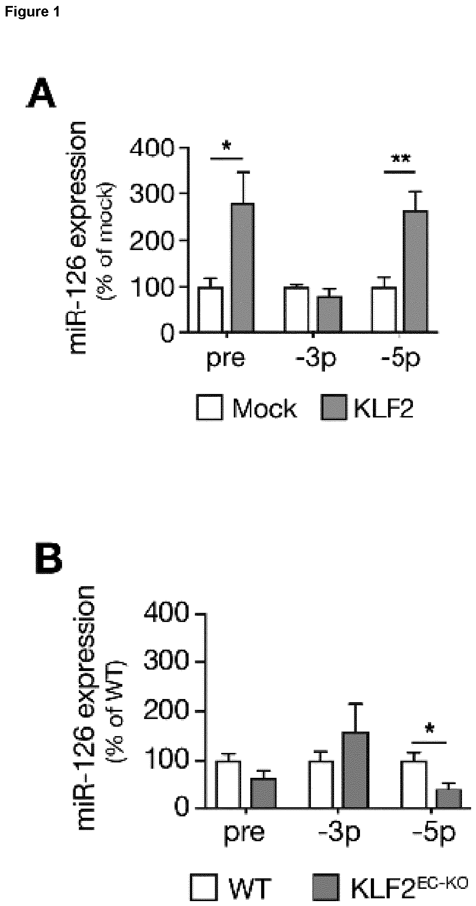

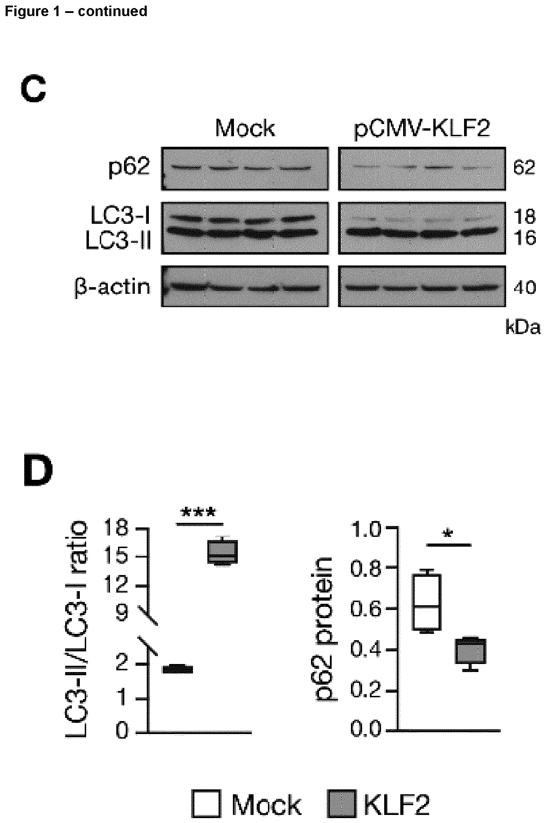

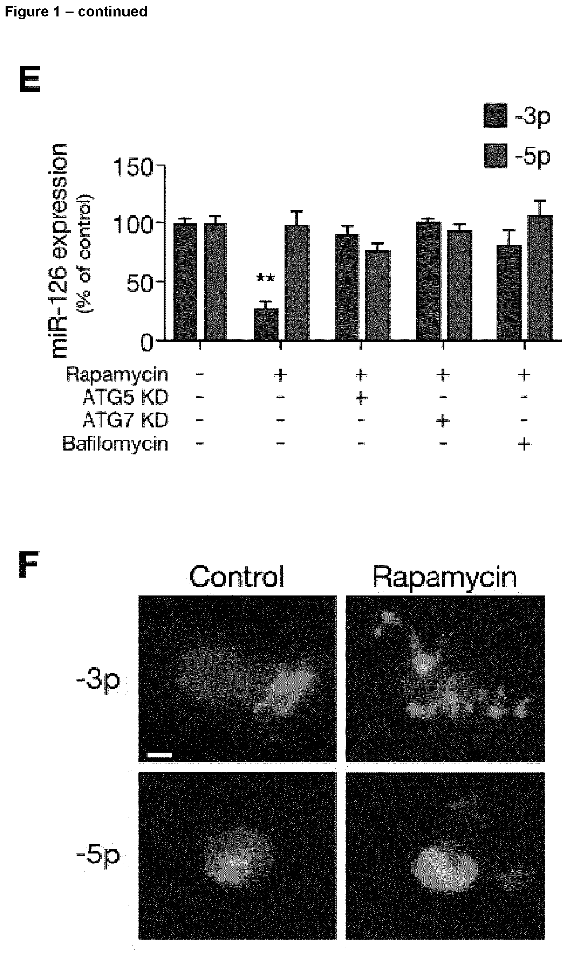

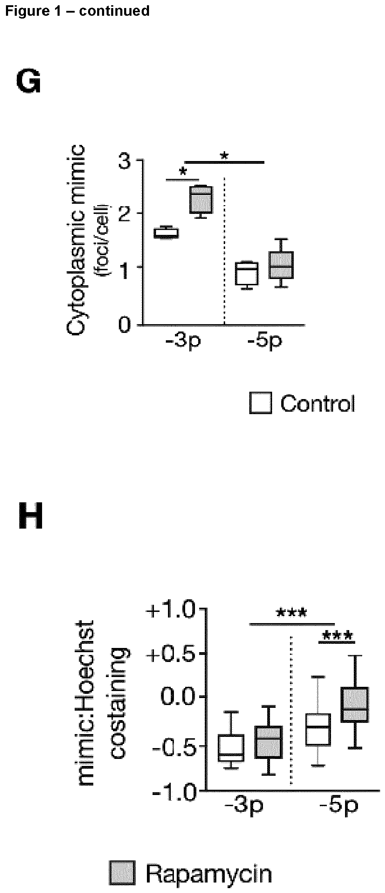

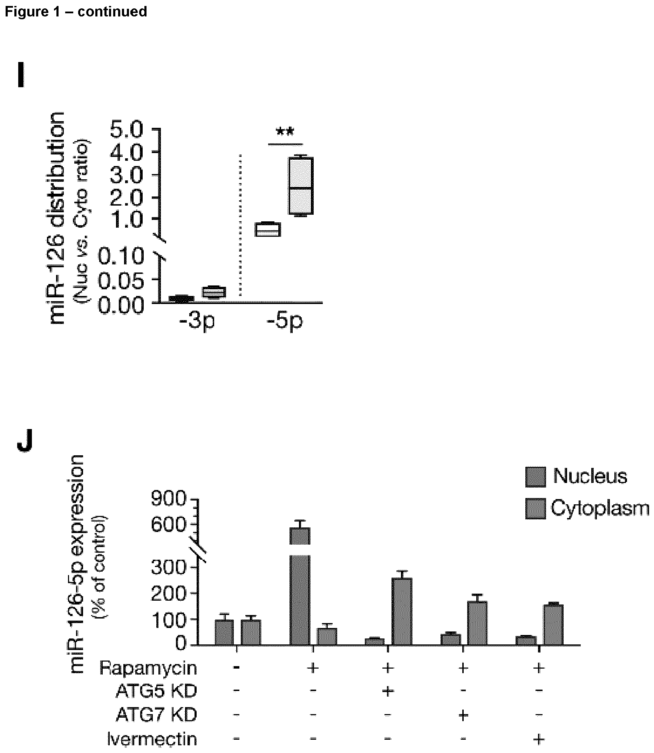

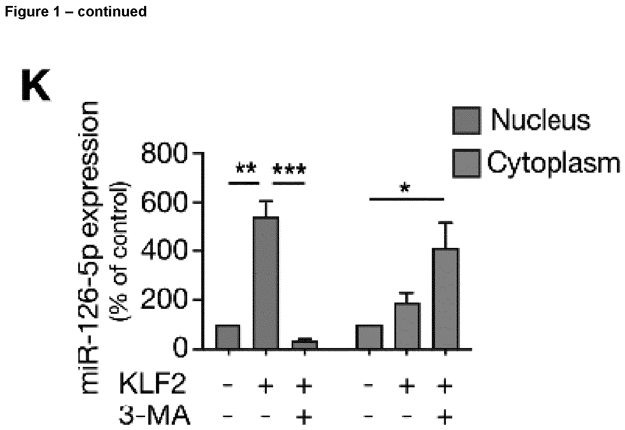

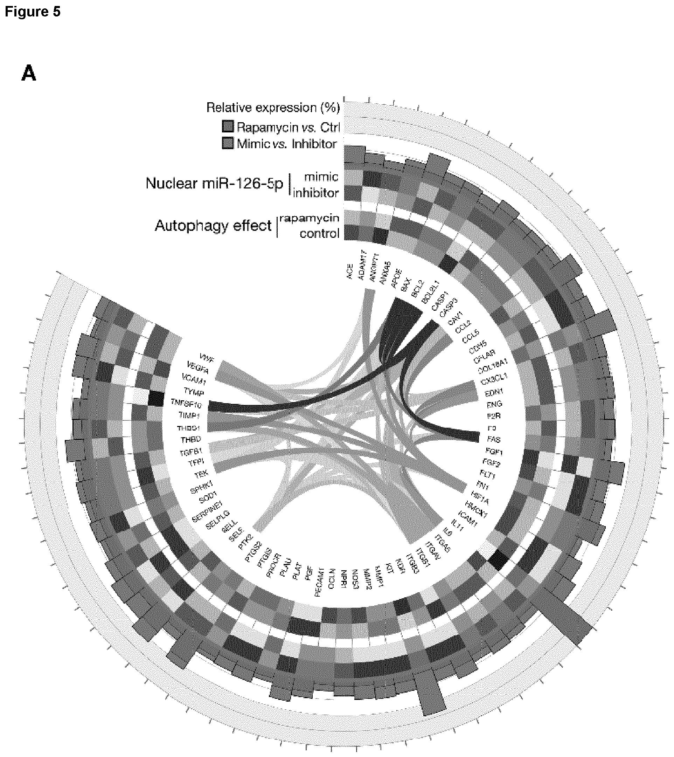

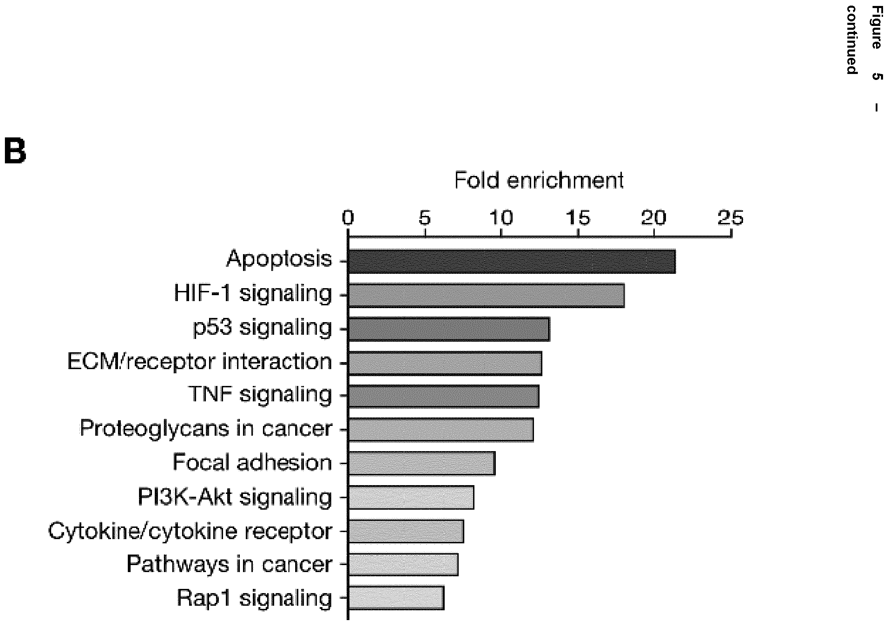

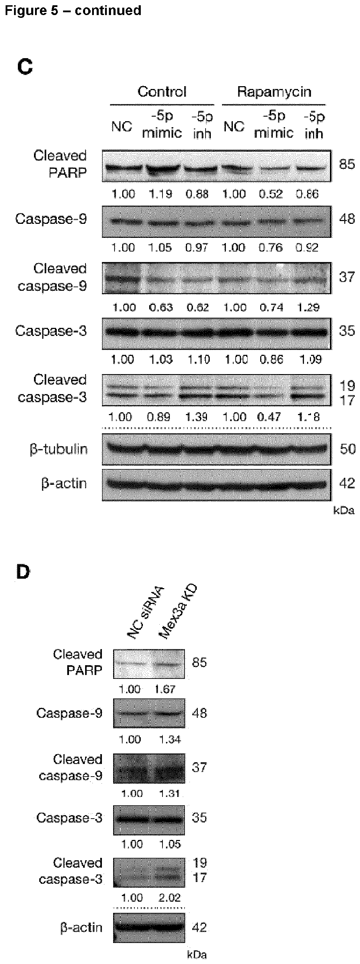

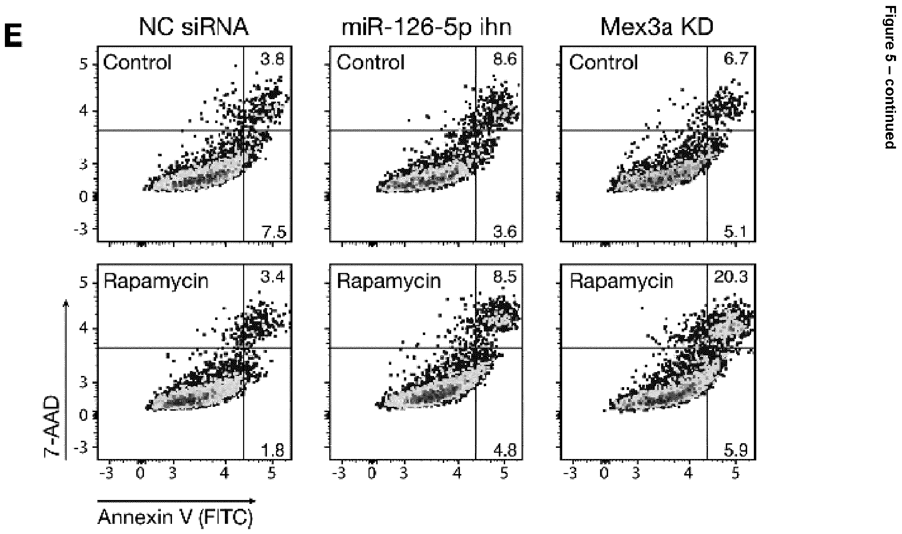

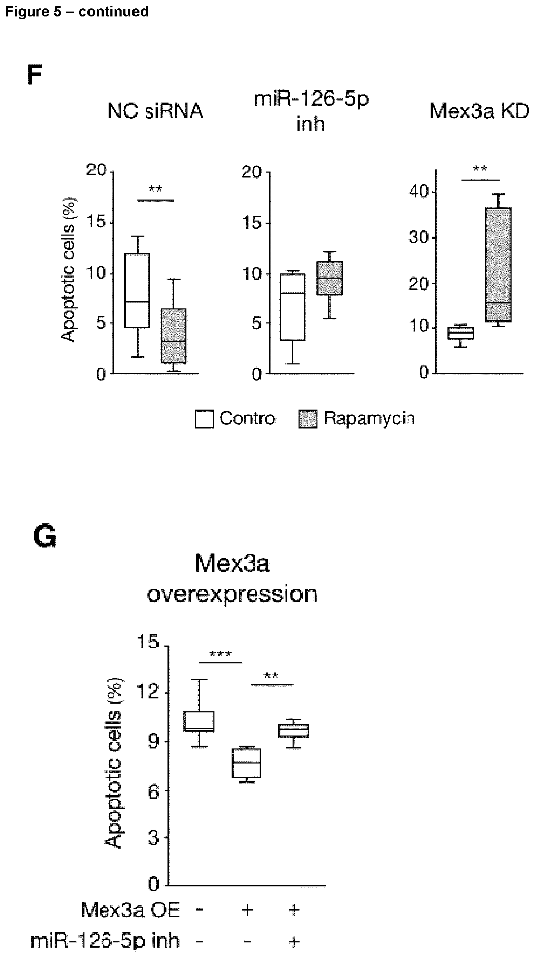

It is furthermore reported in Li et al. (2019), European Review for Medical and Pharmacological Sciences 23(3 Suppl):327-333 that miR-126 promotes endothelial apoptosis by targeting PIK23/Akt. As can be taken from Figure 5 of Li et al. (2019) here miR-126-3p and not miR-126-5p was under investigation. The *in vivo* bearing of the findings being described in Li et al. (2019) is questionable since the expression of miR-126-3p was raised (see Results section of the abstract) whereas its is shown in the examples that naturally only the expression of miR-126-5p but not of miR-126-3p is increased in endothelial cells (Fig. 1). In addition, the finding in miR126-3p that promotes apoptosis rather teaches away from the finding that miR-126-5p prevents apoptosis in endothelial cells.

Finally, it is of note that it is reported in Schober at al. (2014), Nat. Med., 20(4):368-376 that miR-126-5p promotes the proliferation of endothelial cells. This technical effect of miR-126-5p is distinct from the finding being demonstrated in the appended examples that miR-126-5p prevents the apoptosis of endothelial cells.

The findings in the appended examples have therefore unexpectedly revealed that miR-126-5p is furthermore helpful to treat disease conditions in a patient, wherein the prevention or stop of apoptosis can provide a curative effect. For instance, in human atherosclerotic plaques reduced nuclear miR-126-5p and concomitant increased active caspase-3 is shown in the examples in areas of disturbed flow where the disease develops. The direct inhibition of caspase-3 by nuclear miR-126-5p reveals a non-canonical mechanism, by disease-related apotosis e.g. in atherosclerosis can be treated or prevented.

In accordance with a preferred embodiment of the first aspect of the invention the vector expresses the nucleic acid sequence of any one of (a) to (d) under the control of an endothelial cell-specific promoter.

Within the vector the nucleic acid sequence encoding the nucleic acid sequence of any one of (a) to (d) is functionally linked to the endothelial cell-specific promoter, such that the endothelial cell-specific promoter controls the expression of the nucleic acid sequence of any one of (a) to (d).

Examples of endothelial cell-specific promoters are known in the art and are described, for example, in Wermuth et al., Laboratory Investigation (2017), 97:806-818 or Dai et al., J Virol. (2004), 78(12): 6209-6221. They include, but are not limited to pCdh5, pTie2 and pBMX promoters.

In accordance with a further preferred embodiment of the first aspect of the invention the vector is an adeno-associated vector (AAV).

Adeno-associated virus (AAV) is a non-enveloped virus that can be engineered to deliver DNA to target cells, and has attracted a significant amount of attention in the field, especially in clinical-stage experimental therapeutic strategies. The ability to generate recombinant AAV particles lacking any viral genes and containing DNA sequences of interest for various therapeutic applications has thus far proven to be one of the safest strategies for gene therapies (for review, Naso et al. (2017), BioDrugs; 31(4): 317-334.).

In accordance with a more preferred embodiment of the first aspect of the invention the AVV is AVV9, AVV1 or AAV5.

AAV1 and AAAV5 are reported in Chen et al. (2005), Hum Gene Ther.; 16(2):235-47 to efficiently transduce endothelial cells. This has also been found for AVV9 by Bostick et al., Gen Ther 2007, 14(22):1605-9.

Moreover, Varadi et al. Gen Ther (2012), 19(8):800-9 show that the modification of AAV9 by displaying on their surface members of a peptide library may further increase the specificity of AAV9 for endothelial cells. Hence, the AAV vectors and in particular AAV9 as described herein also encompass modified versions thereof that display a member of a peptide library on the virus capsid. Most preferably the displayed peptide is S^{L}/ₗRSPPS (SEQ ID NO: 4) and/or NLHSPPA (SEQ ID NO: 5).

In accordance with an even more preferred embodiment the AAV is AAV9.

AAV9 has a preference for primary cell binding through galactose as a result of unique amino acid differences in its capsid sequence. It has been postulated that this preferential galactose binding could confer to AAV9 the unique ability to cross the blood-brain barrier (BBB) and infect cells of the CNS, including primary neurons. AAV9 has also been shown to be very effective at delivering genes to endothelial cells.

In accordance with a preferred embodiment of the first aspect of the invention the disease is a disease with involvement of macro- or microvascular endothelial apoptosis, and is preferably selected from the group consisting of atherosclerosis, allograph vasculopathy, pulmonary hypertension, ischemia-reperfusion injury, congestive heart failure, sepsis, microvascular complications of diabetes, systemic sclerosis, inflammatory vasculitis, and thrombotic thrombocytopenic purpura.

Endothelial cells can be distinguished into microvascular and macrovascular endothelial cells. Microvascular endothelial cells are found in small blood vessles while macrovascular endothelial cells are found in small blood vessels. Human microvascular endothelial cells differ from macrovascular endothelial cells, for example, in their expression of matrix metalloproteinases (Jackson and Nguyen, Int J Biochem Cell Biol. 1997 Oct; 29(10):1167-77).

Accordingly, a macrovascular disease is a disease affecting any large (macro) blood vessels in the body whereas a macrovascular disease is a disease affecting any small (micro) blood vessels. For instance, a coronary microvascular disease is a heart disease that affects the walls and inner lining of tiny coronary artery blood vessels that branch off from the larger coronary arteries.

The diseases with the above-recited group of diseases are known to be associated with endothelial cell apoptosis. In this respect reference the scientific articles refrred to below:

### Atherosclerosis

Tricot O, Mallat Z, Heymes C, Belmin J, Leseche G, Tedgui A. Relation between endothelial cell apoptosis and blood flow direction in human atherosclerotic plaques. Circulation 2000; 101: 2450-3.

### Allograph vasculopathy

Borghi-Scoazec G, Scoazec JY, Durand F, Bernuau J, Belghiti J, Feldmann G, Henin D, Degott C. Apoptosis after ischemia-reperfusion in human liver allografts. Liver Transpl Surg 1997; 3: 407-15.
Panizo-Santos A, Lozano MD, Distefano S, Inoges S, Pardo J. Clinico-pathologic, immunohistochemical, and TUNEL study in early cardiac allograft failure. Cardiovasc Pathol 2000; 9: 153-9.
Hall AV, Jevnikar AM. Significance of endothelial cell survival programs for renal transplantation. Am J Kidney Dis 2003; 41: 1140-54.

### Pulmonary Hypertension

White K, Dempsie Y, Caruso P, Wallace E, McDonald RA, Stevens H, Hatley ME, Van Rooij E, Morrell NW, MacLean MR, Baker AH. Endothelial apoptosis in pulmonary hypertension is controlled by a microRNA/programmed cell death 4/caspase-3axis. Hypertension. 2014; 64:185-94.

### Ischemia-reperfusion injury

Matsushita H, Morishita R, Nata T, Aoki M, Nakagami H, Taniyama Y, Yamamoto K, Higaki J, Yasufumi K, Ogihara T. Hypoxia-induced endothelial apoptosis through nuclear factor-kappaB (NF-kappaB)-mediated bcl-2 suppression: in vivo evidence of the importance of NF-kappaB in endothelial cell regulation. Circ Res 2000; 86: 974-81.
Scarabelli T, Stephanou A, Rayment N, Pasini E, Comini L, Curello S, Ferrari R, Knight R, Latchman D. Apoptosis of endothelial cells precedes myocyte cell apoptosis in ischemia/reperfusion injury. Circulation 2001; 104: 253-6.
Scarabelli TM, Stephanou A, Pasini E, Comini L, Raddino R, Knight RA, Latchman DS. Different signaling pathways induce apoptosis in endothelial cells and cardiac myocytes during ischemia/reperfusion injury. Circ Res 2002; 90: 745-8.
Cheng T, Liu D, Griffin JH, Fernandez JA, Castellino F, Rosen ED, Fukudome K, Zlokovic BV. Activated protein C blocks p53-mediated apoptosis in ischemic human brain endothelium and is neuroprotective. Nat Med 2003; 9: 338-42.

### Congestive Heart Failure

Rossig L, Haendeler J, Mallat Z, Hugel B, Freyssinet JM, Tedgui A, Dimmeler S, Zeiher AM. Congestive heart failure induces endothelial cell apoptosis: protective role of carvedilol. J Am Coll Cardiol 2000; 36: 2081-9.
Rossig L, Hoffmann J, Hugel B, Mallat Z, Haase A, Freyssinet JM, Tedgui A, Aicher A, Zeiher AM, Dimmeler S. Vitamin C inhibits endothelial cell apoptosis in congestive heart failure. Circulation 2001; 104: 2182-7.

### Sepsis

Haimovitz-Friedman A, Cordon-Cardo C, Bayoumy S, Garzotto M, McLoughlin M, Gallily R, Edwards CK Jr, Schuchman EH, Fuks Z, Kolesnick R. Lipopolysaccharide induces disseminated endothelial apoptosis requiring ceramide generation. J Exp Med 1997; 186: 1831-41. Kawasaki M, Kuwano K, Hagimoto N, Matsuba T, Kunitake R, Tanaka T, Maeyama T, Hara N. Protection from lethal apoptosis in lipopolysaccharide-induced acute lung injury in mice by a caspase inhibitor. Am J Pathol 2000; 157: 597-603.

### Emphysema

Kasahara Y, Tuder RM, Cool CD, Lynch DA, Flores SC, Voelkel NF. Endothelial cell death and decreased expression of vascular endothelial growth factor and vascular endothelial growth factor receptor 2 in emphysema. Am J Respir Crit Care Med 2001; 163: 737-44.

### Microvascular complication of diabetes: Diabetic retinopathy

Mizutani M, Kern TS, Lorenzi M. Accelerated death of retinal microvascular cells in human and experimental diabetic retinopathy. J Clin Invest 1996; 97: 2883-90.

### Systemic sclerosis

Sgonc R, Gruschwitz MS, Dietrich H, Recheis H, Gershwin ME, Wick G. Endothelial cell apoptosis is a primary pathogenetic event underlying skin lesions in avian and human scleroderma. J Clin Invest 1996; 98: 785-92.
Maehara T, Kaneko N, Perugino CA, Mattoo H, Kers J, Allard-Chamard H, Mahajan VS, Liu H, Murphy SJ, Ghebremichael M, Fox DA, Payne AS, Lafyatis R, Stone JH, Khanna D, Pillai S. Cytotoxic CD4+ T lymphocytes may induce endothelial cell apoptosis in systemic sclerosis. J Clin Invest. 2020;131700.

### Allograph or microvascular vasculopathies: Sickle Cell Disease. Systemic lupus erythmatosis (SLE) and Thrombotic thrombocytopenic purpura (TPP)

Solovey A, Lin Y, Browne P, Choong S, Wayner E, Hebbel RP. Circulating activated endothelial cells in sickle cell anemia. N Engl J Med 1997; 337: 1584-90.
Rajagopalan S, Somers EC, Brook RD, Kehrer C, Pfenninger D, Lewis E, Chakrabarti A, Richardson BC, Shelden E, McCune WJ, Kaplan MJ. Endothelial cell apoptosis in systemic lupus erythematosus: a common pathway for abnormal vascular function and thrombosis propensity. Blood 2004; 103: 3677-83.
Dang CT, Magid MS, Weksler B, Chadburn A, Laurence J. Enhanced endothelial cell apoptosis in splenic tissues of patients with thrombotic thrombocytopenic purpura. Blood 1999; 93: 1264-70.

In accordance with another preferred embodiment of the first aspect of the invention the nucleic acid molecule is formulated for delivery to the cell nucleus.

As discussed herein above, miRNA126 is initially expressed as a duplex. This duplex is enzymatically processed into the single strands miR-126-5p and miR-126-3p. miR-126-5p then enters the nucleus of the endothelial cells, wherein it prevents the apoptosis of endothelial cells by binding to caspase-3.

The nuclear enrichment can be enhanced by formulating the nucleic acid molecule according to the invention for delivery to the cell nucleus. Examples of such formulation will be provided herein below.

In accordance with a further preferred embodiment of the first aspect of the invention the nucleic acid molecule is (i) complexed with a nuclear localization signal (NLS) or a (poly)peptide comprising a NLS, wherein the NLS preferably comprises or consists of PKKKRKV (SEQ ID NO: 6) or KKKX₅₋₂₀RK (SEQ ID NO: 7, wherin X can be any amino acid), (ii) complexed with a polymer, wherein the polymer preferably comprises of consists of prepolyamidoamine (PAMAM) dendrimers or polyethyleneimine (PEI), (iii) comprised in a nanoparticle, preferably a PEGylated-polylysine/lipid/DNA nanoparticle, (iv) complexed with a transcription factor or a (poly)peptide comprising transcription factor-binding site, wherein the transcription factor is preferably an endothelial cell-specific transcription factor, (v) complexed with a transcription factor-binding site or a (poly)peptide comprising transcription factor-binding site, wherein the transcription factor-binding site is preferably an endothelial cell-specific transcription factor-binding site, (vi) complexed with a small molecule ligand of importin, wherein the small molecule ligand is preferably biotin-streptavidin conjugate, and/or (vii) formulated together with a small molecule that reversibly disrupts the nuclear pore complex, wherein the small molecule is preferably trans-cyclohexane-1,2-diol.

Items (ii) to (viii) define art-established formulations for the nuclear targeting of the nucleic acid molecule according to the invention (Lam and Dean, Gene Ther. 2010 Apr; 17(4): 439-447). Hence, these formulations will facilitate the transport of the nucleic acid molecule according to the invention to the nucleus of endothelial cells, wherein it can bind to caspase-3 thereby preventing apoptosis of the endothelial cells.

In accordance with a still further preferred embodiment of the first aspect of the invention in addition (i) a polypeptide comprising or consisting of the amino acid sequence of SEQ ID NO: 2 or an amino acid sequence being at least 80% identical thereto, or (ii) a second nucleic acid molecule expressing the polypeptide of (i) is used in the prevention and/or treatment of disease-related apoptosis.

SEQ ID NO: 2 is the amino acid sequence of the human Mex3a protein. Mex3a is an RNA binding protein shuttling between the cytoplasm and the nucleus and is believed to be involved in post-transcriptional regulatory mechanisms.

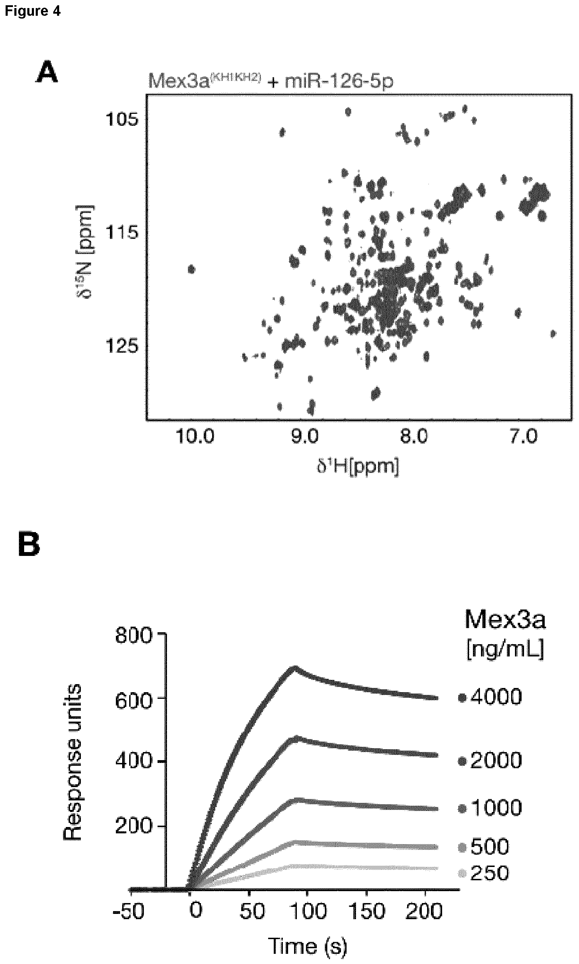

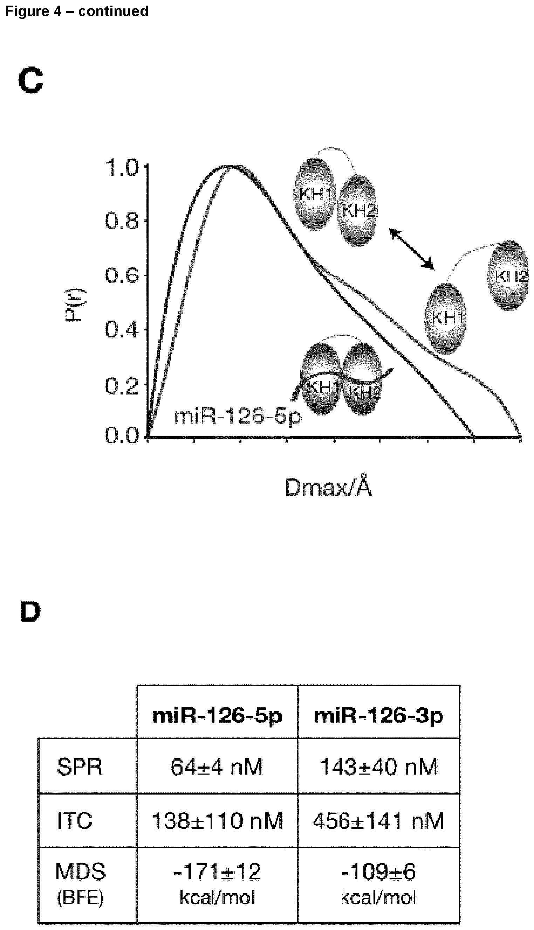

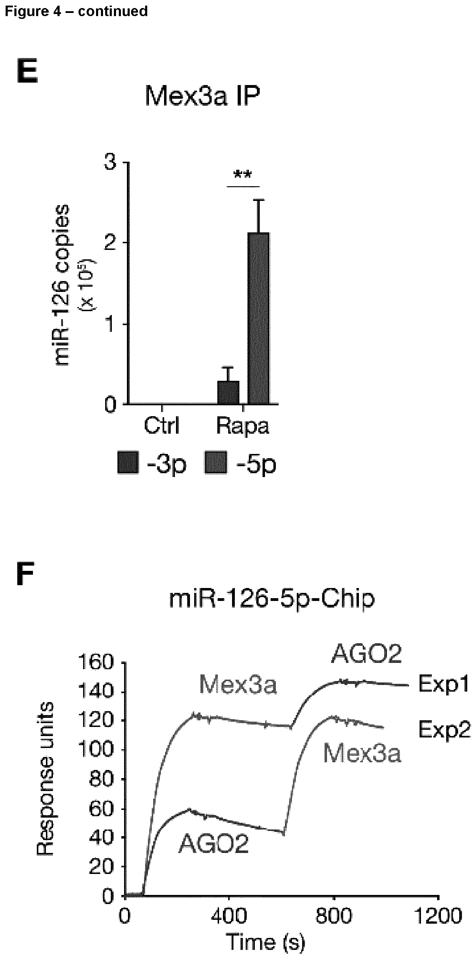

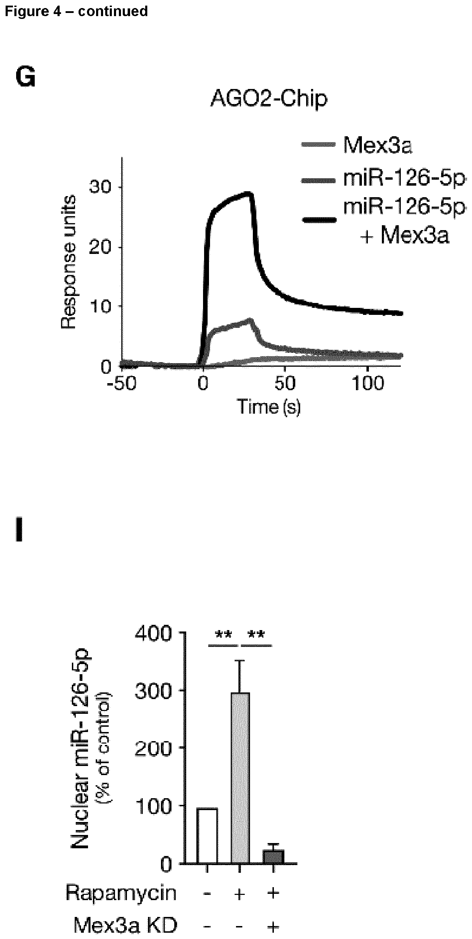

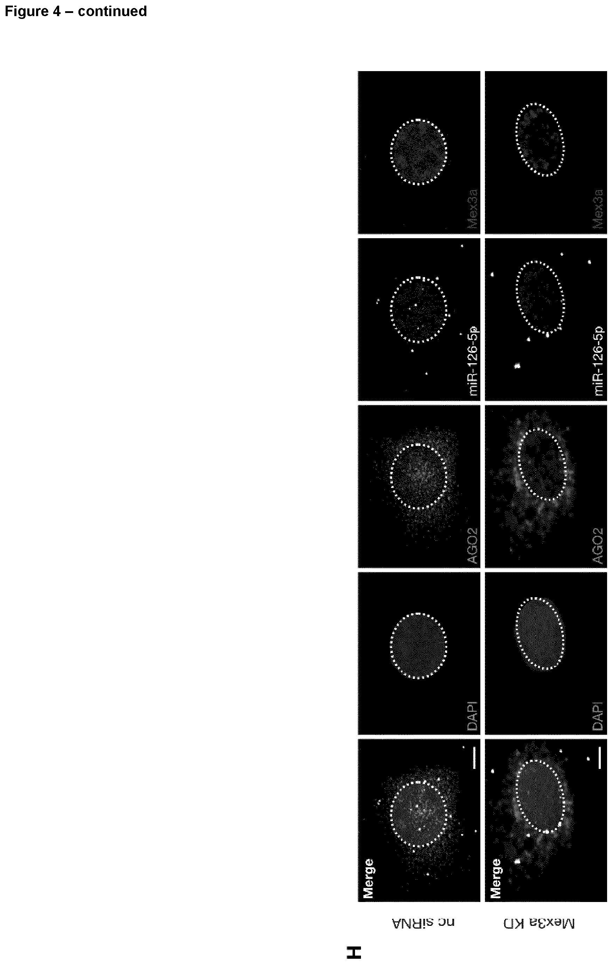

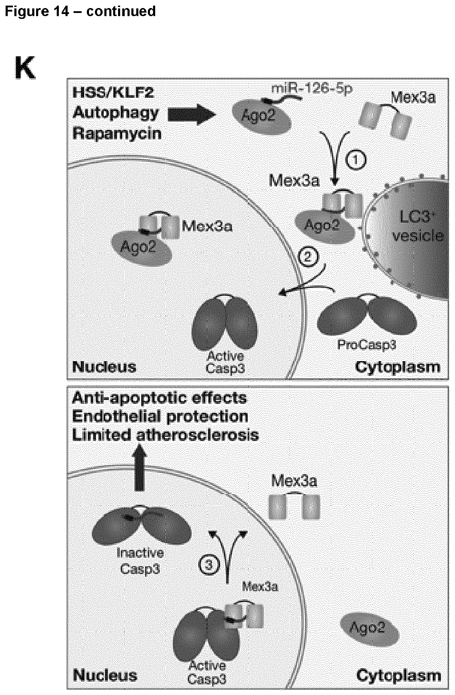

Mex3a promotes the nuclear enrichment of miR-126-5p. It is shown in the appended examples that Mex3a complexes with miRNA126-5p (Fig 4) and then guides the nuclear transfer of miRNA126-5p (Fig. 14K).

The amino acid sequence being at least 80% identical to SEQ ID NO: 2 is with increasing preference at least 85%, 90%, 95%, 96%, 97%, 98% and 99% identical to SEQ ID NO: 2. These amino acid sequences preferably retain the capability of SEQ ID NO: 2 to bind to miR-126-5p as well as the capability of SEQ ID NO: 2 to enter the nucleus of endothelial cells.

Mex3a binds to miRNA126-5p via its KH-domains 1 and 2 (Fig. 4). KH1 maps in amino acid positions 221-306 of SEQ ID NO: 2 and KH2 maps in amino acid positions 320-396 of SEQ ID NO: 2.

Within the amino acid sequence being at least 80% identical to SEQ ID NO: 2 and the preferred examples thereof requiring a higher sequence identity it is therefore preferred that the amino acid positions corresponding to positions 221-306 and/or the amino acid positions corresponding to positions 320-396 of SEQ ID NO: 2 are at least 95%, 96%, 97%, 98%, 99% and 100% identical to amino acid positions 221-306 and/or amino acid positions 320-396 of SEQ ID NO: 2.

Mex3a is transported to the nucleus via an importin-dependent nuclear localization sequence within the RING domain of Mex3a. The RING domain maps in amino acid positions 417-445 of SEQ ID NO: 2.

Within the amino acid sequence being at least 80% identical to SEQ ID NO: 2 and the preferred examples thereof requiring a higher sequence identity it is therefore also preferred that the amino acid positions corresponding to positions 417-445 of SEQ ID NO: 2 are at least 95%, 96%, and 100% identical to amino acid positions 417-445 of SEQ ID NO: 2.

The second nucleic acid molecule expressing the polypeptide of (i) is preferably an expression vector as described herein above. The vector may also be the same as the one used for the expression of the (first) nucleic acid molecule. In this case the vector may comprise two expression cassettes expression the (first) nucleic acid molecule and the second nucleic acid molecule, respectively. The vector may also comprise one expression cassettes expression the (first) nucleic acid molecule and the second nucleic acid molecule.

In accordance with a more preferred embodiment of the first aspect of the invention the second nucleic acid molecule is a vector, preferably an AAV, more preferably AAV9, AAV, AAV5, and most preferably AAV9.

The definitions and preferred embodiments of the AVV as discussed in connection with the (first) nucleic acid molecule according to the invention apply *mutatis mutandis* to the AVV to be used for the expression of the second nucleic acid molecule.

The AVV may also be the same as the one used for the expression of the (first) nucleic acid molecule. In this case the AVV may comprise two expression cassettes expression the (first) nucleic acid molecule and the second nucleic acid molecule, respectively. The AVV may also comprise one expression cassettes expression the (first) nucleic acid molecule and the second nucleic acid molecule.

In the case of one expression cassette the expression is generally controlled by one promoter, said promoter being preferably an endothelial cell-specific promoter.

In accordance with another more preferred embodiment of the first aspect of the invention the second nucleic acid molecule is expressed under the control of an endothelial cell-specific promoter.

The definitions and preferred embodiments of the endothelial cell-specific promoter as discussed in connection with the (first) nucleic acid molecule according to the invention apply *mutatis mutandis* to the endothelial cell-specific promoter to be used for the expression of the second nucleic acid molecule.

In accordance with another preferred embodiment of the first aspect of the invention in addition (i') a second polypeptide comprising or consisting of the amino acid sequence of SEQ ID NO: 3 or an amino acid sequence being at least 80% identical thereto, or (ii") a third nucleic acid molecule expressing the polypeptide of (i') is used in the prevention and/or treatment of disease-related apoptosis.

SEQ ID NO: 3 is the nucleic acid sequence of AGO2 (protein argonaute-2). AGO2 is required for RNA-mediated gene silencing (RNAi) by the RNA-induced silencing complex (RISC). The 'minimal RISC' appears to include AGO2 bound to a short guide RNA such as a microRNA (miRNA) or short interfering RNA (siRNA). These guide RNAs direct RISC to complementary mRNAs that are targets for RISC-mediated gene silencing. The precise mechanism of gene silencing depends on the degree of complementarity between the miRNA or siRNA and its target. Binding of RISC to a perfectly complementary mRNA generally results in silencing due to endonucleolytic cleavage of the mRNA specifically by AGO2. Binding of RISC to a partially complementary mRNA results in silencing through inhibition of translation, and this is independent of endonuclease activity. AGO2 is ubiquitously expressed in cells.

It can be taken from the appended examples that at the cytoplasmic site Mex3a co-localizes with AGO2 (Fig. 4) and the presence of miR126-5p is required in order to form a ternary complex of Mex3a-miR126-5p-AGO2 without a target mRNA (Fig. 4).

In accordance with a more preferred embodiment of the first aspect of the invention the third nucleic acid molecule is a vector, preferably an AAV, more preferably AAV9, AAV1 or AAV5 and most preferably AAV9.

The definitions and preferred embodiments of the AVV as discussed in connection with the (first) nucleic acid molecule according to the invention apply *mutatis mutandis* to the AVV to be used for the expression of the third nucleic acid molecule.

The AVV may also be the same as the one used for the expression of the (first) and/or second nucleic acid molecule. In this case the AVV may comprise two or three expression cassettes expression the three nucleic acid molecules. The AVV may comprise one expression cassettes expression the three nucleic acid molecules.

In the case of one expression cassette expression two or all three of three nucleic acid molecules the expression is generally controlled by one promoter, said promoter being preferably an endothelial cell-specific promoter.

In accordance with another more preferred embodiment of the first aspect of the invention the second nucleic acid molecule is expressed under the control of an endothelial cell-specific promoter.

The definitions and preferred embodiments of the endothelial cell-specific promoter as discussed in connection with the (first) nucleic acid molecule according to the invention apply *mutatis mutandis* to the endothelial cell-specific promoter to be used for the expression of the third nucleic acid molecule.

As regards the embodiments being characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.
- **Fig.1.**: KLF2-induced autophagy triggers differential regulation of miR-126 strands and miR-126-5p nuclear localization. **A,B,** Expression of pre-miR-126, miR-126-3p and miR-126-5p in human umbilical vein endothelial cells (HUVECs) overexpressing KLF2 (n=5-7) (**A**) and in aortic intima of *Cdh5^{Cre}Klf2*^{*fl*/*fl*} (KLF2^{EC-KO}) and control mice (WT) (n=4-5) (**B**). **C,D,** Western blot analysis of autophagy markers upon KLF2 overexpression and densitometric quantification of the LC3-II/LC3-I ratio and p62 expression (normalized to β-actin) (n=4). **E,** Amounts of miR-126-3p and miR-126-5p in HUVECs treated with rapamycin and bafilomycin (n=6). **F,** Representative immunofluorescence of miR-126-3p and miR-126-5p mimics (green) in HUVECs treated with or without rapamycin. Scale bars, 5 µm. **G,H,** Quantification of cytoplasmic mimic foci per cell (n=4-5) (**G**) and Pearson's colocalization coefficient for mimic and Hoechst nuclear dye (n=39-69 cells) (**H**). **I,** Nucleocytoplasmic distribution of endogenous miR-126 strands in HUVECs upon rapamycin treatment (Rapa) (n=4). **J,K,** Nuclear and cytoplasmic amounts of miR-126-5p in HUVECs treated with rapamycin and silencing of autophagy rate-limiting proteins (ATG5 or ATG7) or co-treated with the importin inhibitor ivermectin (n=3-6) (**J**) and in HUVECs overexpressing KLF2 treated with the autophagy inhibitor 3-Methyladenine (3-MA) (n=3) **(K).** **P*<0.05, ***P*<0.01, ****P*<0.001. Two-tailed Student's *t*-test **(A,B,D)** or factorial ANOVA with Bonferroni *post-hoc* test (**E,G-I,K**).

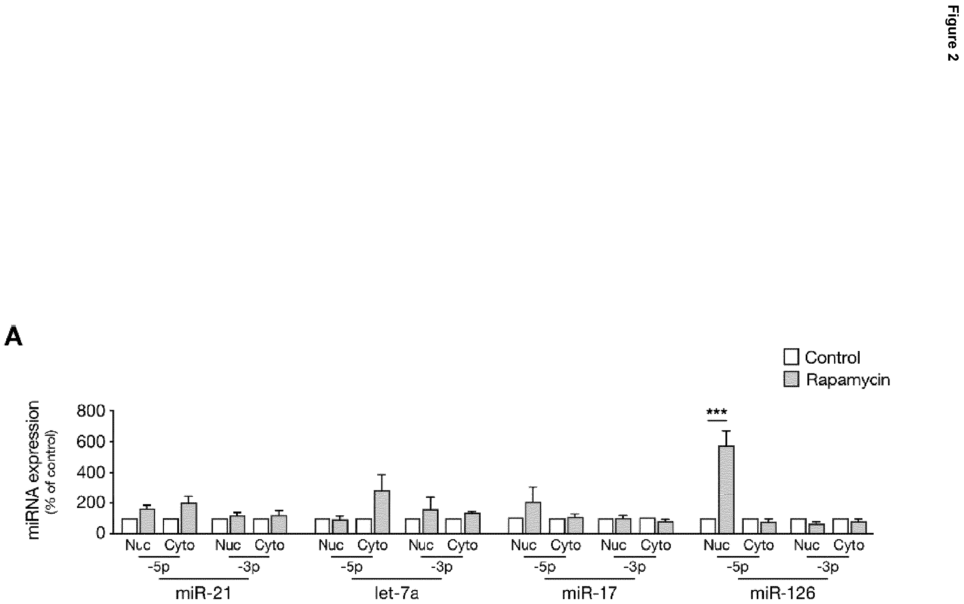

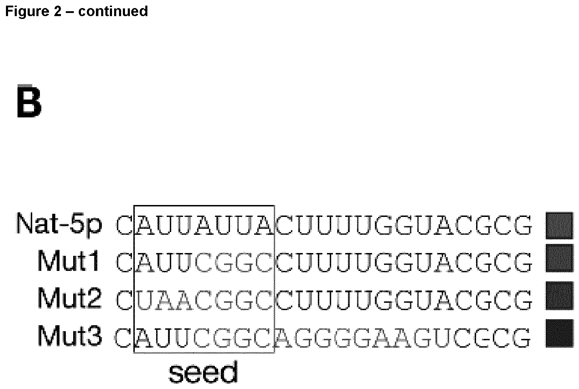

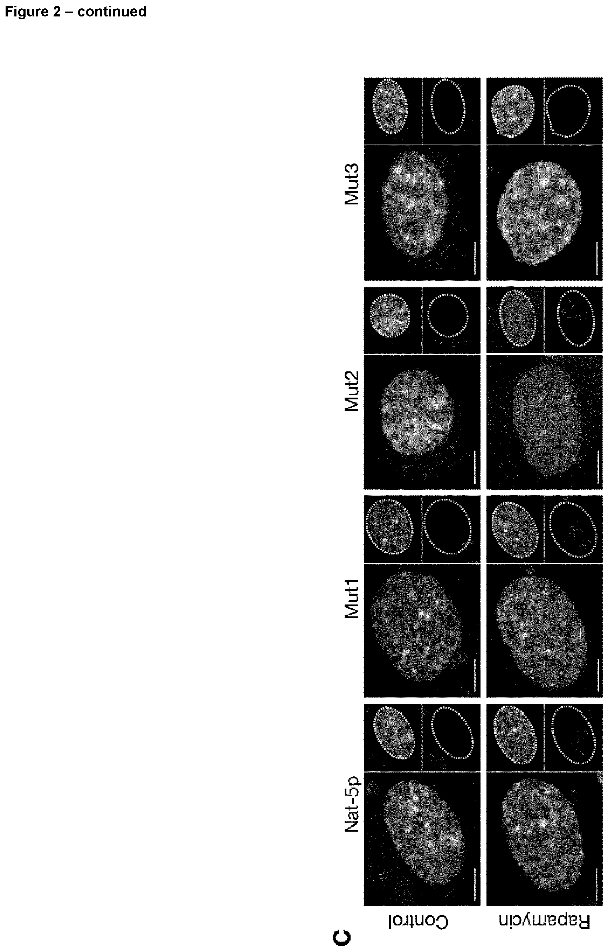

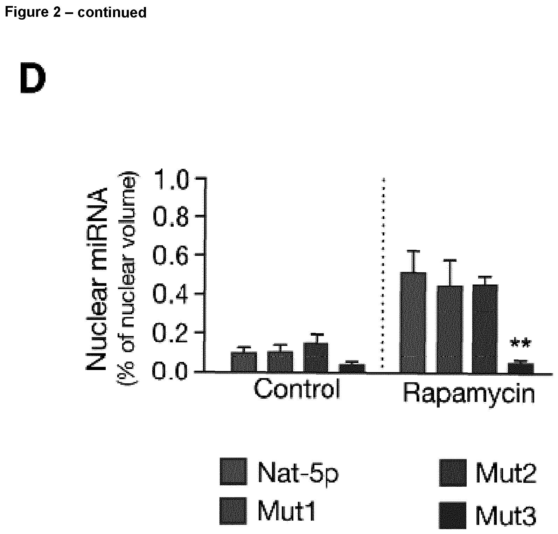

- **Fig. 2.**: Preferential nuclear transfer of miR-126-5p is unrelated to its seed sequence. **A,** Nuclear and cytoplasmic amount of both strands of miR-21, let-7a, miR-17 and miR-126 in HUVECs treated with or without rapamycin, as assessed by qPCR (n=3-4). **B,** Sequences of miR-126-5p and mutants (Mut1-3) transfected in HUVECs. **C,** Confocal microscopy showing localization of miR-126-5p (Nat-5p) and mutants (Mut1-3, red) in DAPI-stained nuclei (gray). **D,** Quantification of mean nuclear volume occupied by mutants assessed after 3D deconvolution and rendering (n=4-6). Scale bar, 5 µm. **P<0.01, ***P<0.001. Factorial ANOVA with Bonferroni *post-hoc* test (**A,D**).

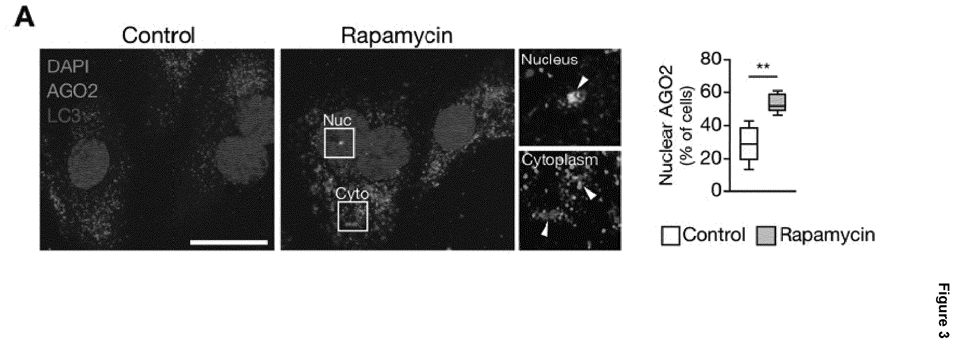

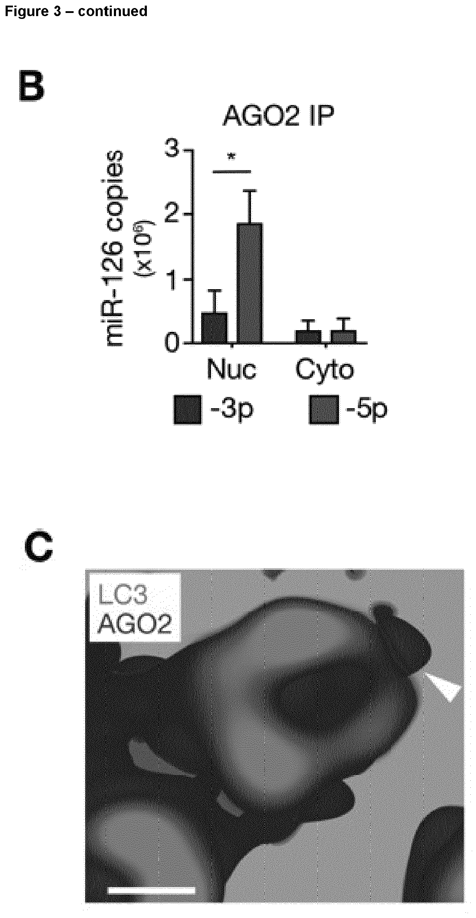

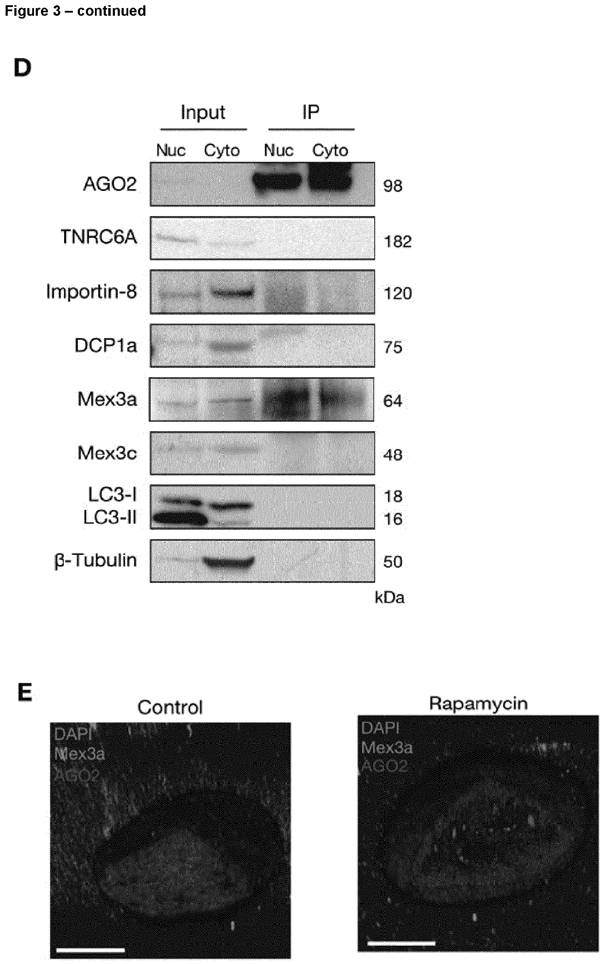

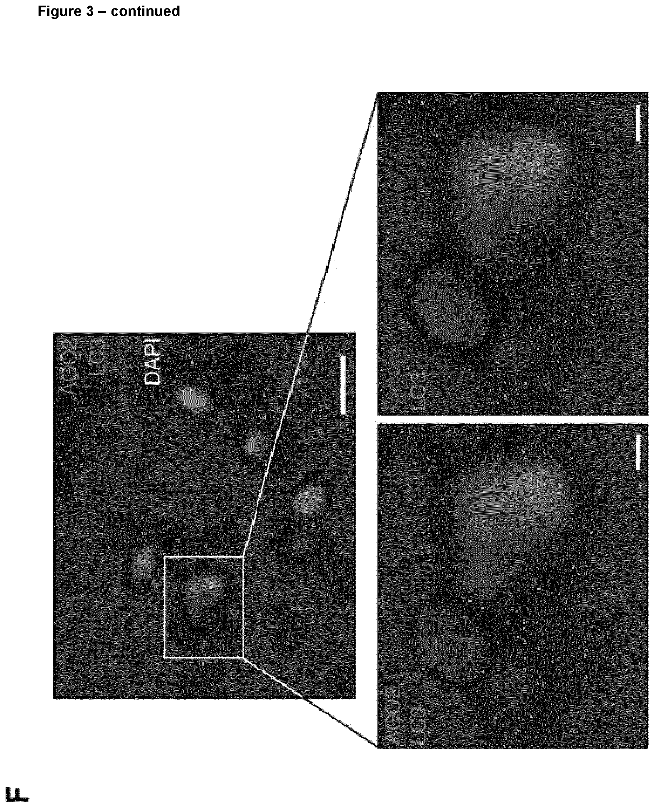

- **Fig. 3.**: Nuclear miR-126-5p interacts with the RNA-binding protein Mex3a and AGO2. **A,** Immunofluorescence for AGO2 and LC3 in rapamycin-treated HUVECs and controls, and quantification of nuclear AGO2-positive cells. Insets show colocalizing signals (white arrowheads) (n=5). Scale bar, 20 µm. **B,** RNA immunoprecipitation of nuclear and cytoplasmic AGO2 in rapamycin-treated HUVECs showing miR-126 strand loading. **C,** STED nanoscopy showing AGO2 signals on the outer surface of a LC3-demarcated vesicle (white arrowheads). Scale bar, 200 nm. **D,** Co-immunoprecipitation of AGO2 and Mex3a in rapamycin-treated HUVECs. **E,** Three-dimensional confocal microscopy for AGO2 and Mex3a in the nucleus of rapamycin-treated HUVECs and controls. Scale bar, 5 µm. **F,** STED nanoscopy detailing the AGO2:Mex3a:LC3 cytoplasmic complex in rapamycin-treated HUVECs. Scale bar, 1 µm (upper panel). Magnification of the complex is shown for the two fluorescent channels (AGO2 with LC3 and Mex3a with LC3) in the lower panels. Scale bar, 200 nm. *P<0.05, **P<0.01. Two-tailed Student's t-test (**A**), two-factor repeated-measures ANOVA with Bonferroni *post-hoc* test (**B**).
- **Fig. 4.**: Mex3a preferentially binds miR-126-5p to drive nuclear enrichment. **A,** Overlay of ¹H-¹⁵N HSQC spectra of Mex3a^{(KH1KH2)} in absence (blue cross-peaks) or presence (red) of miR-126-5p (molar ratio 1:2). **B,** SPR sensorgram for interaction between immobilized miR-126-5p and full-length Mex3a. KD=3.6±2.9 nM. **C,** Pairwise distance distribution derived from SAXS of Mex3a^{(KH1KH2)} in absence (blue) or presence (red) of miR-126-5p. **D,** Estimated binding affinity parameters for the interaction of Mex3a with miR-126-5p and miR-126-3p. **E,** RNA immunoprecipitation of Mex3a in nuclear lysates of HUVECs treated without (Ctrl) and with rapamycin (Rapa) showing miR-126 strand loading (n=3). **F,** SPR sensorgrams showing interaction of sequentially loaded full-length recombinant Mex3a and AGO2 (Exp1) and vice versa (Exp2) on immobilized miR-126-5p. **G,** SPR sensorgrams for full-length Mex3a, miR-126-5p or their combination perfused on AGO2, showing a marked interaction of Mex3a and AGO2 in a complex with miR-126-5p. **H,** Representative deconvoluted images for fluorescence *in situ* hybridization (FISH) for miR-126-5p (grey) and co-immunostaining for AGO2 (green) and Mex3a (red) in rapamycin-treated HUVECs with or without Mex3a knock-down (KD). Scale bar, 5 µm. **I,** Amount of nuclear miR-126-5p in HUVECs upon rapamycin and Mex3a silencing (n=5). **P<0.01. Two-factor repeated-measures ANOVA (**E**) or univariate ANOVA with Bonferroni *post-hoc* test (**I**).
- **Fig. 5.**: Nuclear miR-126-5p inhibits apoptosis. **A,B,** Circos plot (**A**) showing changes in EC-relevant genes induced by rapamycin and miR-126-5p. Heat maps show relative expression in rapamycin-treated HUVECs vs. controls (inner) and cells treated with rapamycin and miR-126-5p mimic or inhibitor (outer). KEGG functional categories are indicated by ribbons (**A**) and ranked according to fold-enrichment (**B**). **C,D,** Western blot analysis of apoptosis mediators in controls and rapamycin-treated HUVECs with gain- or loss-of-function for miR-126-5p (**C**) or after Mex3a knock-down (KD) vs. control siRNA (NC) (**D**) including normalized densitometry analysis. **E-G,** Representative flow cytometry analysis (**E**) and quantification of apoptosis rates (percentage of Ann-V⁺ cells) after rapamycin treatment for 3 h (n=10) and miR-126-5p inhibition (-5p inh) (n=10) or Mex3a KD (n=10) (**F**) and upon Mex3a overexpression (**G**) with or without miR-126-5p inhibitor (n=7). **P<0.01, ***P<0.001. Mann-Whitney *U*-test (**F**) or univariate ANOVA (**G**) with Bonferroni *post-hoc* test.
- **Fig. 6.**: miR-126-5p interacts with caspase-3 to inhibit its activity. **A,** Representative deconvoluted image for fluorescence *in situ* hybridization (FISH) for miR-126-5p (red) and co-immunostaining for caspase-3 (green) showing co-localizing signals in nucleus and perinuclear area (white arrowheads). Plot shows normalized fluorescence intensity profiles for green and red channels in the position marked by orange arrow. Scale bar, 5 µm. **B,C,** Representative deconvoluted images for FISH for miR-126-5p and co-immunostaining for caspase-3 in rapamycin-treated HUVECs transfected with non-specific siRNA (nc siRNA) or siRNA against Mex3a (Mex3a KD) (**B**) and Pearson's coefficients for caspase-3 and miR-126-5p colocalization, red line denotes median (**C**, n=100-110). **D,** RNA immunoprecipitation of nuclear and cytoplasmic caspase-3 for miR-126 strand loading in HUVECs treated with rapamycin (n=6) or exposed to laminar shear stress (HSS, 12 dyne/cm2) (n=3) and controls (n=9). **E,** Biotin-RNA pull-downs of miR-126 strands transfected in HUVECs showing interaction with Mex3a and caspase-3 after rapamycin treatment. **F,** Pearson's coefficient for miR-126-5p mutants and caspase-3 colocalization in rapamycin-treated HUVECs (n=4). Sequences of native miR-126-5p (Nat-5p) and mutants (Mut1-3) are given below. Red denotes mutated nucleotides. **G,** Superposition of two-dimensional NMR spectra of ¹H¹³C methyl ILVM, ²H-labeled caspase-3 (blue) and in complex with miR-126-5p (red), molar ratio 1:3. **H,** Competition-binding assay using AGO2 and caspase-3 on immobilized miR-126-5p (n=3). IC₅₀=126.9 nM. **I,** Competition-binding assay using caspase-3 monomer and miR-126-5p (n=3). IC₅₀=6.0 µM. **J,** Cell-free analysis of caspase-3 activity in presence of miR-126-5p (Nat-5p), miR-126-5p mutant (Mut3), miR-126-3p (Nat-3p) (4, 8 or 16 µM), RNAse, the caspase-3 inhibitors Z-DEVD-FMK (DEVD) or Z-VAD-FMK (10 µM) (n=3). *P<0.05, **P<0.01, ***P<0.001. Factorial (**D,J**) or univariate ANOVA (**F**) with Bonferroni *post-hoc* test.

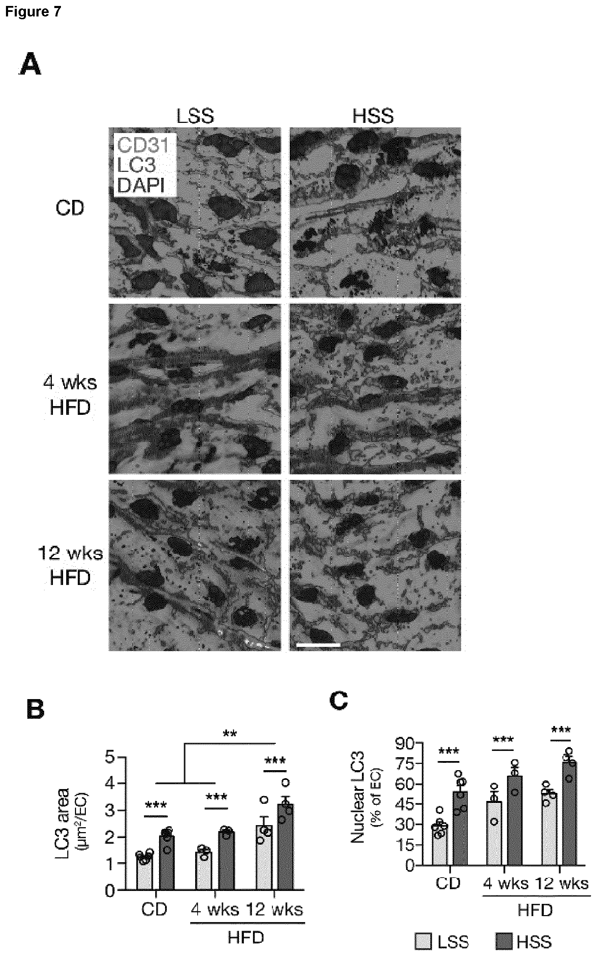

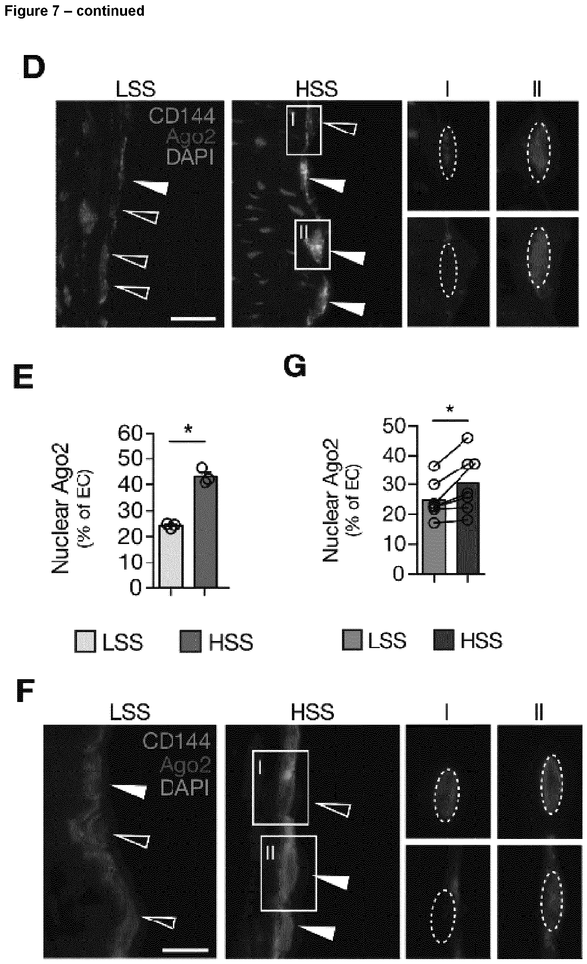

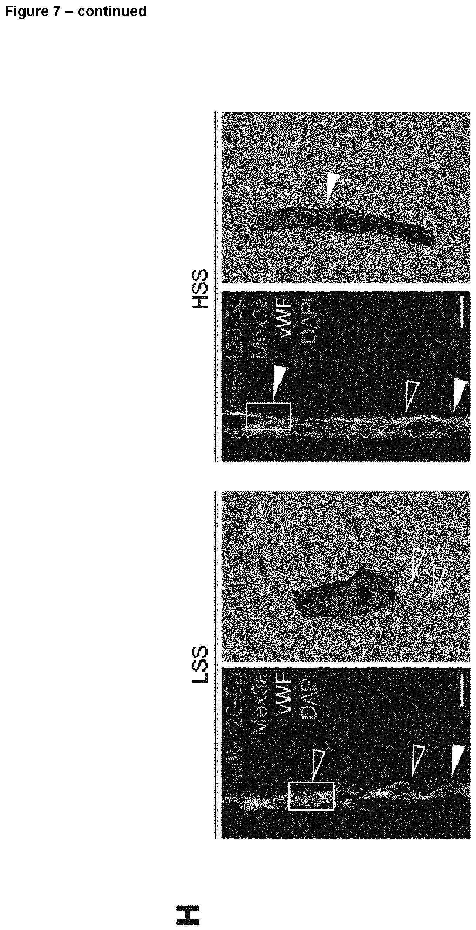

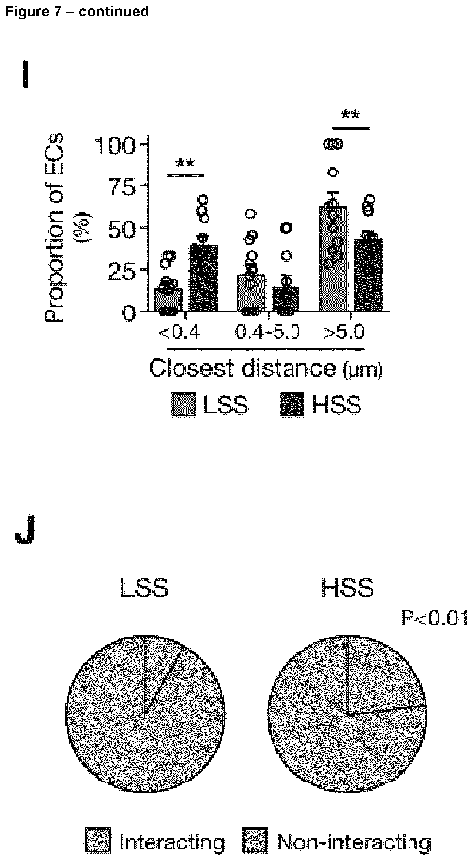

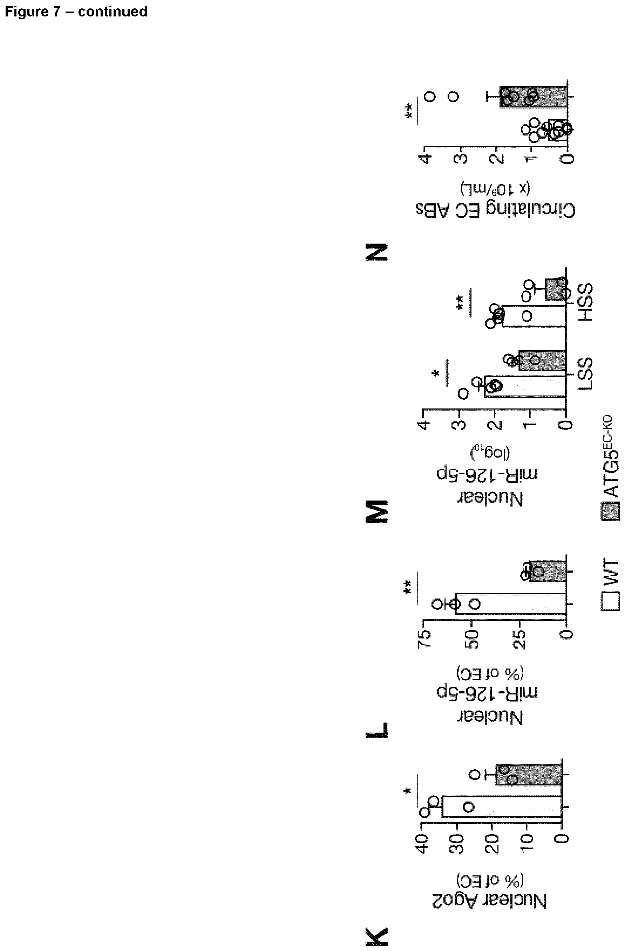

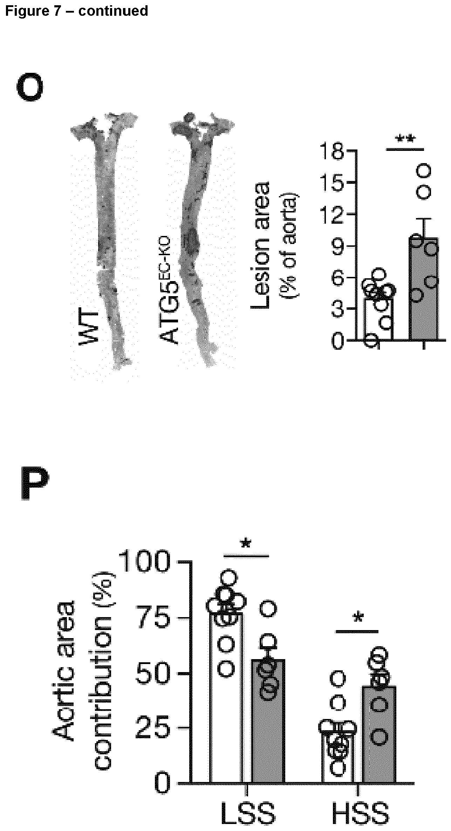

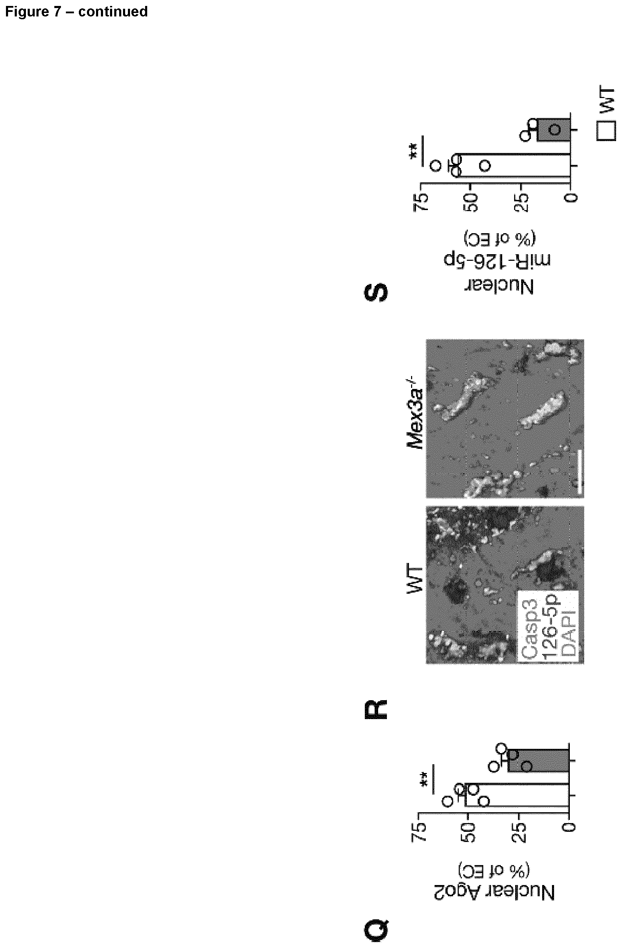

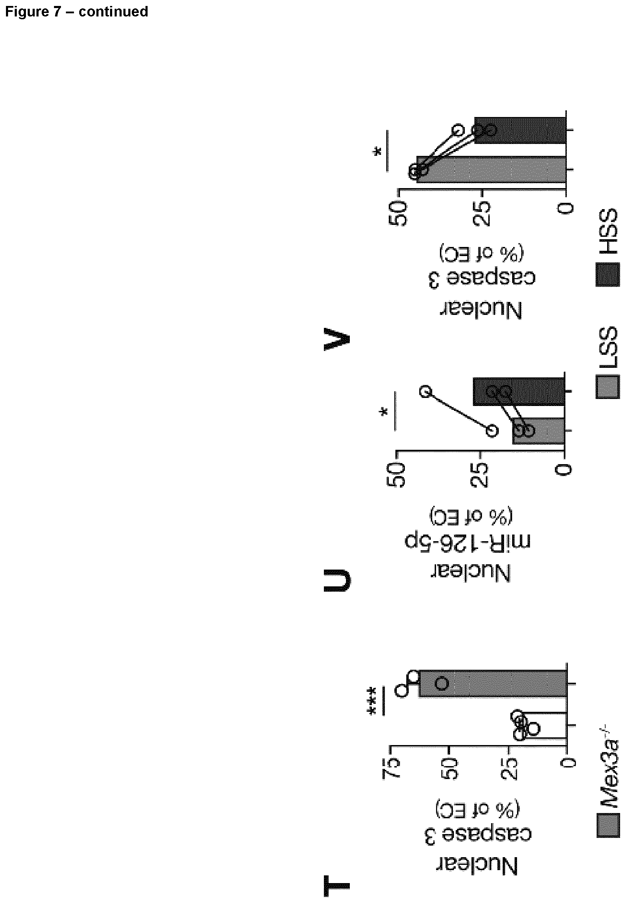

- **Fig. 7.**: Deleting *Atg5* or Mex3a attenuates nuclear miR-126-5p-AGO2 shuttling to drive endothelial apoptosis and atherosclerosis *in vivo.* **A-C,** Three-dimensional deconvolution microscopy (**A**) with quantification of LC3⁺ area (**B**) and percentage of ECs showing nuclear LC3 (**C**) in *en face* aortic arch (low-shear stress, LSS) vs. thoracic aorta (high-shear stress, HSS) of *Apoe*^{*-*/*-*} mice (n=3-6) fed chow (CD) or high-fat diet (HFD) for 4-12 weeks. Scale bar, 20 µm. **D-G,** Immunofluorescence (**D,F**) and percentage of ECs showing nuclear AGO2 (**E,G**) in aortic arch (LSS) and thoracic aorta (HSS) of *Apoe*^{*-*/*-*} mice (n=3) on HFD for 12 weeks (**D,E**), or in human carotid artery specimens collected upstream (HSS) or downstream (LSS) of the bifurcation (n=7) (**F,G**). Regions I-II in **D,F** are magnified in insets to show single channel details for ECs without (I or empty arrowheads) and with (II or filled arrowheads) nuclear AGO2. Scale bar, 20 µm. **H-J,** Confocal deconvoluted microscopy for Mex3a immunostaining and fluorescence *in situ* hybridization (FISH) for miR-126-5p in the nucleus of ECs stained for von Willebrand factor (vWF) in human carotid artery specimens upstream (HSS) or downstream (LSS, covering atheroma) of the bifurcation (n=3). Regions in the box are magnified on the right (**H**). Scale bar, 20 µm. The closest distance between Mex3a and miR-126-5p in EC nuclei was quantified in 12 fields of view per region. Distributions are shown for overall distances (**I**) and for a proxy of interaction defined by a distance below the resolution limit of 240 nm (**J**). **K-P,** Characterization of *Apoe*^{*-*/}*⁻Cdh5^{cre+}Atg5*^{*fl*/*fl*} (ATG5^{EC-KO}) and *Apoe*^{*-*/}*⁻Cdh5^{Cre-}Atg5*^{*fl*/*fl*} (wild-type, WT) mice on HFD for 12 weeks. Percentage of ECs showing nuclear AGO2 (immunofluorescence, **K**) and miR-126-5p (*in situ* PCR, **L**) in thoracic aorta (n=3). **M,** Nuclear expression of miR-126-5p in aortic arch (LSS) vs. thoracic aorta (HSS) (n=4-5). **N,** Circulating endothelial ABs in plasma (n=8-10). **O,P,** Atherosclerotic lesion area in Oil-red-O-stained aortas (**O**) and fractional contribution of aortic arch (LSS) and thoraco-abdominal aorta (HSS) (**P**) (n=6-9). **Q,** Percentage of thoracic aortic ECs showing nuclear AGO2 in C57BI6/NCrl (WT) and *Mex3a*^{-/-} mice (n=4). **R-T,** FISH for miR-126-5p and co-immunostaining for caspase-3 and CD31 in *en face* thoracic aorta from WT and *Mex3a*^{*-*/*-*} mice. Scale bar, 20 µm. (**R**). Percentage of ECs showing nuclear miR-126-5p (**S**) and caspase-3 (**T**) (n=3-4). **U,V,** Percentage of ECs showing nuclear miR-126-5p (**U**) and caspase-3 (**V**) in human carotid artery specimens downstream (LSS) or upstream (HSS) of the bifurcation (n=3). * P<0.05, **P<0.01, ***P<0.001. Two-factor repeated measures ANOVA (**B,C**) or factorial ANOVA **(I,M,P)** with Bonferroni *post-hoc* test, paired t-test **(E,G,U,V),** unpaired Student's *t*-test **(K,L,N,O,Q,S,T),** or Fisher's X² test (**J**).

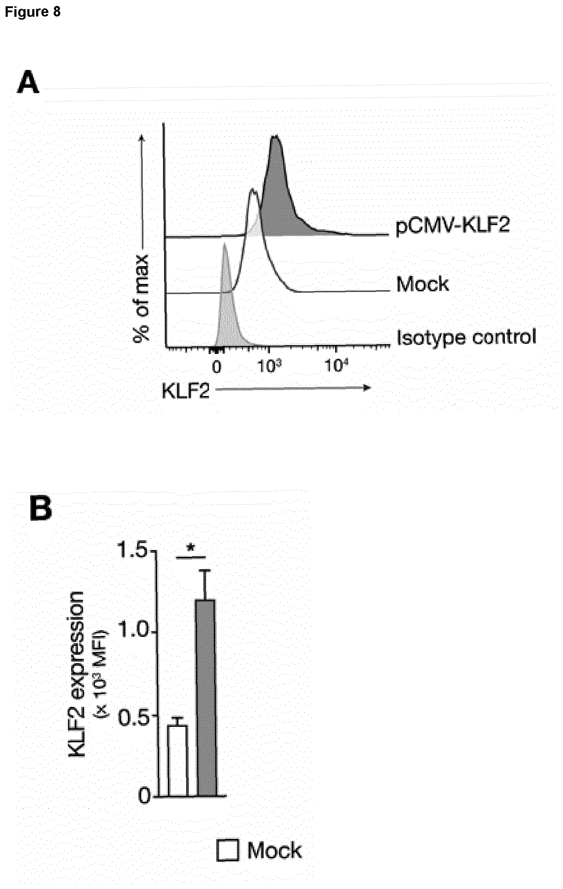

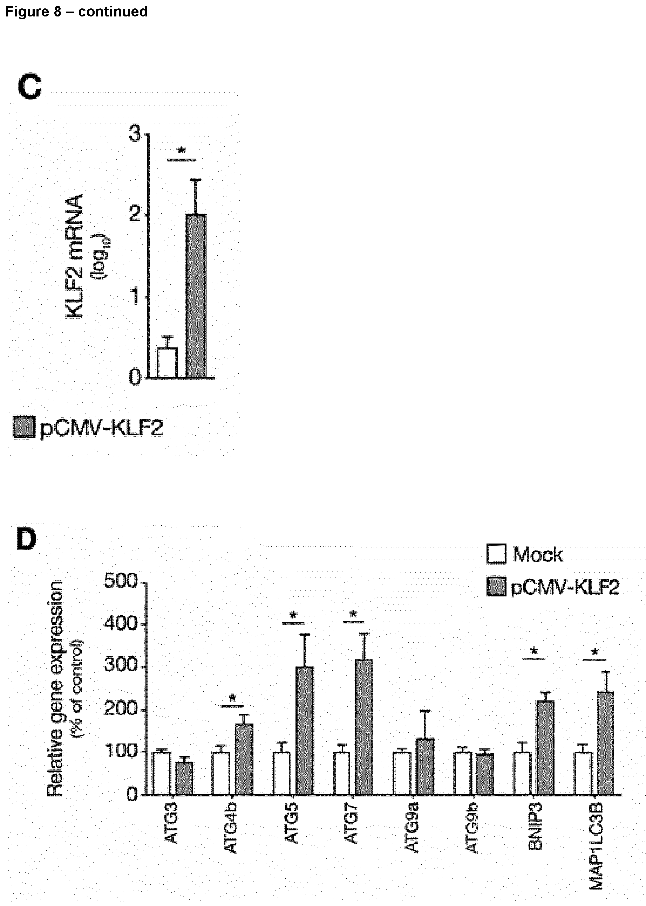

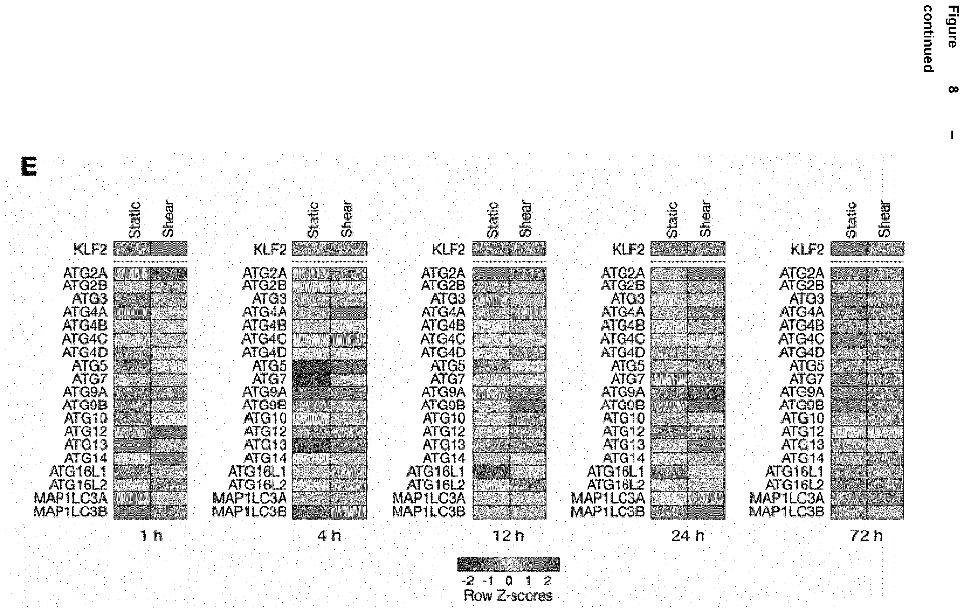

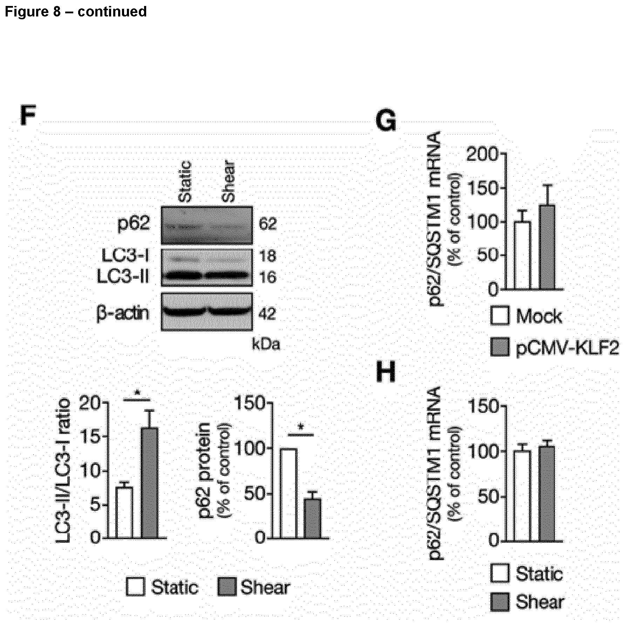

- **Fig. 8.**: Transcriptional effects of KLF2 and shear stress on autophagy-related genes. **A-C,** Representative flow cytometry histogram (**A**), quantification of KLF2 protein expression as mean fluorescence intensity (MFI) (**B**), and validation of KLF2 overexpression at the mRNA level by qPCR (**C**) in HUVECs transfected with a pCMV-KLF2 or an empty vector (n=3-4). **D,** Expression of autophagy-related genes (ATGs) reported as modulated upon autophagy (78) in HUVECs overexpressing KLF2 (n=3-6). **E,** Analysis of deposited genome-wide expression datasets of HUVECs exposed to atheroprotective shear stress (12±4 dyne/cm²) (GEO accession codes: GSE92506, GSE103672, GSE54384) or grown under static conditions revealed a time-dependent trend for the activation of multiple ATGs together with increased KLF2 expression. **F,** Western blot analysis of autophagy markers in HUVECs cultivated in static conditions or exposed to laminar shear stress (12 dyne/cm²) and densitometric quantification of the amount of LC3-II/LC3-I ratio and p62 (normalized to β-actin) (n=4). **G,H,** mRNA expression of p62/SQSTM1 measured by qPCR in HUVECs overexpressing KLF2 (**G,** n=6) or exposed to laminar shear stress (12 dyne/cm²) (**H**, n=6). Data are shown as mean ± s.e.m. **P*<0.05. Unpaired Student's t-test **(B,C,F-H)** with step-up correction for false discovery rate (**D**).

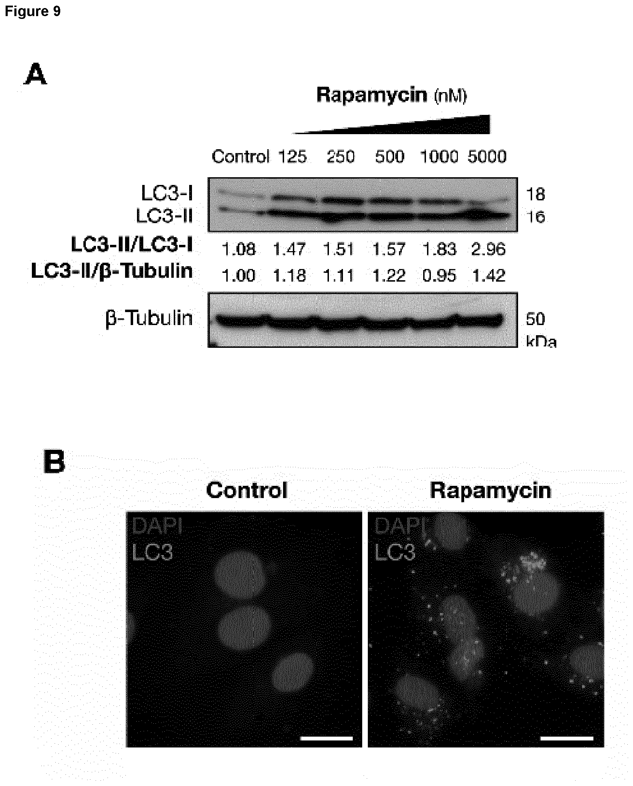

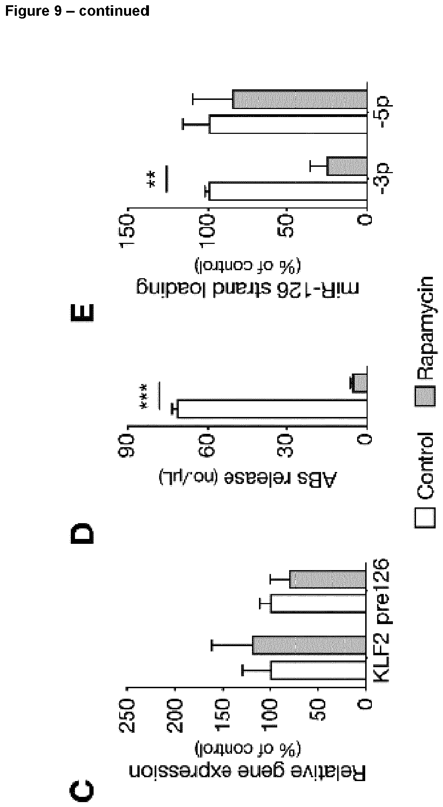

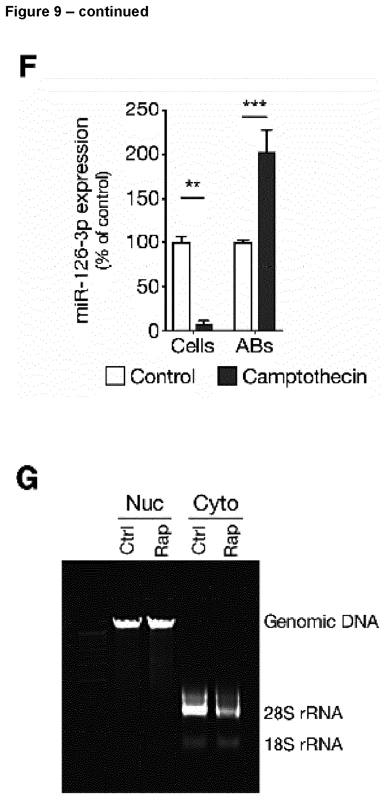

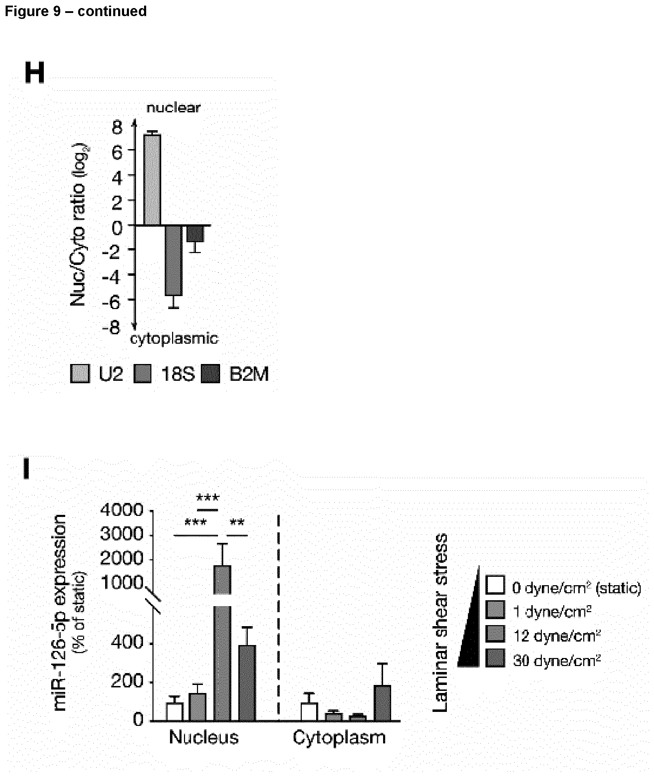

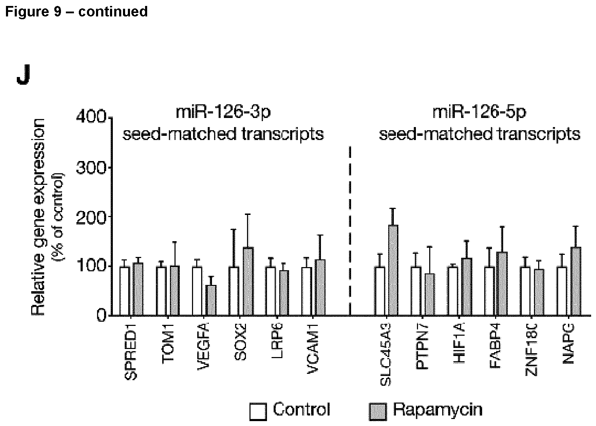

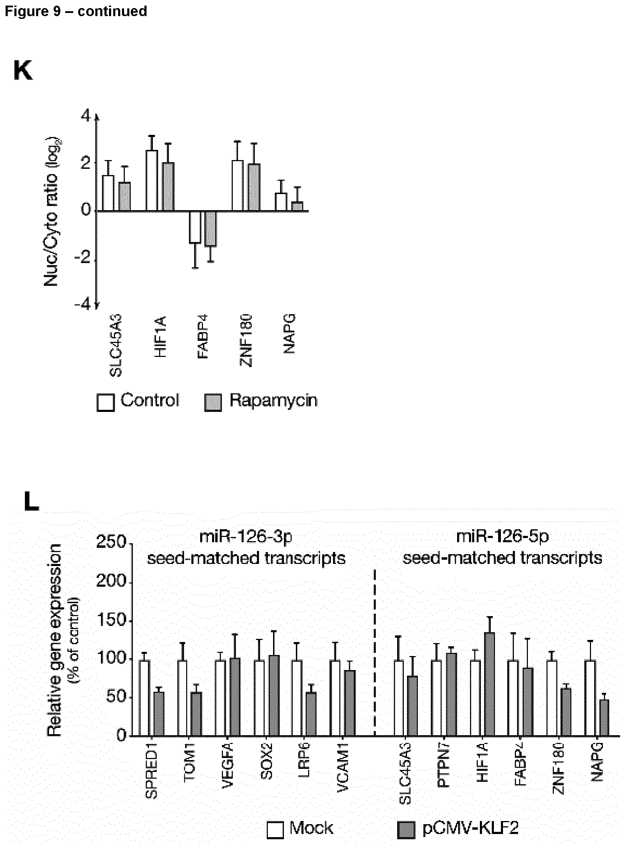

- **Fig. 9.**: Effect of short-term rapamycin treatment on miR-126 strand release and miR-126 strand-specific transcripts expression and distribution. **A,B,** Representative immunoblot shows LC3-I to LC3-II conversion in HUVECs treated with indicated concentrations of rapamycin for 3 h (**A**) and immunofluorescence image for LC3 in HUVECs treated with rapamycin (5 µM) (**B**), both confirming the activation of autophagic flux. **C,** Expression of KLF2 in HUVECs treated with or without rapamycin, as assessed by qPCR (n=6). **D,** Concentration of apoptotic bodies (ABs) in conditioned medium of HUVECs treated with or without rapamycin (n=4). **E,** Quantity of miR-126 strands in ABs sorted from conditioned medium of HUVECs treated with or without rapamycin (n=6). **F,** Intracellular and AB-loaded miR-126-3p in HUVECs treated with or without camptothecin to elicit apoptosis (n=3-6) as a positive control for miR-126-3p release. **G,** Representative agarose gel for nuclear (lanes 2-3) and cytoplasmic (lanes 4-5) RNA showing genomic DNA only in nuclear samples and rRNAs in cytoplasmic fractions. **H,** Nuclear and cytoplasmic expression of nuclear (U2 RNA) and cytoplasmic (18S rRNA and B2M) markers of separation, as measured by qPCR (n=3). **I,** Amounts of nuclear and cytoplasmic miR-126-5p in HUVECs cultivated in static condition or exposed to increasing intensity of laminar shear stress (1, 12, and 30 dyne/cm²) (n=5-6). **J-L,** Expression of selected seed-matched targets for miR-126 strands (identified by mirTarBase and miRbase) upon rapamycin treatment (**J**) or KLF2 overexpression (**L**) and nucleo-cytoplasmic distribution of selected seed-matched transcripts for miR-126-5p strands upon rapamycin treatment (**K**), as assessed by qPCR (n=3-4). Data are shown as mean ± s.e.m. ***P*<0.01; ****P*<0.001. Two-way ANOVA with Bonferroni *post-hoc* test **(C,E,F,I),** unpaired Student's *t*-test (**D**) with step-up correction for false discovery rate (**J-L**).

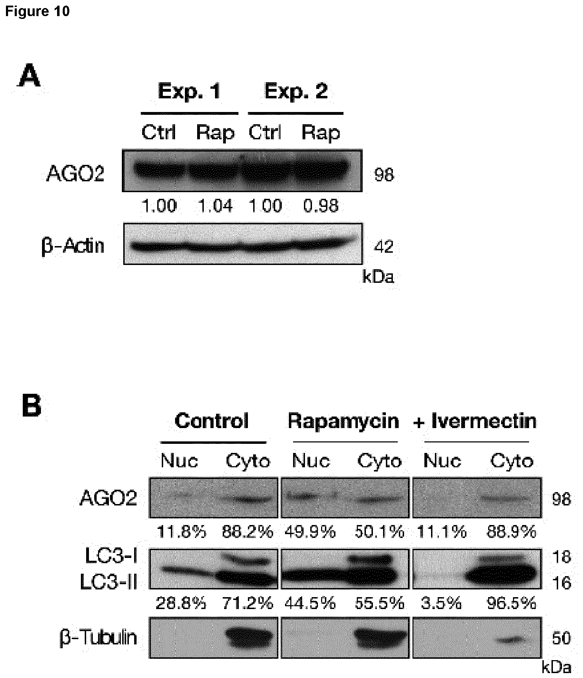

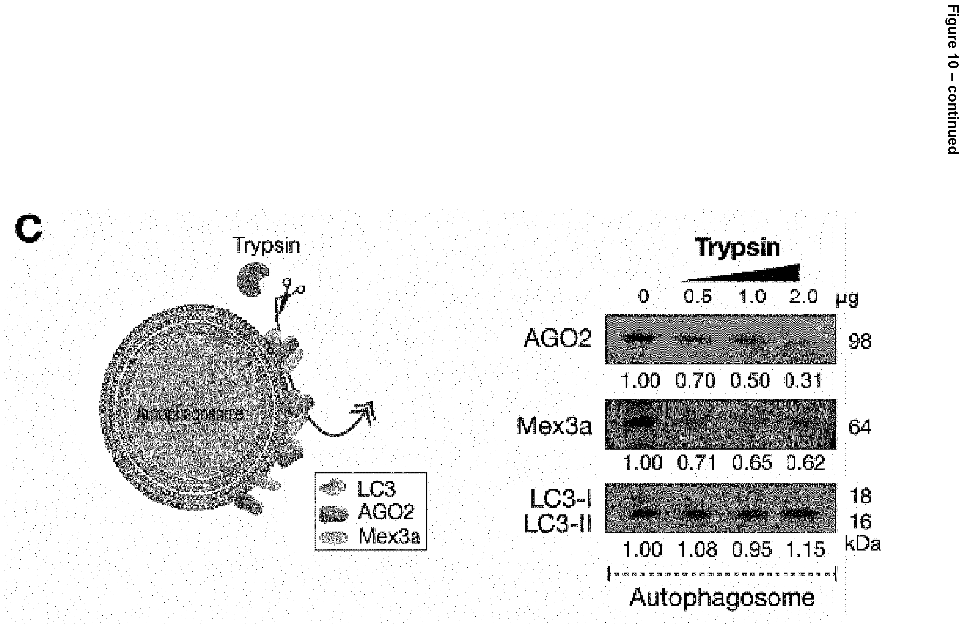

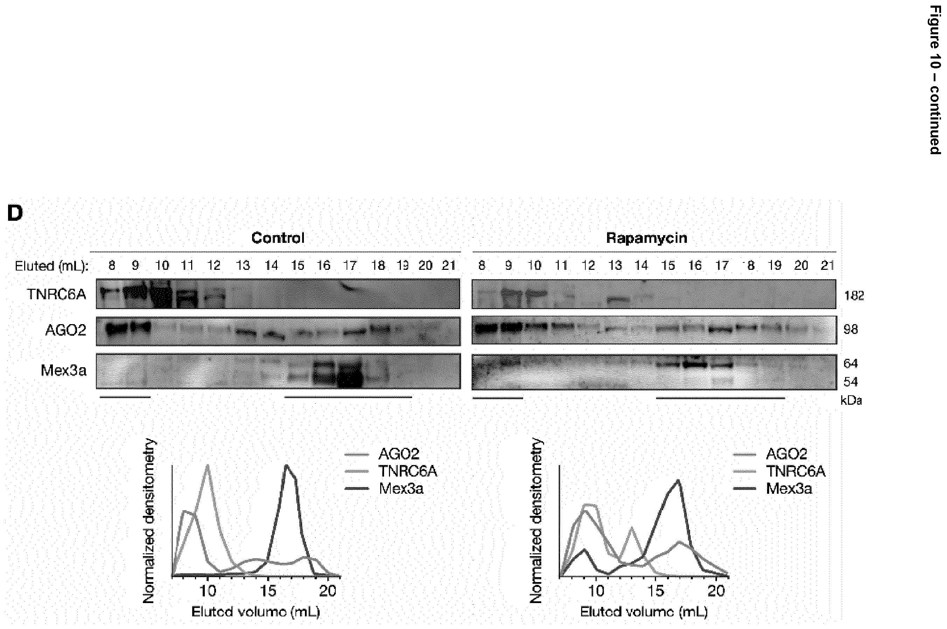

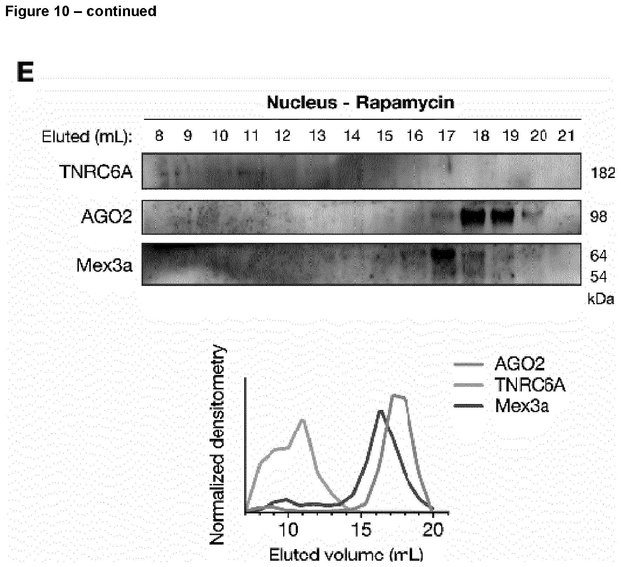

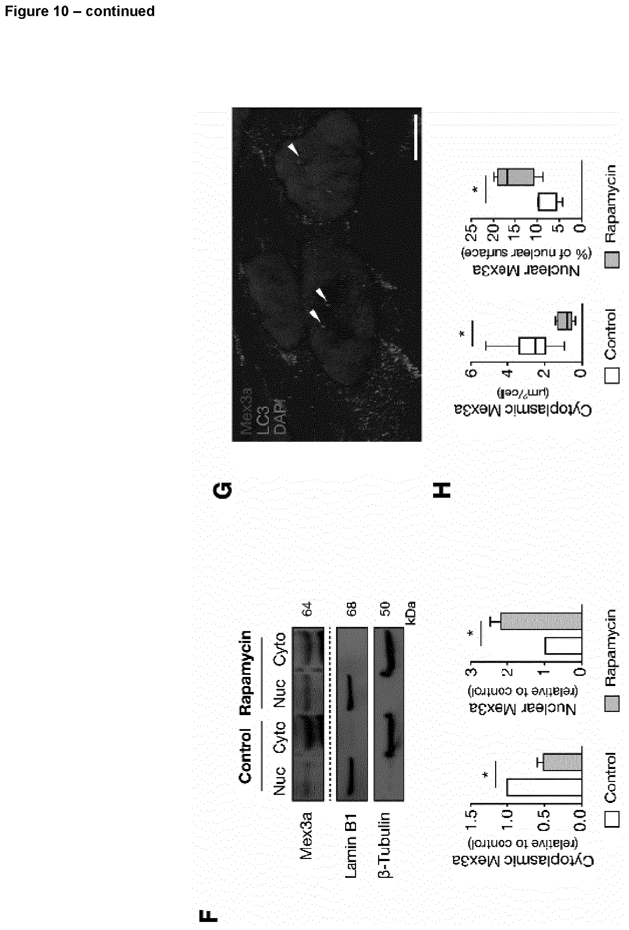

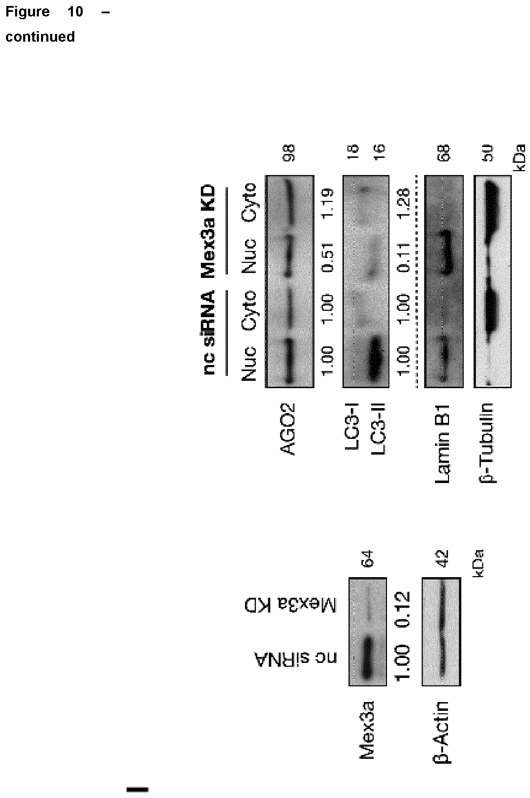

- **Fig. 10.**: Autophagy promotes nuclear enrichment of AGO2 and Mex3a. **A,** Representative immunoblot for AGO2 on whole cell lysates of HUVECs treated with (Rap) or without (Ctrl) rapamycin. **B,** Representative immunoblots for AGO2 and LC3 on subcellular fractions isolated from HUVECs treated with rapamycin or with rapamycin and ivermectin. **C,** Model depicting the principle of the autophagosome protection assay with the ability of trypsin to degrade extraluminal proteins (right panel) and immunoblots for AGO2, Mex3a and LC3 in autophagosomes isolated by density-gradient centrifugation and treated with different concentrations of trypsin. **D,E,** Size exclusion chromatography fractionation followed by immunoblot analysis for AGO2, Mex3a and TNRC6A from cytoplasmic lysates of control and rapamycin-treated HUVECs (**D**) and nuclear extracts of rapamycin-treated HUVECs (**E**). Lower panels show relative densitometric analysis of AGO2, Mex3a and TNRC6A content in each fraction. **F,** Immunoblot for Mex3a on nuclear and cytoplasmic fractions of HUVECs treated with rapamycin with relative densitometric analysis. **G,** Representative confocal microscopy image showing co-localizing signals for LC3 and Mex3a (white arrowheads) in the nucleus of rapamycin-treated HUVECs. Scale bar, 10 µm. **H,** Quantification of nuclear and cytoplasmic immunofluorescence signals for Mex3a in HUVECs treated with or without rapamycin (n=4-6). **I,** Immunoblots for AGO2 and LC3 on subcellular fractions isolated from HUVECs transfected with a non-specific siRNA (nc siRNA) or with a siRNA against Mex3a (Mex3a KD) and treated with rapamycin (left panel). β-Tubulin and lamin B1 are used as markers for cytoplasm (Cyto) and nuclear (Nuc) fractioning. Immunoblots for Mex3a showing efficiency of silencing (right panel). **P*<0.05. Unpaired Student's t-test (**F,H**).

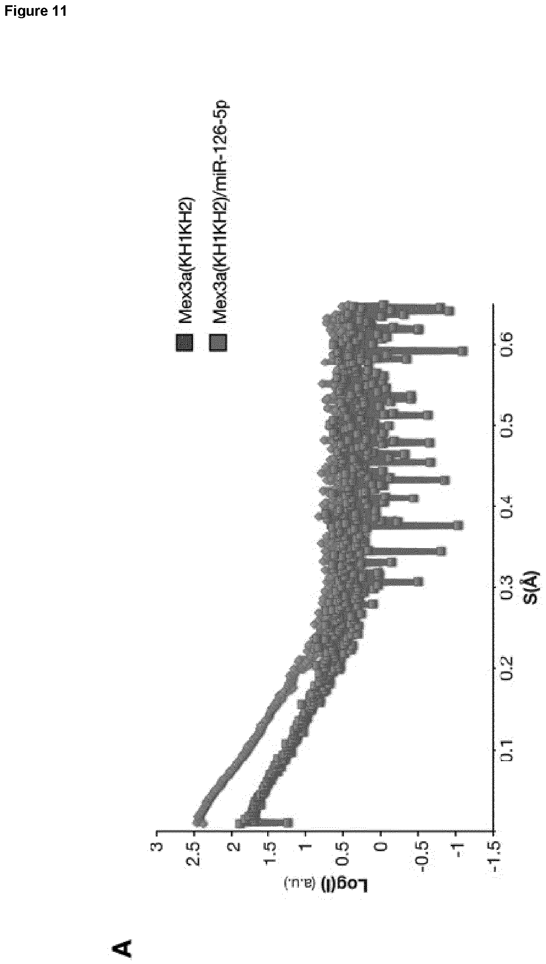

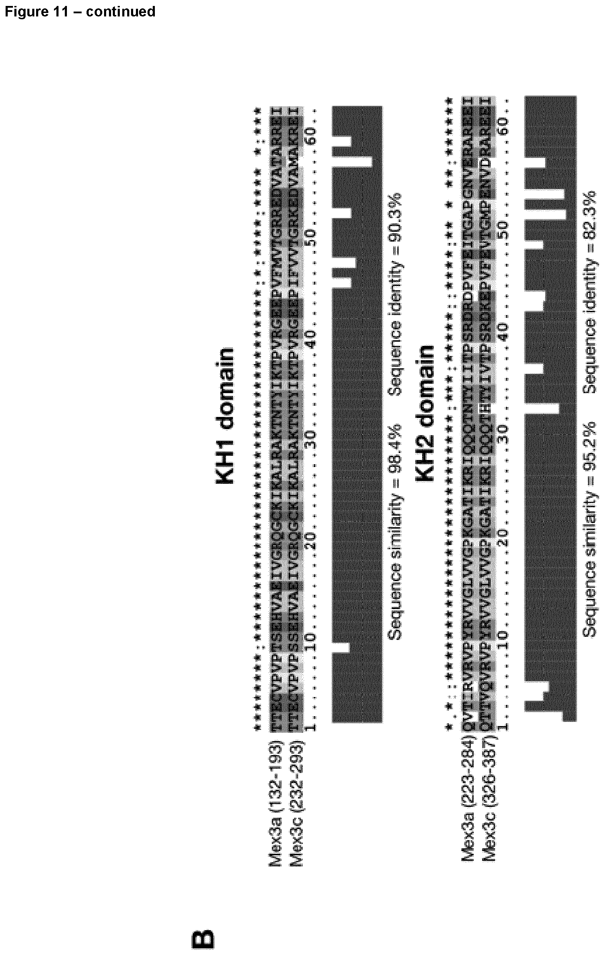

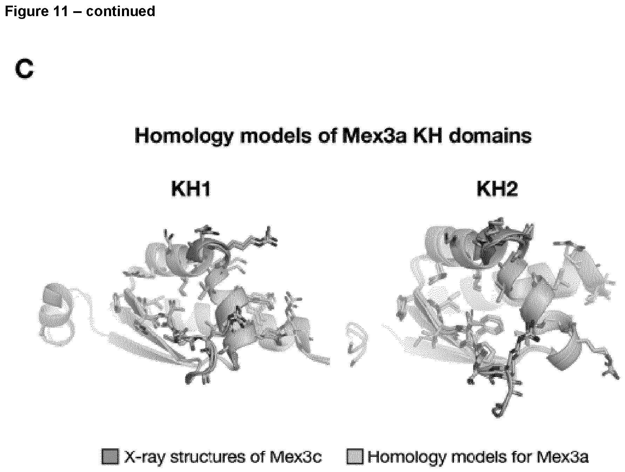

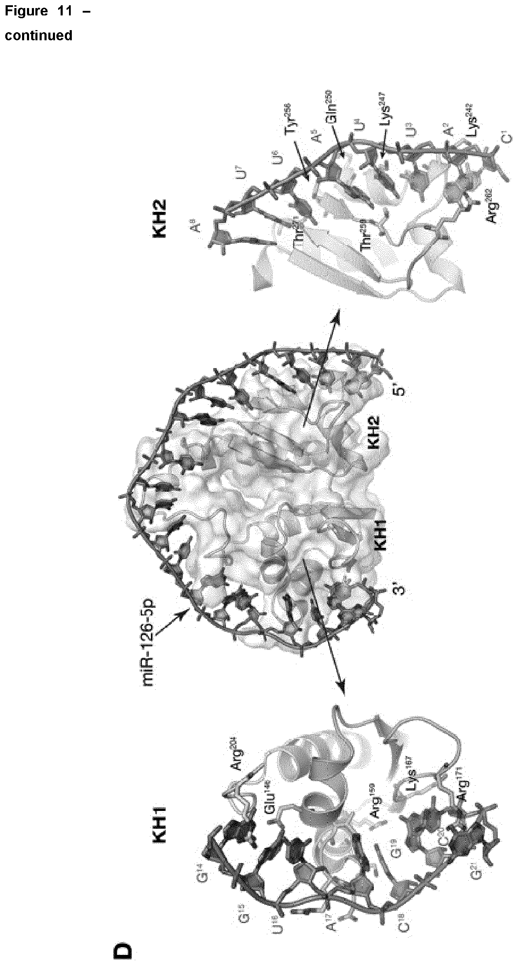

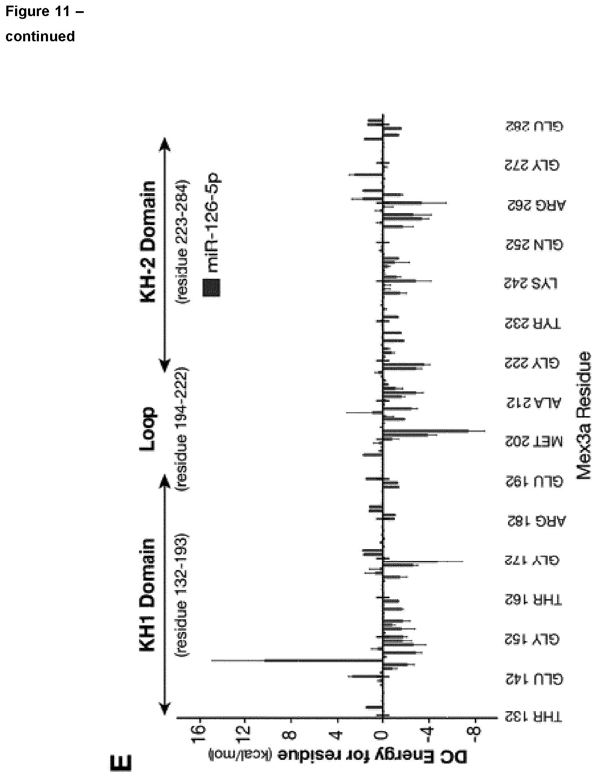

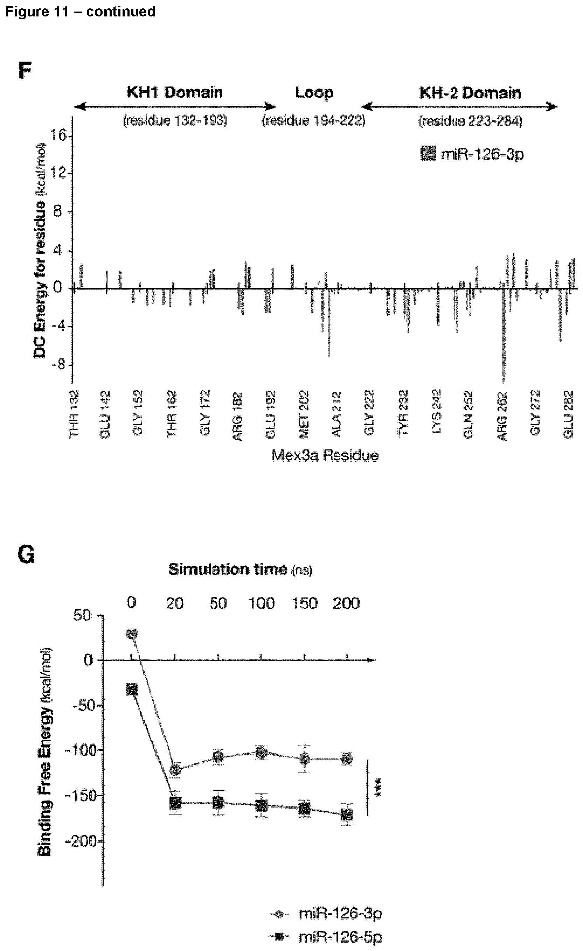

- **Fig. 11.**: Basis for computational modelling for generation of homology models for Mex3a KH domains and molecular dynamics simulation of interactions with miR-126. **A,** Experimental solution small-angle X-ray scattering (SAXS) analysis of Mex3a(^{KH1KH2}) unbound (blue) or bound to miR-126-5p (red). **B,** Sequence alignment of the KH domains of Mex3a and Mex3c. Identical residues are annotated by an asterisk, whereas similar or lightly similar residues are marked with two (:) or one (.) dot, respectively. **C,** Comparison between the homology models for KH1 and KH2 domains in Mex3a (cyan) and the X-ray crystallography structures of the homologous domains in Mex3c (green, PDB codes: 5WWW; 5WWX). **D,** Snapshot of the molecular docking model after refining by molecular dynamics (MD) simulation (200 ns) showing the predicted binding mode of miR-126-5p with the Mex3a KH domains homology model derived from co-crystallized structures of Mex3c and Nova1 (PDB codes: 5WWW, 5WWX, 2ANR). The model includes KH1 (green) and KH2 (cyan) domains as well as the loop connecting them (see Methods for details). **E,F,** Computed decomposition (DC) energies per residue for the interaction between the Mex3a KH domains homology model and miR-126-5p (**E**) or miR-126-3p (**F**). **G,** Binding free energy (BFE) for the interaction of the miR-126 strands with the Mex3a KH domains homology model at different time points of MDS. Lower BFEs were computed for miR-126-5p, suggesting higher affinity and stability of the interaction. Data are shown as mean ± s.d. ****P*<0.001. Paired t-test (**G**).

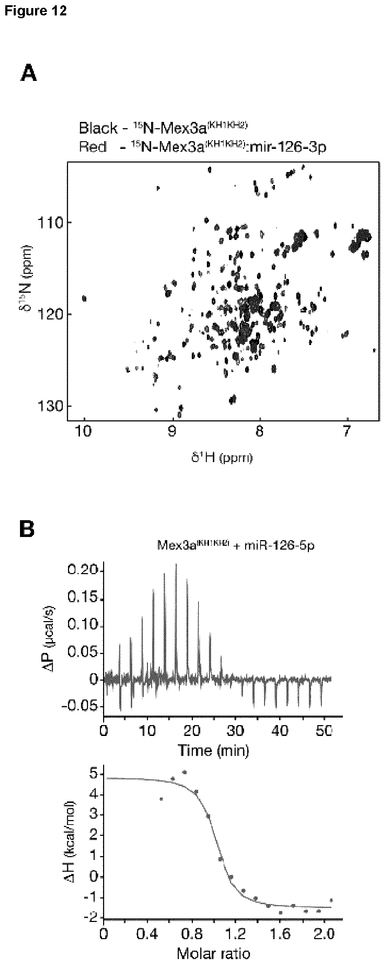

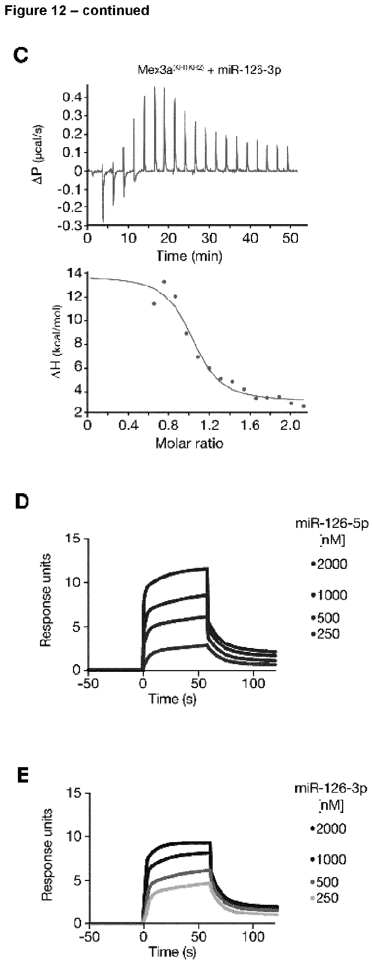

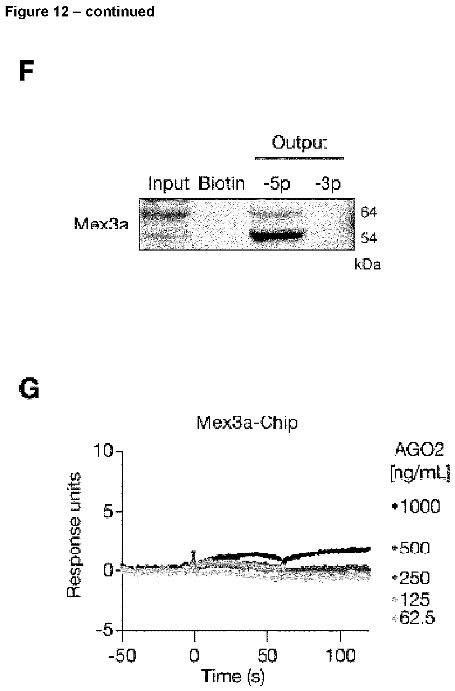

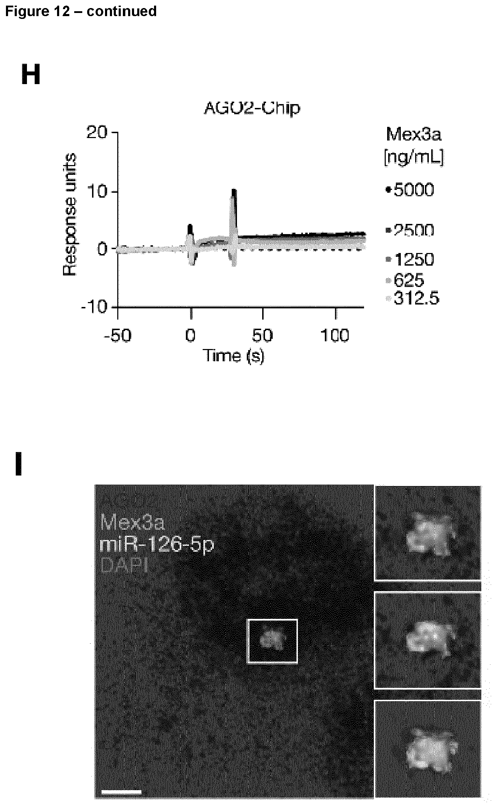

- **Fig. 12.**: Mex3a preferentially binds miR-126-5p. **A,** NMR spectroscopic analysis of miR-126-3p interaction with the KH domains of Mex3a [Mex3a^{(KH1KH2)}]. 2D HSQC NMR spectra of ¹⁵N-Mex3a^{(KH1KH2)} in the absence (black cross-peaks) or presence (red cross-peaks) of miR-126-3p (molar ratio, 1:2). **B,C**, Isothermal titration calorimetry (ITC) binding curves of Mex3a^{(KH1KH2)} binding to miR-126-5p (**B**) or miR-126-3p (**C**). The thermograms resulting from the titration are shown in the top panel, and heat changes are plotted as a function of the molar miR-126/protein ratio in the bottom panel. Please note that binding curves indicate a more complex model with the conformational rearrangement upon miR-126-5p binding, as shown by SAXS (Fig.4C). **D,E,** Surface plasmon resonance (SPR) sensorgrams for interaction between immobilized full-length Mex3a and miR-126-5p (**D**) or miR-126-3p (**E**). **F,** Biotin-RNA pull-downs using miR-126 strand sequences in nuclear extracts of rapamycin-treated HUVECs showing interaction with Mex3a. **G,** SPR sensorgrams for AGO2 perfused at indicated concentrations on immobilized full-length Mex3a in absence of miR-126-5p, revealing no detectable interaction. **H,** SPR sensorgrams for full-length Mex3a perfused on AGO2 in the absence of miR-126-5p. **I,** Representative confocal microscopy image of co-immunostaining for AGO2, Mex3a and fluorescence *in situ*-hybridization with miR-126-5p in the nucleus of rapamycin-treated HUVECs.

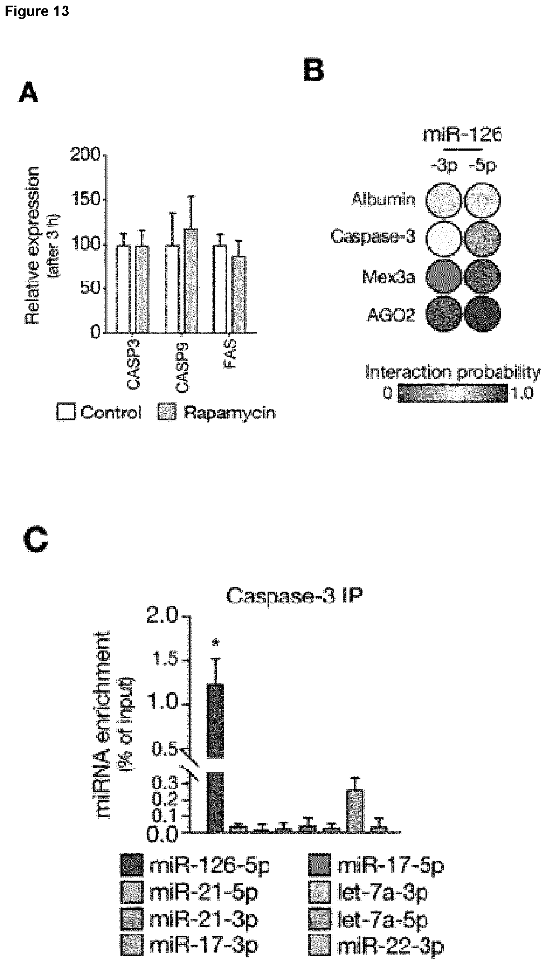

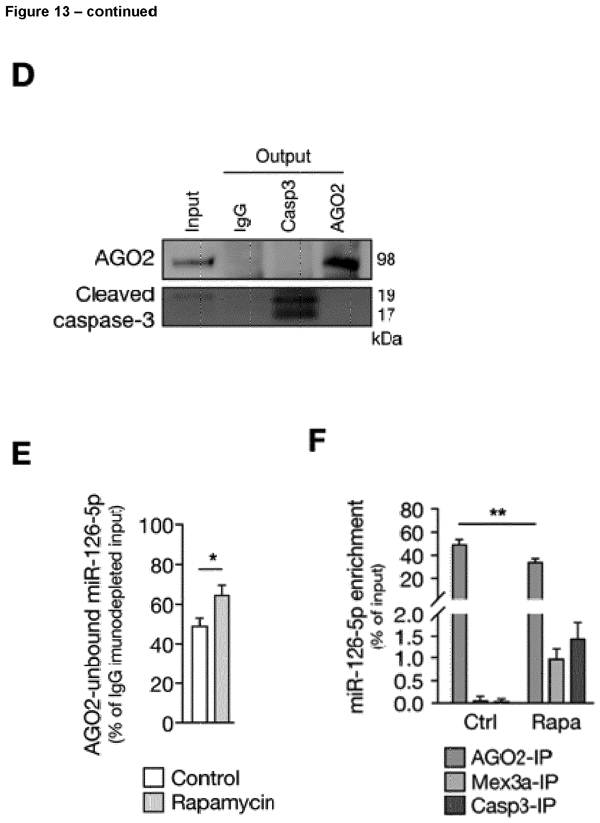

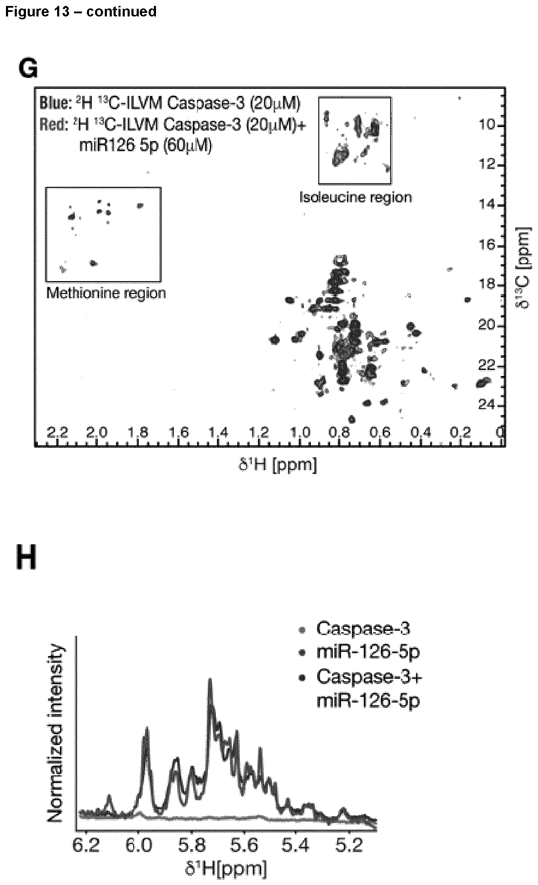

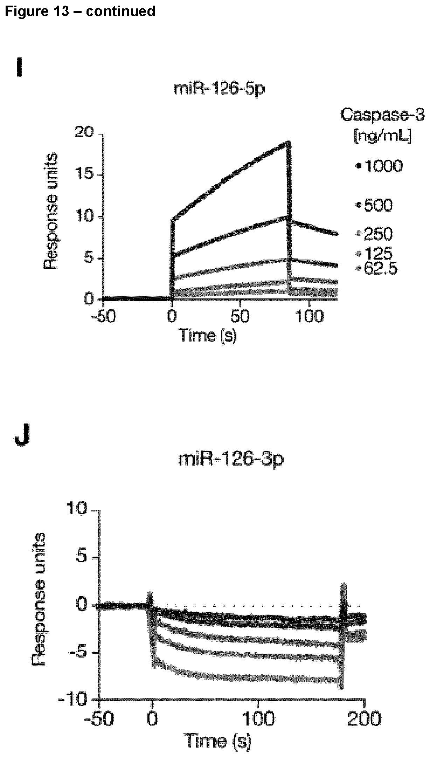

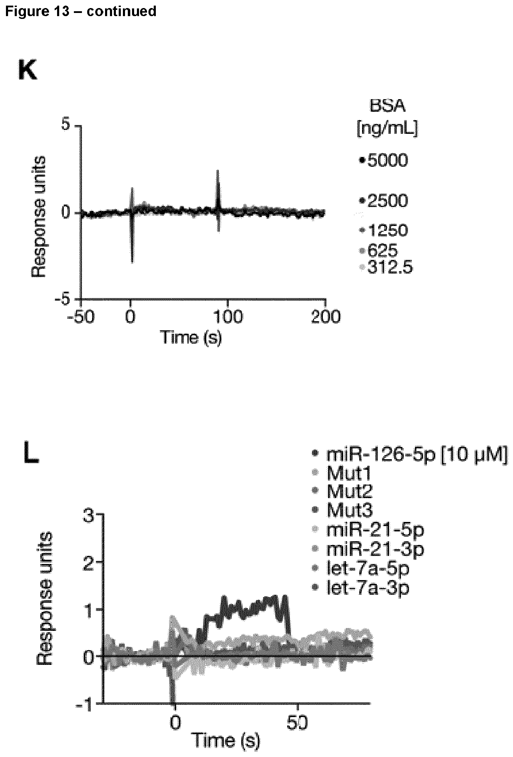

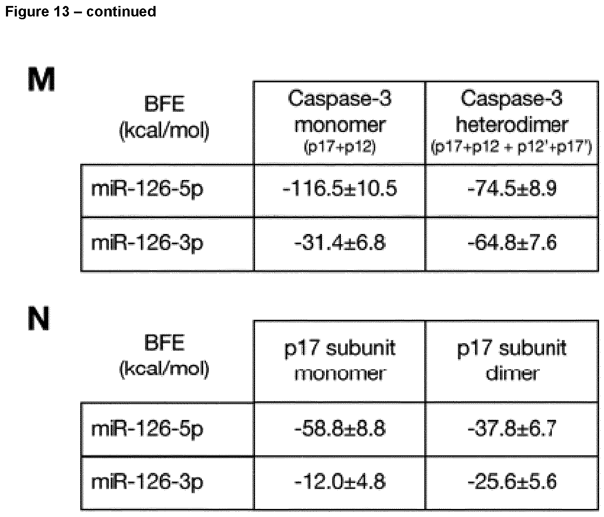

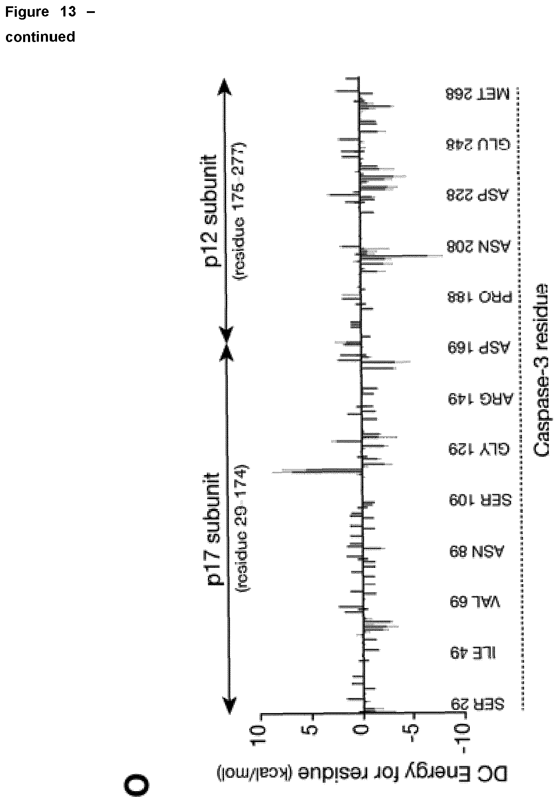

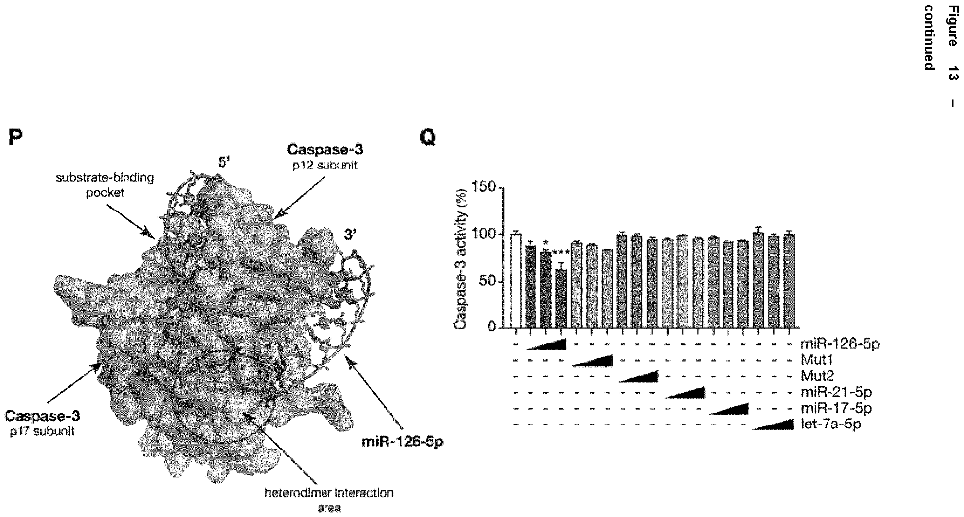

- **Fig. 13.**: miR-126-5p interacts with caspase-3. **A,** Expression of transcripts involved in apoptosis in HUVECs after 3 h of rapamycin treatment (n=6). **B,** Probability of interaction of miR-126 strands with the enlisted proteins computed applying a support vector machine classifier based on sequences (RPIseq-SVM). Albumin and AGO2 are shown as negative and positive controls for interaction, respectively. **C,** RNA immunoprecipitation of nuclear caspase-3 in rapamycin-treated HUVECs showing loading of miR-126-5p and other ECs miRNAs (n=2). **D,** Immunoblot for AGO2 and caspase-3 immunoprecipitates from nuclear lysates of rapamycin-treated HUVECs showing no interaction. **E,** Proportion of miR-126-5p not bound to AGO2 as assessed by qPCR on AGO2-immunodepleted nuclear lysates of rapamycin-treated HUVECs and untreated controls (n=4). Data are normalized to IgG immunodepleted nucleus and enrichment in AGO2 immuno-precipitates. **F,** RNA immunoprecipitation of nuclear AGO2, Mex3a, and caspase-3 in rapamycin-treated HUVECs and controls (n=4-6). **G.** Overlay of ¹H-¹⁵N HSQC spectra of caspase-3 in absence (blue cross-peaks) or presence (red) of miR-126-5p (molar ratio 1:2). **H,** Superposition of two-dimensional NMR spectra of ¹H¹³C methyl ILVM, ²H-labeled caspase-3 (blue) and in complex with miR-126-5p (red). A magnification of the isoleucine region is shown in Fig.6G. **I-K,** Surface plasmon resonance (SPR) sensorgrams for interaction between caspase-3 (p17 subunit) and immobilized biotinylated miR-126-5p (**I**) or miR-126-3p (**J**). K_{D(miR-126-5p:casp3)}: 1.95±0.3 µM. Bovine serum albumin (BSA) is shown as a negative control without interactions on immobilized miR-126-5p (**K**). **L,** SPR sensorgrams for interaction between immobilized caspase-3 (p17 subunit) and miR-126-5p, miR-126-5p mutants, and other ECs miRNAs. **M,N,** Binding free energy (BFE) derived from MDS for the interaction between miR-126 strands and caspase-3 monomer and heterodimer (**M**) or monomer and dimer for p17 subunit (**N**). Lower BFEs were computed for miR-126-5p interaction with monomers. **O,** Computed decomposition (DC) energies per residue for the interaction between the caspase-3 and miR-126-5p. **P,** Snapshot of the molecular docking model after refining by MDS (20 ns) showing the predicted binding mode of miR-126-5p with caspase-3 heterodimer (PDB code: 2DKO). The model includes subunit p12 (cyan) and p17 (green). **Q,** Cell-free analysis of caspase-3 activity in presence of miR-126-5p, miR-126-5p mutant (Mut1, Mut2), miR-21-5p, miR-17-5p, and let-7a-5p (4, 8 or 16 µM) (n=3-6). **P*<0.05, ***P*<0.01, ****P*<0.001. Unpaired Student's t-test with step-up correction for false discovery rate (**A,E**), univariate (**C**) or two-factor paired measures ANOVA (**F,Q**) with Bonferroni *post-hoc* test.

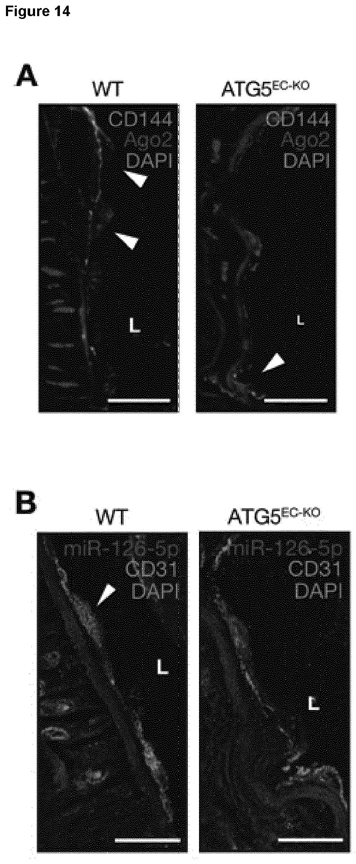

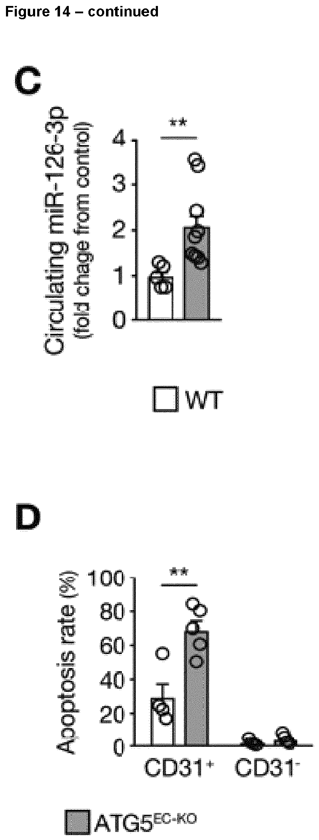

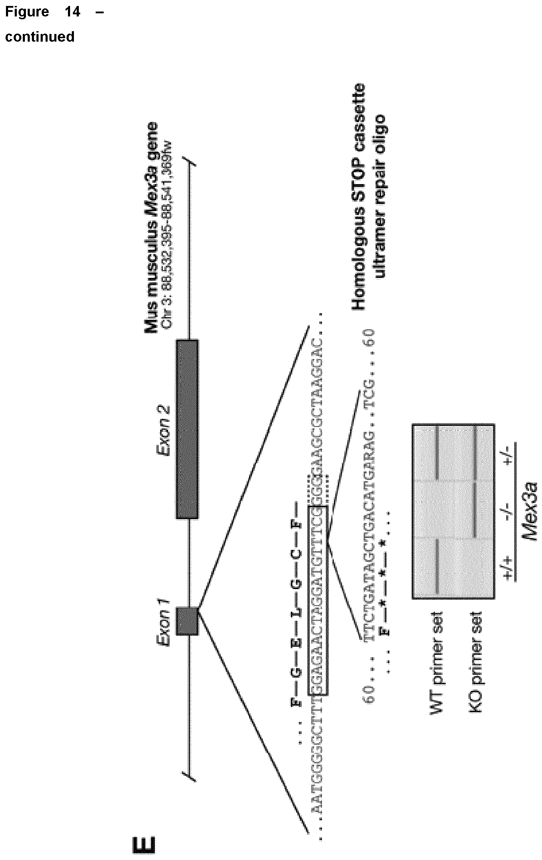

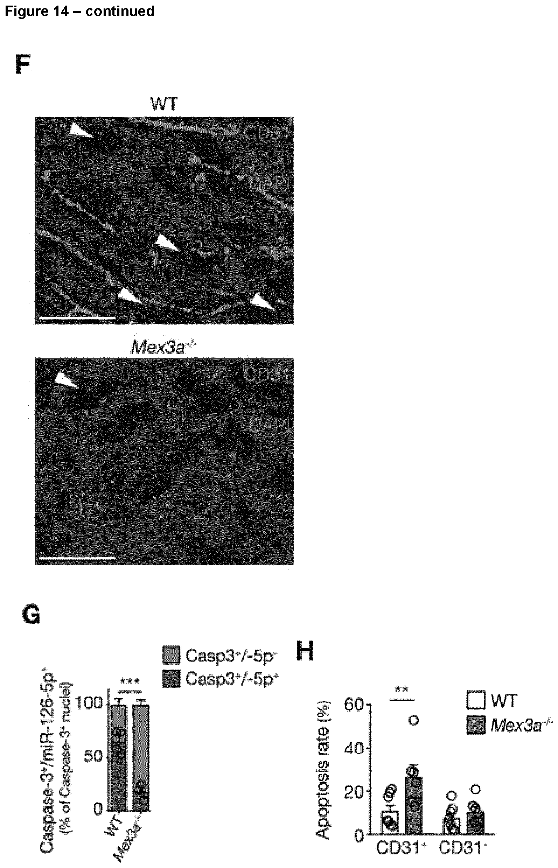

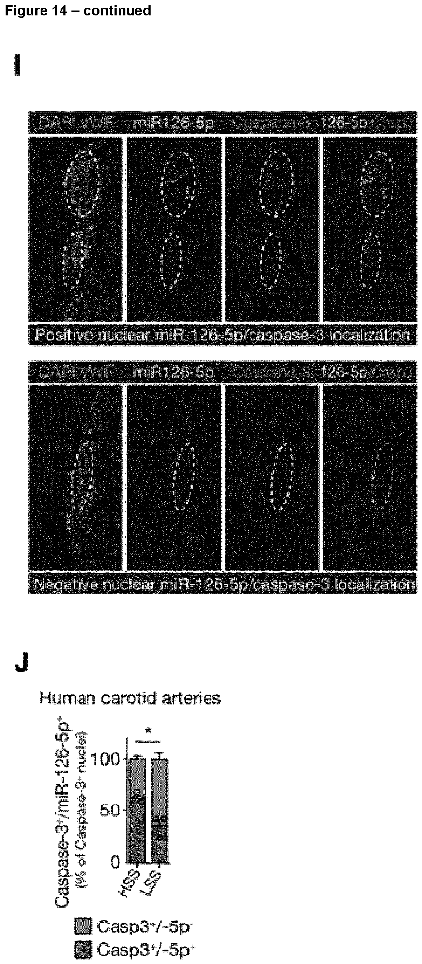

- **Fig. 14.**: Impeding nuclear Ago2:miR-126-5p transfer promotes apoptosis *in vivo.* **A,B,** Representative images for Ago2 immunostaining (**A**) and in situ PCR for miR-126-5p (**B**) in longitudinal sections of thoracic aorta of *Apoe*^{*-*/}*⁻Cdh5^{Cre-}Atg5*^{*fl*/*f*/} (wild-type, WT) and *Apoe*^{*-*/}*⁻Cadh5^{cre+}AtgS*^{*fl*/*fl*} (ATG5^{EC-KO}) mice fed a high-fat diet for 12 weeks. White arrowheads mark ECs with nuclear signals for Ago2 and miR-126-5p, respectively. CD144 and CD31 are applied as endothelial markers. Quantification is provided in Fig.7K,L (n=3). L, lumen. Scale bar, 20 µm. **C,** Relative quantification of miR-126-3p in plasma circulating vesicles of WT and ATG5^{EC-KO} mice fed a high-fat diet for 12 weeks (n=5-9). **D,** Apoptosis rate as assessed by flow cytometry for annexin-V on CD31⁺ (ECs) and CD31⁻ cells from lungs of WT and ATG5^{EC-KO} mice fed a high-fat diet for 12 weeks (n=4-5). **E,** Strategy applied for Mex3a silencing in vivo with the CRISP-Cas9 genome editing. A homologous STOP cassette ultramer repair oligo was applied in the Exon 1 of murine Mex3a (see Materials and Methods for details); *denotes STOP codon. **F,** Representative 3D deconvoluted images for AGO2 immunostaining on *en-face* thoracic aortas of wild-type C57BL/6 (WT) or *Mex3a*^{*-*/*-*} mice. Quantification is provided in Fig. 7Q (n=4). Scale bar, 20 µm. **G,** Percentage of ECs with nuclear caspase-3 staining which show concomitant miR-126-5p signals (Casp3⁺/-5p⁺) in thoracic aorta of WT and *Mex3a*^{*-*/-} mice, as assessed by FISH for miR-126-5p with co-immunostaining for caspase-3 (see also Fig.7R-T). **H,** Apoptosis rate as assessed by flow-cytometry for annexin-V on CD31⁺ (ECs) and CD31⁻ cells from lungs of WT and *Mex3a*^{*-*/*-*} (n=6-7). **I,** Representative deconvoluted images for fluorescent in situ hybridization for miR-126-5p and co-immunostaining for caspase-3 and von Willebrand factor (vWF, applied as endothelial marker) in human carotid artery specimens. Representative ECs with positive nuclear signals for miR-126-5p and caspase-3, as evident in HSS areas (upper panel), or with absence of nuclear miR-126-5p, typically occurring in LSS areas (lower panel). Quantification in Fig.7U,V. Dashed lines delimitate nuclear areas. **J,** Percentage of ECs with nuclear caspase-3 staining which show concomitant miR-126-5p signals (Casp3⁺/-5p⁺) in human carotid artery samples in areas upstream (HSS) or downstream (LSS) of the bifurcation (n=3). **K,** Synopsis of the pathway: Activation of autophagic flux by HSS, KLF2 or mTOR-inhibition favors the assembly of a ternary complex of miR-126-5p with Mex3a and AGO2 (1) leading to its nuclear transfer (2). In the nucleus, miR-126-5p dissociates from AGO2 and Mex3a to bind the effector caspase-3, which can be transferred to the nucleus when activated. This direct interaction inhibits caspase-3 catalytic activity (3), thus reducing apoptotic cell death and preserving ECs viability to protect against atherosclerosis. Data are shown as mean ± s.e.m. *P<0.05, **P<0.01, ***P<0.001. Mann-Whitney U-test (**C**) and factorial ANOVA with Bonferroni *post-hoc* test (**D,G,H,J**).

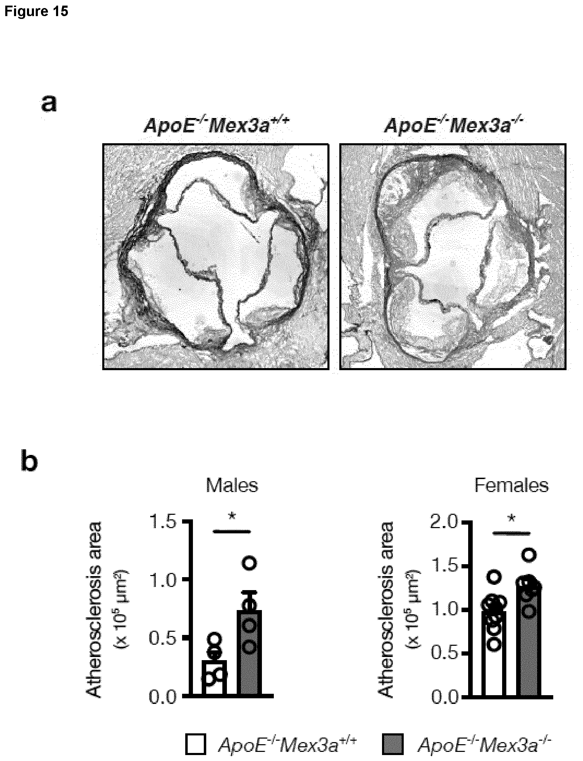

- **Fig. 15**: Mex3a deficiency is associated with atherosclerosis. **a,b,** Atherosclerosis area was evaluated in the aortic root of WT (*ApoE*^{*-*/}*⁻Mex3a*^{*+*/*+*}) and KO *(ApoE*^{*-*/}*⁻Mex3a^{-l-})* mice (**a**) and quantification is provided for male (n= 4) and female (n= 7-9) mice (**b**).

The Examples illustrate the invention.

### Example 1 - High shear stress and autophagy promotes nuclear miR-126-5p enrichment

Beyond shaping gene expression by guiding argonaute (AGO) proteins to target RNA transcripts in the RNA-induced silencing complex (RISC), it remains unclear whether miRNAs can also regulate cell functions by additional mechanisms. To exert their post-transcriptional activity, microRNAs (miRNAs) require multiple components (*e.g.* TNRC6A) in high-molecular weight RISCs to allow for interaction of the miRNA with the 3'UTR of target mRNAs and with enzymes mediating inhibition of translation or RNA decay *(1, 2).* Beyond such prototypic effects, miRNAs can be retained in low-molecular weight (LMW) complexes with AGO2 not bound to mRNA or engaged in target repression. Although viewed as a miRNA reservoir, it is unknown and debatable whether these complexes may enable interactions of hosted miRNAs with proteins not involved in the canonic functions of the RISC, whether these miRNAs can thereby exert direct post-translational effects on protein activities and how this is related to their intracellular trafficking.

Assembly of LMW complexes is controlled by mammalian target of rapamycin (mTOR) complex I and prevail upon mTOR-inhibition *(3-5).* Inhibition of mTOR is amid the most powerful triggers of autophagy, a catabolic process used by eukaryotic cells for adapting to stress, for maintaining nutrient homeostasis, and for mediating quality control and lysosomal degradation of damaged organelles and protein aggregates *(6, 7).* In endothelial cells (ECs), autophagic flux is triggered via the mTOR-pathway by high-shear stress (HSS) and is impaired at predilection areas for atherosclerosis *(8)* but the mechanisms by which laminar shear stress and autophagy regulate miRNA fate and function in this context are poorly understood.

The Krüppel-like family of transcription factors (KLFs) is an important regulator of autophagy, which has emerged as a rheostat of the miRNA-processing machinery to control mature miRNA expression *(9-12).* KLF2 coordinates most of the transcriptional programs evoked by shear stress in arterial ECs and is required for blood flow-dependent miR-126 expression in zebrafish *(12-15).* Amongst the most highly expressed endothelial miRNAs, the miR-126 duplex is instrumental for angiogenesis and vascular integrity featuring strand-specific functions and homeostasis *(16-19).* Whereas miR-126-3p mediates pro-angiogenic and anti-inflammatory signaling, miR-126-5p maintains the EC proliferative reserve, modulating distinct targets and pathways *(16, 17, 19-22).* In line with differential functions, only miR-126-5p is up-regulated by laminar flow in arterial ECs to limit atherosclerosis in areas of HSS *(16).* Although both strands are processed from a common precursor by the endoribonuclease Dicer, their arterial quantities are not affected by EC-specific Dicer deletion *(23).* Thus, the differential regulation of miR-126 strands does not rely on transcription or processing, leaving underlying mechanisms such as shear stress-elicited autophagy to be elucidated.

In an effort to better understand the interplay of laminar shear stress and autophagy in controlling the expression of miRNAs, namely miR-126, KLF2 was overexpressed in human umbilical vein ECs, showing increased pre-miR-126 and miR-126-5p but not miR-126-3p expression (Fig.1A; Fig.8A-C). Accordingly, lower amounts of miR-126-5p were detected but not of miR-126-3p in the aortic intima of *Cdh5^{Cre}Klf2*^{*fl*/*fl*} mice with EC-specific *Klf2* deletion (Fig.1B), thus implicating mechanisms of post-transcriptional strand regulation. Notably, KLF2 overexpression and HSS (12 dyne/cm²) increased autophagy-related gene (ATG) transcripts and promoted autophagic flux, as indicated by the conversion of LC3-I to autophagosome-associated LC3-II and by the reduction of the autophagy substrate p62 at the protein but not at the transcript level (Fig.1C,D; Fig.8D-H). It was next scrutinized whether autophagy is involved in the differential regulation of miR-126 strands. Eliciting autophagy with the mTOR inhibitor rapamycin in ECs reduced the amount of miR-126-3p, whereas that of miR-126-5p was preserved (Fig.1E; Fig.9A-C). This was not due to extracellular release, as shedding and miR-126-3p content of apoptotic bodies (ABs) were attenuated (Fig.9D-F), but rather due to autophagy-related degradation activity, as shown by silencing of ATG5 or ATG7, or by blocking autophagosome/lysosome fusion with bafilomycin, which restored miR-126-3p amounts (Fig.1E).

Since the localization to subcellular compartments is pivotal to miRNA function (2, 3), it was probed whether this segregation also accounts for regulating miR-126 levels. Transfecting fluorescently-tagged mimics revealed a prominent nuclear localization enhanced by rapamycin for miR-126-5p but not for miR-126-3p (Fig.1F-H). Fractional qPCR confirmed rapamycin-induced nuclear enrichment of endogenous miR-126-5p (Fig.1I; Fig.9G,H). Notably, silencing of ATG5 and ATG7 or using the α/β-importin inhibitor ivermectin prevented nuclear miR-126-5p accumulation (Fig.1J). Finally, limiting autophagy by 3-methyladenine blunted the KLF2-driven increase in nuclear miR-126-5p (Fig.1K). Likewise, exposure of ECs to physiological laminar shear stress (12 dyne/cm²) *(24)* revealed a nuclear enrichment of miR-126-5p when compared to static conditions, exposure to low shear stress (LSS ,1 dyne/cm²) or to shear stress exceeding the physiological range (30 dyne/cm²) (Fig.9I). Taken together, autophagic flux triggered by KLF2 or mTOR-inhibition governs trafficking of miR-126 strands, preserving miR-126-5p by nuclear transfer.

Studies employing compartment-specific microinjection found synthetic miR-21 and let-7a to shuttle between nucleus and cytoplasm *(25);* however, effects of rapamycin on nuclear amounts of let-7a-3p/-5p, miR-21-3p/-5p were not detected, nor the miR-17 duplex with two functional strands (26) (Fig.2A). Hence, autophagy-driven nuclear enrichment is not a general mechanism pertaining to all miRNA strands but preferentially affects miR-126-5p, although it lacks the hexanucleotide AGUGUU motif known to facilitate nuclear enrichment *(27).* Availability or subcellular distribution of strand-specific target transcripts, which can influence nuclear shuttling (25), were unaltered by rapamycin or KLF2 overexpression (Fig.9J-L). To explore sequence determinants for nuclear translocation, mutational changes were assessed in fluorescent synthetic miR-126-5p. While the seed-sequence required for canonical binding of RNA targets *(2)* was dispensable for rapamycin-promoted nuclear shuttling (Mut1, Mut2), more extensive mutation of nucleotides spanning to the 3'-end (Mut3) abrogated nuclear enrichment (Fig.2B-D). Thus, the specificity of nuclear miR-126-5p translocation does not rely on its seed sequence or seed-matched transcripts but requires other sequence motifs.

### Example 2 - Nuclear translocation of miR-126-5p requires the RNA-binding protein Mex3a

Stabilization by AGO proteins contributes to the nuclear persistence of shuttled miRNAs, and AGO2 is capable of shuttling and indispensable for unwinding and strand selection *(3, 25, 28).* Although the overall amount of AGO2 was unaltered (Fig.10A), it was found that rapamycin elicited nuclear accumulation of AGO2 and LC3 (showing colocalized signals by immunofluorescence), which was blocked by ivermectin (Fig.3A; Fig.10B). RNA-immunoprecipitation (IP) verified preferential binding of miR-126-5p to nuclear AGO2 (Fig.3B). At the nanoscopic level, AGO2 was detectable on the outer surface rather than in the lumen of LC3-demarcated vesicles in rapamycin-treated ECs (Fig.3C). Moreover, autophagosomes were isolated by density-gradient centrifugation to employ a protease protection assay, based on the principle that components included inside the autophagosome will resist digestion by proteases *(29).* Indeed, incubation of isolated autophagosomes with trypsin dose-dependently decreased the amount of AGO2 but not that of LC3-II (which is also bound to the intraluminal membrane), thus further supporting the localization of AGO2 on the extraluminal surface (Fig.10C). This complies with evidence that AGO2 binds to intracellular membranes, including those likely involved in phagophore elongation (30), and could explain why AGO2:miR-126-5p complexes are preserved from degradation. Together, our data support the concept that sorting of AGO2:miR-126-5p complexes on autophagosomal surfaces promotes nuclear translocation.

How does autophagy drive nuclear enrichment? In T cells, mTOR controls the assembly of LMW miRNA:AGO2 complexes, which represent a cellular miRNA reservoir and the prevalent nuclear form *(3-5, 31).* Notably, size exclusion chromatography in ECs revealed that mTOR inhibition shifted AGO2 co-elution towards LMW complexes, which prevail in nuclear fractions and were devoid of the RISC protein TNRC6A (Fig.10D,E), implying that autophagy shapes AGO2 interactions by degrading RISC components to favor nuclear transfer. Hence, AGO2-IP was performed to identify interacting proteins. Despite the contiguity observed in Fig.3A,B, LC3 was not detected in AGO2 immunoprecipitates of rapamycin-treated cells nor were the nuclear AGO2 navigators TNRC6A and importin-8 *(32-34)* (Fig.3D), thus excluding their involvement. Among AGO2-interacting proteins capable of nuclear-cytoplasmic shuttling, Mex3a, a member of the RNA-binding Mex3 family, forms RNA-mediated bonds in P-bodies *(35, 36).* Co-IP revealed interactions of AGO2 and Mex3a in both cytoplasm and nuclei, and this was validated by confocal microscopy of autophagic ECs (Fig.3D,E). Mex3a was degraded by trypsin in the autophagosome protection assay and co-eluted with AGO2 in LMW complexes, suggesting that it localizes on the outer autophagosome membrane to mark and facilitate AGO2-complexes suited for nuclear translocation (Fig.10C-E). Conversely, it was failed to detect interactions with Mex3c or the P-body core-protein decapping protein 1a (DCP1a), indicating a role of Mex3a unrelated to P-bodies (Fig.3D). Using STED nanoscopy to detail this complex confirmed co-localizing signals for AGO2 and Mex3a, whereas LC3 signals showed only proximity (Fig.3F). In addition, rapamycin reduced cytoplasmic Mex3a, increasing its nuclear localization in the vicinity of LC3 (Fig.10F-H).

### Example 3 - Mex3a preferentially binds to miR-126-5p

Next, RNA binding of Mex3a was characterized, which is imparted by two KH-domains *(35).* Nuclear magnetic resonance (NMR) titrations indicated that the heterogeneous tandem KH-domains bind to miR-126-5p consistent with submicromolar affinity (Fig.4A). Surface plasmon resonance (SPR) confirmed high-affinity binding of full-length Mex3a to immobilized miR-126-5p (Fig.4B). To verify how a structural accommodation of miR-126-5p binding by both KH domains can occur, small-angle X-ray scattering (SAXS) was performed to show that the free KH1 and KH2 tandem domains are flexibly connected resembling pearls on a string, whereas they form a compact arrangement when bound to miR-126-5p (Fig.4C; Fig.11A). Next, homology models were computed based on the co-crystal structures of individual Mex3c KH-domains with RNA *(37)* (Fig.11B-D). These models imply a mode of interaction where the two KH-domains recognize nucleotides U₃-U₆ and G₁₅-C₁₈ in miR-126-5p. Estimated binding-free energies (BFE) indicated preferential binding of the 5p *vs.* 3p strand (Fig.4D; Fig.11E-G), which was consistent with reduced spectral changes in NMR titrations (Fig.12A). Preferential binding of miR-126-5p was experimentally confirmed by SPR on immobilized Mex3a, by isothermal titration calorimetry (ITC), and by pull-down of biotinylated miR-126 strands after exposure to nuclear lysates (Fig.4D; Fig.12B-F). Consistently, RNA-IP of nuclear Mex3a in rapamycin-treated ECs unveiled a preferential enrichment of miR-126-5p (Fig.4E). Notably, SPR revealed that AGO2:Mex3a interactions only occur in the presence of miR-126-5p, either immobilized or in solution, indicating privileged binding by ternary interactions in a complex (Fig.4F,G, Fig.12G,H). In the nucleus, the presence of a ternary complex was visualized by fluorescence *in situ*-hybridization (FISH) for miR-126-5p and co-immunostaining for Mex3a and AGO2 (Fig.12I). Lastly, silencing Mex3a abrogated the rapamycin-induced AGO2 and LC3 accumulation (Fig.4H; Fig.10I) and miR-126-5p enrichment in EC nuclei (Fig.4I). Together, our findings establish that nuclear shuttling of a miR-126-5p:AGO2 complex is mediated by a Mex3a-dependent process.

### Example 4 - Nuclear miR-126-5p exerts anti-apoptotic properties

Nuclear AGO2:miRNA complexes have been described to affect the transcriptome via diverse mechanisms *(3, 25, 33, 38).* Hence, transcriptional effects of nuclear miR-126-5p transfer were assessed by evaluating changes in gene expression after short-term exposure to rapamycin combined with gain- and loss-of-function for miR-126-5p. Expression of multiple transcripts involved in EC biology were altered and the functional annotation for those undergoing concordant regulation were analyzed with both rapamycin and miR-126-5p mimics (Fig.5A). Because KEGG pathway analysis enrichment revealed predominant effects on transcriptional profiles of apoptosis-related pathways (Fig.5B), apoptosis mediators were assessed at the protein level. Overexpression of miR-126-5p only slightly attenuated caspase-3/9 expression but markedly reduced the active form of caspase-3 and cleavage of its substrates, an effect which required the presence of rapamycin to induce nuclear transfer of miR-126-5p, and was not observed upon miR-126-5p inhibition (Fig.5C). Conversely, impeding nuclear enrichment of miR-126-5p by Mex3a knock-down increased caspase-3 cleavage (Fig.5D). A reduction in EC apoptosis after rapamycin treatment was prevented by silencing miR-126-5p or Mex3a (Fig.5E,F). Likewise, anti-apoptotic effects mediated by Mex3a overexpression were impaired by miR-126-5p silencing (Fig.5G).

### Example 5 - Direct interaction with caspase-3 conveys anti-apoptotic effects of miR-126-5p

Anti-apoptotic effects occurred rapidly upon rapamycin and preceded transcriptional changes in relevant mediators (Fig.5F; Fig.13A), leading us to postulate direct anti-apoptotic properties of miR-126-5p. As an effector caspase, caspase-3 acts downstream in the apoptotic cell death cascade and can translocate to the nucleus after proteolytic activation and substrate recognition (39). Therefore, potential interactions with the miR-126-5p complex were tested. Applying a support vector machine classifier based on sequences of caspase-3 and miR-126-5p (RPISeq-SVM), a probability of interaction was derived, which was higher than miR-126-3p (Fig.13B). Similarly, molecular dynamics simulation (MDS) supported a preferential binding of miR-126-5p compared to other abundant endothelial miRNAs. Experimentally, FISH acquired by confocal microscopy showed co-localizing signals for miR-126-5p and caspase-3, particularly in nuclear and perinuclear areas, and this co-localization was reduced by Mex3a knock-down (Fig.6A-C). RNA-IP demonstrated a marked enrichment of miR-126-5p (but not other miRNAs) associated with nuclear caspase-3 upon rapamycin treatment or under conditions of HSS (Fig.6D, Fig.13C). While no interaction of caspase-3 and AGO2 was detectable in co-IPs (Fig.13D), caspase-3 and Mex3a were detected using pull-down of biotinylated miR-126-5p from nuclear extracts of rapamycin-treated ECs (Fig.6E). Indeed, analysis of nuclear AGO2-immunodepleted RNA fractions showed that a relevant fraction of the nuclear miR-126-5p pool is not associated with AGO2 and RNA-IP confirmed its interaction with Mex3a or caspase-3 in rapamycin-treated ECs (Fig.13E,F). The co-localization of caspase-3 with fluorescently tagged synthetic miR-126-5p was reduced by partial (C₅-C₈) or full disruption of the seed sequence (Mut1, Mut2) or more extensive mutation (Mut3) (Fig.6F). The interaction of recombinant caspase-3 and synthetic miR-126-5p was confirmed by NMR titrations *in vitro.* Significant spectral changes of caspase-3 methyl-signals upon addition of miR-126-5p (Fig.6G; Fig.13G) as well as RNA signals upon addition of caspase-3 (Fig.13H) demonstrated direct binding. SPR on immobilized miR-126-5p revealed a K_{D} of 1.95±0.30 µM for caspase-3 (Fig.13I-K). SPR on immobilized caspase-3 p17 subunit revealed binding of miR-126-5p but failed to detect responses with other miRNAs or miR-126-5p mutated in the seed sequence (Mut1-3), corroborating the specificity of miR-126-5p (Fig.13L).

To verify that miR-126-5p can dissociate from AGO2 in favor of caspase-3, competition assays on immobilized miR-126-5p were performed, showing that caspase-3 displaces AGO2 binding to miR-126-5p (Fig.6H). Computational modelling further indicated that miR-126-5p may engage in interactions with the substrate-binding pocket and the heterodimer interface of caspase-3. The computed BFEs *in silico* suggested a privileged binding of miR-126-5p to caspase-3 monomers rather than heterodimers (Fig.13M-P). Accordingly, SPR revealed that dimerization of caspase-3 p17 subunits was dose-dependently disrupted by miR-126-5p (Fig.6I). As dimerization of caspase-3 is critical for proper assembly of its active site *(40),* its functional role was explored. In cell-free assays, miR-126-5p dose-dependently reduced substrate cleavage by recombinant caspase-3 with an IC₅₀ of 14.4 µM comparable to inhibition with Z-VAD-FMK (IC₅₀ 75.9 µM) or Z-DVED-FMK (IC₅₀ 2.8 µM) (Fig.6J), supporting the notion that miR-126-5p interferes with both binding pocket and heterodimer-driven active site formation. In contrast, seed-mutated miR-126-5p (Mut1-3), miR-126-3p, let-7a-5p, miR-17-5p and miR-21-5p were ineffective (Fig.6J; Fig.13Q). Collectively, our data establish that nuclear miR-126-5p guided by Mex3a and after its dissociation from AGO2 can protect ECs from apoptosis by direct inhibition of caspase-3.

### Example 6 - Nuclear miR-126-5p:Mex3a pathway is operative in vivo and in human disease

To provide *in vivo* evidence for the relevance of the autophagy-driven pathway in vascular disease, endothelial staining patterns for LC3 *en face* in aortas of atherosclerosis-prone *Apoe*^{*-*/*-*} mice were analyzed by 3D-deconvolution immunofluorescence. LC3-staining was more prevalent in areas exposed to HSS (*i.e.* thoracic aorta) vs. LSS *(i.e.* the aortic arch as a predilection site) (Fig.7A,B). Since lipoproteins trigger autophagic flux in ECs *in vitro (41),* effects of high-fat diet (HFD) were analyzed. Preserving a preferential activation in the thoracic aorta, HFD for 12 but not for 4 weeks significantly increased endothelial LC3-staining (P=0.0016) (Fig.7A,B). Moreover, nuclear LC3 localization was less common and the percentage of nuclear Ago2⁺ ECs was lower in areas of LSS, namely in the inner curvature of the aortic arch, than in those of HSS (Fig.7C-E). Notably, endothelium of non-diseased human carotid arteries upstream of the bifurcation (exposed to HSS) showed more prominent nuclear AGO2 localization than atheromatous areas with disturbed flow downstream of the bifurcation (LSS) (Fig.7F-G). Moreover, confocal deconvoluted microscopy unveiled closely localizing signals for Mex3a and miR-126-5p in the nucleus of ECs (Fig.7H). The closest distance between Mex3a and miR-126-5p was quantified in EC nuclei as a proxy of interaction, and found that this distance appears to be lower in ECs of undiseased vascular areas than in ECs overlying atherosclerotic lesions, thus reflecting a higher probability of interaction (Fig.7I,J). Taken together, these findings extend the relevance of our results to human disease.

Next, *Apoe*^{-/}*⁻Cdh5^{cre+}Atg5*^{*fl*/*fl*} (ATG5^{EC-KO}) mice with defective endothelial autophagy were evaluated *(42).* After 12 weeks of HFD, the percentage of ECs with nuclear Ago2 and miR-126-5p signals (as assessed by *in situ* PCR) in the thoracic aorta (Fig.7K-L; Fig.14A,B) and the content of nuclear miR-126-5p (as determined by qPCR) in both areas of LSS and HSS (Fig.7M) was lower in ATG5^{EC-KO} mice than in controls. Increased circulating CD31⁺annexin-V⁺ ABs and miR-126-3p concentrations *(19)* implied enhanced EC apoptosis in ATG5^{EC-KO} mice (Fig.7N; Fig.14C,D). As apoptosis disrupts endothelial integrity to promote atheroprogression *(8, 16, 17, 43),* the burden of atherosclerosis was accordingly increased in ATG5^{EC-KO} mice after 12 weeks of HFD (Fig.7O), an effect due to a prominent exacerbation in areas of HSS (Fig.7P). As another approach to prevent nuclear enrichment of miR-126-5p *in vivo,* mice with a genetic deficiency of *Mex3a* were generated (Fig.14E). Similar to ATG5^{EC-KO} mice, the thoracic aorta of *Mex3a^{-l-}* mice displayed fewer ECs with nuclear Ago2 staining (Fig.7Q; Fig.14F), and *in situ* RNA hybridization revealed a marked reduction in nuclear miR-126-5p⁺ ECs (Fig.7R-S). Conversely, nuclear staining for caspase-3, reflecting the active form, was increased in *Mex3a*^{*-*/*-*} mice (Fig.7T; Fig.14G) and, consistent with enhanced endothelial apoptosis, confirmed in *Mex3a^{-l-}* pulmonary ECs (Fig.14H). In human carotid artery samples, close localized signals for miR-126-5p and caspase-3 were detectable with a higher prevalence in non-diseased *vs*. atheromatous areas (Fig.7U-V; Fig. 14I-J). By the evaluation of the atherosclerosis area in the aortic root of WT (*Apoe^{-l-}Mex3a*^{+/+}*)* and KO (*Apoe*^{*-*/}*⁻Mex3a*^{-/-}) mice it is demonstrated that Mex3a deficiency is associated with atherosclerosis (Fig. 15).

Collectively, our data support that the nuclear Ago2:Mex3a:miR-126-5p pathway driven by endothelial autophagy can occur *in vivo* to confer shear-dependent atheroprotection.

### Example 7 - Discussion

Our study unravels an unexpected miRNA function by establishing that nuclear miR-126-5p inhibits pro-apoptotic caspase-3 activity through a direct miRNA-protein interaction. This non-canonical mechanism involves a specific Mex3a-guided nuclear transfer of miR-126-5p (Fig.14K). Intriguingly this expands the known spectrum of post-transcriptional regulation by miRNAs and adds an unexpected mechanism to the repertoire of anti-apoptotic armor for protecting and maintaining EC integrity in the context of autophagy.

### Involvement of Mex3a in miRNA biology

The role of Mex3a in mammals is largely unknown but it has been correlated to stemness in colon cancer cell lines *(36)* and in a subpopulation of slowly proliferating intestinal progenitor cells *(44).* Mex3a was identified and characterized as an RNA-binding protein that shuttles between the nucleus and the cytoplasm, where it co-localizes with AGO2 *(35).* While RNAse could disrupt the interaction with AGO2, the nature of this bridging RNA remained to be defined. Our results reveal that Mex3a can bind to miRNAs through its KH domains, featuring a remarkable preference for miR-126-5p (Fig.4). In fact, the presence of miR-126-5p is required for the formation of a ternary complex with Mex3a and AGO2 without a target mRNA (Fig.4F,G), as it may occur in LMW complexes under conditions of autophagy (Fig.10C).

AGO2 uses guide miRNAs to interrogate mRNAs targeted for post-transcriptional repression. The crystal structures of AGO2 bound to a guide miRNA with and without target RNA suggest a stepwise mechanism, in which AGO2 primarily exposes guide nucleotides 2-5 in the 5' end for initial target pairing, thereby promoting conformational changes that expose nucleotides 2-8 and 13-16 for further target recognition *(45, 46).* In the regular guide, the 5' end stretches across in the central cleft between the two AGO2 domains (N-PAZ and MID-PIWI) and is restricted after nucleotide 8, while the 3' end is threaded through the N-PAZ channel. Ternary complex formation of AGO2, Mex3a and miR-126-5p may thus involve an interaction of the 5' region of miR-126-5p with AGO2, while the Mex3a tandem KH domains may engage in contact with the 3' part of miR-126-5p not bound to AGO2. In addition, direct interactions between AGO2 and other regions in Mex3a, outside the RNA-binding KH domains, might occur to further stabilize the ternary complex.

The mechanism, by which Mex3a binding through ternary complex formation with AGO2 directs miR-126-5p towards the nucleus, remains elusive. The interaction with Mex3a may support retention of the complex on the external surface of the autophagosome and transfer to the nuclear pore for importin-interaction. As a limitation of our study, it remains to be determined whether and which additional adaptor(s) may contribute to the interactions of the complex with LC3. The protein LC3 plays an important role in the selective recruitment of autophagic cargo and can thus serve as docking site for multiple adaptor proteins, which can simultaneously interact with LC3 in autophagosomes and with their cargo *(47).* Hence, one would need to postulate such components to anchor and present the Mex3a:AGO:miR-126-5p complex on the extraluminal autophagosome membrane for nuclear import, as indicated by the absence of LC3 in AGO2-immunoprecipitates. Additional components may support the LC3-AGO2 interaction in a manner sufficient to detect a proximity by STED nanoscopy but not a presence of LC3 in AGO2-immunoprecipitates. Because mutational changes involving nucleotides 2-8 in the 5' end of miR-126-5p do not affect nuclear enrichment, it is conceivable that binding of both 3' and 5' parts of miR-126-5p to the KH domains of Mex3a is dispensable for nuclear transfer. Indeed, the importin-dependent nuclear localization sequence predicted with the highest score is located in the RING-domain (residues 417-445) of Mex3a. Likewise, further efforts are warranted in order to identify protein or RNA components in the nucleus that enable the dissociation of miR-126-5p from AGO2 in favor of caspase-3 and to release the seed sequence required for its interaction with caspase-3. Our data indicate that Mex3a facilitates this process, as evidenced by reduced nuclear co-localization upon its silencing.

### miR-126-5p in the crosstalk between autophagy and apoptosis

Autophagy is integral to the well-orchestrated programs of cellular responses to stress. Many triggers of autophagy can also induce apoptosis with both processes often occurring in the same cell (48). Apoptosis is a modality of programmed cell death mediated by specific cascades of proteases, which culminate in the activation of effector caspases (*e.g.* caspase-3) responsible for killing the cells *(49).* Given the antagonistic role of the two processes, autophagy being cytoprotective and apoptosis being cytodestructive, it appears teleologically conceivable that an inhibitory crosstalk exists. For example, removal of damaged mitochondria or pro-apoptotic proteins during autophagy inhibits apoptosis, whereas caspase-3 may cleave autophagy-specific proteins to impair autophagy *(48).* Our results establish a novel mechanism, by which autophagy can inhibit apoptosis via miR-126-5p (Fig.6). This inhibition mainly affects caspase cleavage rather than expression and its rapid onset preceded transcriptional changes, prompting our hypothesis that miR-126-5p directly acts on effector caspases. Among those, caspase-3 is essential for nuclear changes upon apoptosis and localizes in the nucleus after activation and recognition of substrates, independently of their cleavage *(39).* In this compartment, the interaction with miR-126-5p may prevent catalysis of nuclear components to preserve cell viability. Notably, the inhibition of apoptosis by directly controlling catalytic activity of caspase-3 is typically exerted by protein-protein interactions (*e.g.* XIAP). Our results integrate and extend the pathophysiological relevance of related findings showing that (a) miRNAs can adopt a stable secondary structure resembling aptamers, short oligonucleotide sequences with high affinity for molecular targets including proteins *(50),* (b) miRNAs and AGO2 frequently localize in LMW complexes devoid of functional RISC components to form miRNA reservoirs *in vivo (5),* and (c) the conserved non-coding RNA *vtRNA1-1,* termed riboregulator, can prevent oligomerization of the protein p62, thus altering its function in autophagy *(51).*

Although it was clearly shown that miR-126-5p engage in interactions with caspase-3 to inhibit its pro-apoptotic activity *in vitro* and in ECs, the molecular mechanisms underlying this effect remain to be deciphered in detail. As the four loops forming the active site are provided by both heterodimer subunits, dimerization is crucial for proper assembly and cooperativity of active sites in all caspases *(40).* MDS implied that miR-126-5p preferentially binds to caspase-3 monomers rather than to heterodimers. Indeed, dimerization of the p17 caspase-3 subunit could be inhibited by miR-126-5p *in vitro.* On the other hand, MDS predicted that the 5' end (containing the seed sequence) of miR-126-5p may bind the substrate binding pocket with strong affinity, whereas mutational changes involving nucleotides 2-8 impair the inhibitory activity. While it will be crucial to dissect the precise mechanism of inhibition, it is plausible from our data that miR-126-5p may affect caspase-3 activity by a dual mechanism, (I) disrupting dimerization and (II) preventing substrate binding at its pocket. For pro-caspase-3, the first cleavage by activator caspases disrupts the covalent link between the p17 and p12 subunits. Although p12 remains associated by non-covalent binding, conformational changes occurring as a result of this cleavage may also be targeted by interactions with miR-126-5p.

It was found that rapamycin-transferred nuclear miR-126-5p was associated with altered transcript expression of apoptosis-related genes (Fig.5A). These changes occurred later than the anti-apoptotic effect detected by annexin V staining (Fig.5F). Whereas direct inhibition of caspase-3 activity explains early onset, subsequent changes in gene expression may serve to stabilize anti-apoptotic effects of miR-126-5p. Nuclear miRNAs can affect gene expression by several mechanisms including post-transcriptional and transcriptional regulation, alternative splicing, or epigenetic modifications (38). As most of these processes require AGO2 and other RISC components (*i.e*. TNRC6A), this is likely mediated by the AGO2-bound fraction of miR-126-5p.

### Relevance for atherosclerosis

Although the mechanism identified herein could be potentially operative in every cell, the remarkable abundance of miR-126-5p in ECs compared to other cell types *(52)* indicates that it mainly serves as a critical master-switch to control programmed cell death in ECs. Endothelial apoptosis plays a pivotal role in atherosclerosis and a higher apoptosis rate has been found in areas exposed to LSS in mice and humans *(8, 43).* Conversely, activation of KLF2 and autophagic flux as well as miR-126-5p amounts were significantly reduced in those areas, due to the absence of HSS (Fig.7) *(8, 13, 16).* Our results merge and complement these observations by identifying a common functional pathway preserving endothelial integrity. Overexpression of KLF2 recapitulated the initiation of autophagic flux (Fig.1C, Fig.8) and the preferential up-regulation of miR-126-5p (Fig.1A,B) observed upon HSS (Fig.8F,H, Fig.9I), thus supporting a role of KLF2 as an important relay between HSS and initiation of autophagy.

Endothelial autophagy is required for maintaining lipid homeostasis (41) and for ensuring proper alignment to flow *(8).* Deficiency in EC autophagy (*i.e.* conditional *Atg7*/*Atg5* KO) indeed resulted in more severe atherosclerosis, prevalently at sites exposed to HSS (Fig.7O,P) (8, 41). Interestingly, the higher rate of apoptosis described in aortic ECs of these mice *(8)* is paralleled by our findings showing an impaired nuclear enrichment of miR-126-5p (Fig.7L) and thus support its involvement in preserving endothelial integrity *in vivo.* Consistently, impeding nuclear transfer of miR-126-5p through another strategy (*i.e.* silencing *Mex3a)* led to higher apoptosis rates in ECs (Fig.7S,T; Fig.14H). Future work will scrutinize in detail how genetic deficiency of Mex3a in a cell-specific context may affect the initiation and progression of atherosclerosis.

It was previously found that miR-126-5p preserves the endothelial proliferative reserve during hyperlipidemia by repressing a negative regulator of proliferation, *Dlk1 (16).* Maintenance of endothelial integrity requires the replacement of damaged ECs by proliferating ones. At arterial sites exposed to HSS, miR-126-5p does not only promote regenerative proliferation by targeting *Dlk1* mRNA in the RISC but, as shown herein, it may also preserve EC viability by attenuating apoptotic cell death, when enriched in the nucleus via an autophagy-driven mechanism. In line with higher autophagic activity during HFD, as reflected by LC3 staining (Fig.7A-C), this feature would conceivably be more effective when the baseline regenerative equilibrium is disrupted by additional endothelial injury, *e.g.* during dyslipidemia. While it is currently not known whether these events occur simultaneously or in a sequential manner (e.g. *Dlk1* mRNA decay followed by nuclear transfer and inhibition of caspase-3), the answer to this will be of both scientific and potentially clinical interest. Indeed, employing rapamycin (or other mTOR-inhibitors) for preventing restenosis is limited by defective reendothelialization, possibly due to mTORC2 inhibition *(53).* Thus, emulating the anti-apoptotic downstream pathway involving Mex3a-miR-126-5p might enable more specific and hence safer therapeutic options in this context.

### Translational perspectives of our study

A therapeutic relevance in promoting nuclear enrichment of miR-126-5p to prevent endothelial dysfunction and development of atherosclerosis as well as in other conditions where viability of the endothelium is crucial for cardiovascular outcomes (e.g. reendothelialization after acute cardiovascular events) is expected. To this end, a tailored overexpression of Mex3a could be achieved, e.g. by using endothelial-specific constructs in viral microparticle, which can be delivered systemically or locally. Although additional studies will be required to further verify such hypotheses, it is believe that this approach is suitable to improve the anti-atherosclerotic effects previously achieved by systemic administration of nanoparticles carrying miR-126-5p *(16).*

Although nuclear miR-126-5p could potentially act as a diagnostic marker of endothelial health, its evaluation would require sophisticated analysis of pathological specimens (e.g. by FISH) and is thus unlikely to enhance the diagnostic performance of current tests. On the other hand, quantification of circulating CD31⁺ ABs could reflect the amount of distressed endothelial cells and act as a surrogate marker (or prognostic factor) in cardiovascular disease *(54).* Yet, a validation of the diagnostic performance would require further investigations on large prospective cohorts.

### Beyond atherosclerosis and apoptosis

Our data also reinforce the notion that proteins relevant for cell viability (*e.g.* caspase-3 or p62) have previously escaped recognition as RNA-binding proteins *(51, 55).* The Mex3a-miR-126-5p shuttle operates downstream of EC autophagy and can thus integrate crucial effector mechanisms limiting atherosclerosis and its complications. However, the spectrum of human disease wherein autophagy governs the equilibrium between cell death and survival ranges from aging to cancer and neurodegenerative diseases (*e.g.* Huntington's disease) *(6, 7, 11, 56).* Additionally, the proper functionality of the endothelium is required for angiogenesis in health (e.g. wound healing) and disease (*e.g.* cancer neoangiogenesis or retinopathy) *(57).* Thus, modulating or emulating this pathway composed of sequential miRNA-protein interactions to restore endothelial functionality unfolds.

### Example 8 - Materials and Methods

### Study design

The main objective of our study was to identify non-canonical mechanisms of miRNA function. It was taken advantage of the peculiarity of endothelial miR-126 with both strands active and the mechanisms of strand-specific sorting were investigated. The focus was on autophagic conditions which favor the recruitment of AGO2:miRNAs in LMW complexes devoid of silencing effector components (e.g. TNRC6A). Experiments *in vitro* in human umbilical vein endothelial cells (HUVECs) involved manipulation of autophagic flux by chemical inducers and inhibitors (*i.e*. rapamycin, 3-methyladenine) or gene manipulation *(i.e.* KLF2 overexpression, siRNAs against ATG5/ATG7) for (1) multimodal tracking miR-126 strands (*i.e*. fractional qPCR, FISH, fluorescently tagged miRNAs), (2) identification of interacting proteins which mediate trafficking and functional outcomes *(i.e.* RNA-immunoprecipitation, immunoprecipitation, size exclusion chromatography, immunostainings) and (3) analysis of downstream effects (*i.e*. qPCR and immunoblotting for relevant mediators, assessment of apoptosis). Protein-RNA interactions were detailed *in silico* and by biophysical experiments *(i.e.* SPR, SAXS, NMR spectroscopy). Moreover, the functional relevance was tested (*i.e*. competition and caspase-3 activity assays). Finally, the pathophysiological significance was tested *in vivo* in transgenic mice models disrupting the identified pathway at different layers (*i.e.* ATG5^{EC-KO} and *Mex3a^{-l-}*) studying atherosclerosis as a disease model relevant for EC autophagy and miR-126.

### In vitro experiments in endothelial cells

HUVECs were treated with rapamycin (Santa Cruz Biotechnology) at 5 µM, ivermectin (Santa Cruz Biotechnology) at 10 mM, 3-methyladenine (Sigma Aldrich) at 5 mM, and/or camptothecin (Abcam) at 500 nM. Unless stated otherwise, treatment was applied for 3 h to analyze early processes and to avoid activation of concomitant or compensatory mechanisms. RNA interference technology was applied to generate specific knockdowns with siRNA against ATG5, ATG7, Mex3a (Qiagen). Functional studies on miRNA function were carried with miScript miRNA mimics and inhibitors of miR-126-5p (Qiagen). Overexpression of KLF2 and Mex3a were obtained by pCMV-hKLF2 and pCMV-hMex3a plasmids. Transfections were performed by electroporation using Amaxa Nucleofector technology (Lonza) or Lipofectamine™ 2000 (ThermoFisher Scientific) according to the manufacturer's protocols.

### In silico protein-miRNA docking and molecular dynamics simulation

The potential binding modes between miR-126-3p and miR-126-5p with Mex3a and caspase-3 were predicted by application of protein-RNA docking using HDOCK and HADDOCK WebServer *(58, 59).* The selected docking poses were subjected to 20 ns MDS. Trajectories extracted from 15-20 ns were utilized for calculating BFE with molecular mechanics/generalized Born surface area (MM/GBSA) by applying default parameters and generalized Born model 8 for free energy of solvation as described *(60, 61).* To analyze the stability of binding between Mex3a and miR-126-3p and miR-126-5p, MDS of the final complexes were performed for another 180 ns and MD snapshots from different time points were extracted for BFE calculations.

### Biophysical and biochemical in vitro studies

*Surface Plasmon Resonance (SPR)* experiments were performed using a Biacore X100 (GE Healthcare) using SA chips, as described *(60)*. Sensorgrams were obtained by injecting different concentrations of analytes over the chips. Responses from analyte injections were overlaid with the fit of a 1:1 Langmuir interaction model. For competition assays, the half maximal inhibitory concentration (IC₅₀) was determined using a 4-parameter logistic regression.

SAXS experiments on Mex3a^{(KH1KH2)} or the Mex3a^{(KH1KH2)}-miR-126-5p complex were performed with a Rigaku BioSAX8000 system and a microfocus rotating anode (Cu-Ka 0.154 nm, 40 kV, 30 mA). A Pilatus 100k detector was used for image collection and a built-in photo diode was used for transmission measurements. The Rigaku SaxsLab software 3.1 was used for radial averaging, q calibration and solvent subtraction. Radiation damage, data merging and buffer subtraction were performed on site and later verified manually using the program PRIMUS *(62).* All SAXS parameters such as maximum particle size (Dmax) were extracted using the GNOME from the ATSAS package software *(63).*

ITC experiments were performed using a Microcal ITC200 instrument (Malvern). Both Mex3a^{(KH1KH2)} and RNA (miR-126-5p and -3p) were dissolved in PBS buffer at pH 6.5. 200 µl of Mex3a^{(KH1KH2)} at a concentration of 20 µM was titrated with the two RNA ligands at 200 µM concentration at 25°C. For each ITC experiment, a background curve consisting of the titration of the same RNA sample into buffer was subtracted to account for heat generated by dilution. Measurements were carried out in triplicates. The *MicroCal PEAQ-ITC* (Malvern) software was used to analyse all ITC data. As the binding isotherms suggest a more complex binding event, an estimation of the binding affinity was obtained by only fitting the second part of the curve, as this is expected to report on the RNA binding to Mex3a. The complex binding isotherm likely represents a structural rearrangement of the two KH domains upon RNA binding, as indicated by the SAXS data.

NMR spectroscopy was performed at 600 MHz or 800 MHz spectrometers equipped with cryoprobes. ¹⁵N-labeled Mex3a^{(KH1KH2)} was concentrated to 100 µM and titrated with miR-126-5p or mir-126-3p added at 1:0.5, 1:1, and 1:2 stoichiometry. ¹H¹³C methyl ILVM, ²H-labeled caspase-3 was concentrated to 20 µM and miR-126-5p was added at 3-fold molar excess. NMR spectra were processed with NMR Pipe/NMR Draw *(64)* and analyzed using NMR View *(65).* ¹H NMR spectra of caspase-3 free and bound to miR-126-5p RNA were analyzed with Topspin.

### Animal experiments

Mice with EC-specific deletion of *Atg5* (*Cdh5^{Cre+}Atg5*^{*fl*/*fl*}) were recently described *(42)* and crossed with *Apoe^{-l-}* mice to obtain *Apoe*^{*-*/}*⁻Cdh5^{cre+}Atg5*^{*fl*/*fl*} (termed ATG5^{EC-KO}) mice. Age- and sex-matched control animals were used for *in vivo* experiments. A constitutive knock-out model for Mex3a was generated by CRISP/Cas9 genome editing, as detailed in Supplementary Materials. All animal experiments were approved by the local authorities (District Government of Upper Bavaria, Germany; License Number: 55.2-1-54-2532-14-15) and conducted in accordance with the institutional and national guidelines for the care and use of laboratory animals.

A comprehensive and more detailed description of all experimental procedures can be found in Supplementary Materials.

### Statistical analysis

Data were analyzed using the Prism 8 (GraphPad Inc.) and SPSS v.25 (IBM Corp). Descriptive statistics included mean and standard error from mean (s.e.m.), unless differently indicated. For box plots, center line represents the median; box limits, Q1 and Q3 quartiles; whiskers, 1.5x interquartile range; points, eventual outliers. For animal experiments: a *priori* calculation of power was performed based on previous data in literature and pilot experiments. Sample size was designed to achieve 80% power for detecting biological relevant changes (50%) with an α-value of 0.05. For *in vitro* experiments, sample sizes were selected empirically based on pilot experiments. Data distribution and homogeneity of variance were tested by the Shapiro-Wilk and Levene's test, respectively. Violation of the assumptions were considered at P<0.05. Comparisons between 2 groups were performed by Student's *t*-test, with Welch correction as appropriate, or not-parametric Mann-Whitney *U*-test. Depending on the number of factors involved, univariate o factorial ANOVA with Bonferroni *post-hoc* tests were applied to assess significance in comparisons among three or more groups. Experimental design requiring pairing were analyzed by paired t-test (2 groups) or by two-factor paired measurement ANOVA or by generalized linear model and Bonferroni corrections for pairwise comparisons (≥3 groups). Differences in proportions were assessed by a Fisher's X² test. The statistical test and the *n* values are reported in the respective Figure legends. Differences were deemed significant at 2-tailed P<0.05.

### References and Notes:

1. D. P. Bartel, Metazoan MicroRNAs, Cell 173, 20-51 (2018).
2. L. F. Gebert, I. J. MacRae, Regulation of microRNA function in animals, Nat Rev Mol Cell Bio 20, 21-37 (2019).
3. A. Leung, The Whereabouts of microRNA Actions: Cytoplasm and Beyond, Trends Cell Biol 25, 601-610 (2015).
4. S. H. Olejniczak, G. Rocca, J. J. Gruber, C. B. Thompson, Long-lived microRNA-Argonaute complexes in quiescent cells can be activated to regulate mitogenic responses, Proc National Acad Sci 110, 157-162 (2013).
5. G. Rocca, S. H. Olejniczak, A. J. Gonzalez, D. Briskin, J. A. Vidigal, L. Spraggon, R. G. DeMatteo, M. R. Radler, T. Lindsten, A. Ventura, T. Tuschl, C. S. Leslie, C. B. Thompson, In vivo, Argonaute-bound microRNAs exist predominantly in a reservoir of low molecular weight complexes not associated with mRNA, Proc National Acad Sci 112, 767-772 (2015).
6. B. Levine, G. Kroemer, Biological Functions of Autophagy Genes: A Disease Perspective, Cell 176, 11-42 (2019).
7. J. Pedro, G. Kroemer, L. Galluzzi, Autophagy and Mitophagy in Cardiovascular Disease, Circ Res 120, 1812-1824 (2017).
8. A.-C. Vion, M. Kheloufi, A. Hammoutene, J. Poisson, J. Lasselin, C. Devue, I. Pic, N. Dupont, J. Busse, K. Stark, J. Lafaurie-Janvore, A. I. Barakat, X. Loyer, M. Souyri, B. Viollet, P. Julia, A. Tedgui, P. Codogno, C. M. Boulanger, P.-E. Rautou, Autophagy is required for endothelial cell alignment and atheroprotection under physiological blood flow, Proc National Acad Sci 114, E8675-E8684 (2017).
9. D. Gibbings, S. Mostowy, F. Jay, Y. Schwab, P. Cossart, O. Voinnet, Selective autophagy degrades DICER and AGO2 and regulates miRNA activity, Nat Cell Biol 14, 1314-1321 (2012).
10. P. N. Hsieh, G. Zhou, Y. Yuan, R. Zhang, D. A. Prosdocimo, P. Sangwung, A. H. Borton, E. Boriushkin, A. Hamik, H. Fujioka, C. E. Fealy, J. P. Kirwan, M. Peters, Y. Lu, X. Liao, D. Ramirez-Bergeron, Z. Feng, M. K. Jain, A conserved KLF-autophagy pathway modulates nematode lifespan and mammalian age-associated vascular dysfunction, Nat Commun 8, 914 (2017).
11. S. Lan, S. Wu, R. Zuchini, X. Lin, I. Su, T. Tsai, Y. Lin, C. Wu, H. Liu, Autophagy suppresses tumorigenesis of hepatitis B virus-associated hepatocellular carcinoma through degradation of microRNA-224, Hepatology 59, 505-517 (2014).
12. D. R. Sweet, L. Fan, P. N. Hsieh, M. K. Jain, Kruppel-Like Factors in Vascular Inflammation: Mechanistic Insights and Therapeutic Potential, Frontiers Cardiovasc Medicine 5, 6 (2018).
13. A. Doddaballapur, K. M. Michalik, Y. Manavski, T. Lucas, R. H. Houtkooper, X. You, W. Chen, A. M. Zeiher, M. Potente, S. Dimmeler, R. A. Boon, Laminar Shear Stress Inhibits Endothelial Cell Metabolism via KLF2-Mediated Repression of PFKFB3, Arterioscler Thromb Vasc Biol 35, 137-145 (2015).
14. S. Nicoli, C. Standley, P. Walker, A. Hurlstone, K. E. Fogarty, N. D. Lawson, MicroRNA-mediated integration of haemodynamics and Vegf signalling during angiogenesis, Nature 464, 1196-1200 (2010).
15. K. M. Parmar, B. H. Larman, G. Dai, Y. Zhang, E. T. Wang, N. priya Moorthy, J. R. Kratz, Z. Lin, M. K. Jain, M. A. Jr., G. García-Cardeña, Integration of flow-dependent endothelial phenotypes by Kruppel-like factor 2, J Clin Invest 116, 49-58 (2006).
16. A. Schober, M. Nazari-Jahantigh, Y. Wei, K. Bidzhekov, F. Gremse, J. Grommes, R. T. Megens, K. Heyll, H. Noels, M. Hristov, S. Wang, F. Kiessling, E. N. Olson, C. Weber, MicroRNA-126-5p promotes endothelial proliferation and limits atherosclerosis by suppressing Dlk1, Nat Med 20, 368-376 (2014).
17. A. Schober, M. Nazari-Jahantigh, C. Weber, MicroRNA-mediated mechanisms of the cellular stress response in atherosclerosis, Nat Rev Cardiol 12, 361-374 (2015).
18. T. Thum, G. Condorelli, Long Noncoding RNAs and MicroRNAs in Cardiovascular Pathophysiology, Circ Res 116, 751-762 (2015).
19. A. Zernecke, K. Bidzhekov, H. Noels, E. Shagdarsuren, L. Gan, B. Denecke, M. Hristov, T. Köppel, M. Jahantigh, E. Lutgens, S. Wang, E. N. Olson, A. Schober, C. Weber, Delivery of MicroRNA-126 by Apoptotic Bodies Induces CXCL12-Dependent Vascular Protection, Sci Signal 2, ra81 (2009).
20. J. E. Fish, M. Santoro, S. U. Morton, S. Yu, R.-F. Yeh, J. D. Wythe, K. N. Ivey, B. G. Bruneau, D. Y. Stainier, D. Srivastava, miR-126 regulates angiogenic signaling and vascular integrity, Dev Cell 15, 272-284 (2008).
21. T. A. Harris, M. Yamakuchi, M. Ferlito, J. T. Mendell, C. J. Lowenstein, MicroRNA-126 regulates endothelial expression of vascular cell adhesion molecule 1, Proc National Acad Sci 105, 1516-1521 (2008).
22. S. Wang, A. B. Aurora, B. A. Johnson, X. Qi, J. McAnally, J. A. Hill, J. A. Richardson, R. Bassel-Duby, E. N. Olson, The Endothelial-Specific MicroRNA miR-126 Governs Vascular Integrity and Angiogenesis, Dev Cell 15, 261-271 (2008).
23. P. Hartmann, Z. Zhou, L. Natarelli, Y. Wei, M. Nazari-Jahantigh, M. Zhu, J. Grommes, S. Steffens, C. Weber, A. Schober, Endothelial Dicer promotes atherosclerosis and vascular inflammation by miRNA-103-mediated suppression of KLF4, Nat Commun 7, 10521 (2016).
24. N. Baeyens, S. Nicoli, B. G. Coon, T. D. Ross, K. den Dries, J. Han, H. M. Lauridsen, C. O. Mejean, A. Eichmann, J.-L. Thomas, J. D. Humphrey, M. A. Schwartz, Vascular remodeling is governed by a VEGFR3-dependent fluid shear stress set point, Elife 4, e04645 (2015).
25. S. Pitchiaya, L. A. Heinicke, J. I. Park, E. L. Cameron, N. G. Walter, Resolving Subcellular miRNA Trafficking and Turnover at Single-Molecule Resolution, Cell Reports 19, 630-642 (2017).
26. X. Yang, W. W. Du, H. Li, F. Liu, A. Khorshidi, Z. Rutnam, B. B. Yang, Both mature miR-17-5p and passenger strand miR-17-3p target TIMP3 and induce prostate tumor growth and invasion, Nucleic Acids Res 41, 9688-9704 (2013).
27. H.-W. Hwang, E. A. Wentzel, J. T. Mendell, A Hexanucleotide Element Directs MicroRNA Nuclear Import, Science 315, 97-100 (2007).
28. M. Benhamed, U. Herbig, T. Ye, A. Dejean, O. Bischof, Senescence is an endogenous trigger for microRNA-directed transcriptional gene silencing in human cells, Nat Cell Biol 14, 266 (2012).
29. N. Martinez-Lopez, D. Athonvarangkul, P. Mishall, S. Sahu, R. Singh, Autophagy proteins regulate ERK phosphorylation, Nat Commun 4, 2799 (2013).
30. Y. Kim, A. Maizel, X. Chen, Traffic into silence: endomembranes and post-transcriptional RNA silencing, EMBO J 33, 968-980 (2014).
31. T. Ohrt, J. Mütze, W. Staroske, L. Weinmann, J. Höck, K. Crell, G. Meister, P. Schwille, Fluorescence correlation spectroscopy and fluorescence cross-correlation spectroscopy reveal the cytoplasmic origination of loaded nuclear RISC in vivo in human cells, Nucleic Acids Res 36, 6439-6449 (2008).
32. K. Nishi, A. Nishi, T. Nagasawa, K. Ui-Tei, Human TNRC6A is an Argonaute-navigator protein for microRNA-mediated gene silencing in the nucleus, RNA 19, 17-35 (2013).
33. D. Schraivogel, G. Meister, Import routes and nuclear functions of Argonaute and other small RNA-silencing proteins, Trends Biochem Sci 39, 420-431 (2014).
34. L. Weinmann, J. Höck, T. Ivacevic, T. Ohrt, J. Mütze, P. Schwille, E. Kremmer, V. Benes, H. Urlaub, G. Meister, Importin 8 Is a Gene Silencing Factor that Targets Argonaute Proteins to Distinct mRNAs, Cell 136, 496-507 (2009).
35. K. Buchet-Poyau, J. Courchet, H. Hir, B. Séraphin, J.-Y. Scoazec, L. Duret, C. Domon-Dell, J.-N. Freund, M. Billaud, Identification and characterization of human Mex-3 proteins, a novel family of evolutionarily conserved RNA-binding proteins differentially localized to processing bodies, Nucleic Acids Res 35, 1289-1300 (2007).
36. B. Pereira, M. Borgne, N. T. Chartier, M. Billaud, R. Almeida, MEX-3 proteins: recent insights on novel post-transcriptional regulators, Trends Biochem Sci 38, 477-479 (2013).
37. L. Yang, C. Wang, F. Li, J. Zhang, A. Nayab, J. Wu, Y. Shi, Q. Gong, The human RNA-binding protein and E3 ligase MEX-3C binds the MEX-3-recognition element (MRE) motif with high affinity, J Biol Chem 292, 16221-16234 (2017).
38. T. C. Roberts, The MicroRNA Biology of the Mammalian Nucleus, Mol Ther - Nucleic Acids 3, e188 (2014).
39. S. Kamada, U. Kikkawa, Y. Tsujimoto, T. Hunter, Nuclear Translocation of Caspase-3 Is Dependent on Its Proteolytic Activation and Recognition of a Substrate-like Protein(s), J Biol Chem 280, 857-860 (2005).
40. S. H. MacKenzie, C. A. Clark, Death by Caspase Dimerization, Adv Exp Med Biol 747, 55-73 (2012).
41. K. Torisu, K. K. Singh, T. Torisu, F. Lovren, J. Liu, Y. Pan, A. Quan, A. Ramadan, M. Al-Omran, N. Pankova, S. R. Boyd, S. Verma, T. Finkel, Intact endothelial autophagy is required to maintain vascular lipid homeostasis, Aging Cell 15, 187-191 (2016).
42. D. Sprott, D. M. Poitz, I. Korovina, A. Ziogas, J. Phieler, A. Chatzigeorgiou, I. Mitroulis, A. Deussen, T. Chavakis, A. Ameln, Endothelial-Specific Deficiency of ATG5 (Autophagy Protein 5) Attenuates Ischemia-Related Angiogenesis, Arterioscler Thromb Vasc Biol 39, 1137-1148 (2019).
43. O. Tricot, Z. Mallat, C. Heymes, J. Belmin, G. Lesèche, A. Tedgui, Relation Between Endothelial Cell Apoptosis and Blood Flow Direction in Human Atherosclerotic Plaques, Circulation 101, 2450-2453 (2000).
44. F. M. Barriga, E. Montagni, M. Mana, M. Mendez-Lago, X. Hernando-Momblona, M. Sevillano, A. Guillaumet-Adkins, G. Rodriguez-Esteban, S. Buczacki, M. Gut, H. Heyn, D. J. Winton, O. H. Yilmaz, C. Attolini, I. Gut, E. Batlle, Mex3a Marks a Slowly Dividing Subpopulation of Lgr5+ Intestinal Stem Cells, Cell Stem Cell 20, 801-816 (2017).
45. E. Elkayam, C.-D. Kuhn, A. Tocilj, A. D. Haase, E. M. Greene, G. J. Hannon, L. Joshua-Tor, The Structure of Human Argonaute-2 in Complex with miR-20a, Cell 150, 100-110 (2012).
46. N. T. Schirle, J. Sheu-Gruttadauria, I. J. MacRae, Structural basis for microRNA targeting, Science 346, 608-613 (2014).
47. P. Wild, D. G. McEwan, I. Dikic, The LC3 interactome at a glance, J Cell Sci 127, 3-9 (2014).
48. G. Mariño, M. Niso-Santano, E. H. Baehrecke, G. Kroemer, Self-consumption: the interplay of autophagy and apoptosis, Nat Rev Mol Cell Bio 15, nrm3735 (2014).
49. S. Nagata, Apoptosis and Clearance of Apoptotic Cells, Annu Rev Immunol 36, 1-29 (2018).
50. A. Belter, D. Gudanis, K. Rolle, M. Piwecka, Z. Gdaniec, M. Z. Naskret-Barciszewska, J. Barciszewski, Mature MiRNAs Form Secondary Structure, which Suggests Their Function beyond RISC, Plos One 9, e113848 (2014).
51. R. Horos, M. Büscher, R. Kleinendorst, A.-M. Alleaume, A. K. Tarafder, T. Schwarzl, D. Dziuba, C. Tischer, E. M. Zielonka, A. Adak, A. Castello, W. Huber, C. Sachse, M. W. Hentze, The Small Non-coding Vault RNA1-1 Acts as a Riboregulator of Autophagy, Cell 176, 1054-1067.e12 (2019).
52. D. de Rie, I. Abugessaisa, T. Alam, E. Arner, P. Arner, H. Ashoor, G. Åström, M. Babina, N. Bertin, M. A. Burroughs, A. J. Carlisle, C. O. Daub, M. Detmar, R. Deviatiiarov, A. Fort, C. Gebhard, D. Goldowitz, S. Guhl, T. J. Ha, J. Harshbarger, A. Hasegawa, K. Hashimoto, M. Herlyn, P. Heutink, K. J. Hitchens, C. Hon, E. Huang, Y. Ishizu, C. Kai, T. Kasukawa, P. Klinken, T. Lassmann, C.-H. Lecellier, W. Lee, M. Lizio, V. Makeev, A. Mathelier, Y. A. Medvedeva, N. Mejhert, C. J. Mungall, S. Noma, M. Ohshima, M. Okada-Hatakeyama, H. Persson, P. Rizzu, F. Roudnicky, P. Sætrom, H. Sato, J. Severin, J. W. Shin, R. K. Swoboda, H. Tarui, H. Toyoda, K. Vitting-Seerup, L. Winteringham, Y. Yamaguchi, K. Yasuzawa, M. Yoneda, N. Yumoto, S. Zabierowski, P. G. Zhang, C. A. Wells, K. mmers, H. Kawaji, A. Sandelin, M. Rehli, T. Consortium, Y. Hayashizaki, P. Carninci, A. R. Forrest, M. J. de Hoon, An integrated expression atlas of miRNAs and their promoters in human and mouse, Nat Biotechnol 35, 872-878 (2017).
53. A. Barilli, R. Visigalli, R. Sala, G. C. Gazzola, A. Parolari, E. Tremoli, S. Bonomini, A. Simon, E. I. Closs, V. Dall'Asta, O. Bussolati, In human endothelial cells rapamycin causes mTORC2 inhibition and impairs cell viability and function, Cardiovasc Res 78, 563-571 (2008).
54. A.-C. Vion, B. Ramkhelawon, X. Loyer, G. Chironi, C. Devue, G. Loirand, A. Tedgui, S. Lehoux, C. M. Boulanger, Shear stress regulates endothelial microparticle release., Circ Res 112, 1323-33 (2013).
55. M. W. Hentze, A. Castello, T. Schwarzl, T. Preiss, A brave new world of RNA-binding proteins, Nat Rev Mol Cell Bio 19, 327-341 (2018).
56. A. Choi, S. W. Ryter, B. Levine, Autophagy in Human Health and Disease, New Engl J Med 368, 651-662 (2013).
57. P. Carmeliet, Angiogenesis in life, disease and medicine, Nature 438, 932 (2005).
58. Y. Yan, D. Zhang, P. Zhou, B. Li, S.-Y. Huang, HDOCK: a web server for protein-protein and protein-DNA/RNA docking based on a hybrid strategy, Nucleic Acids Res 45, W365-W373 (2017).
59. G. C. P. van Zundert, J. P. G. L. M. Rodrigues, M. Trellet, C. Schmitz, P. L. Kastritis, E. Karaca, A. S. J. Melquiond, M. van Dijk, S. J. de Vries, A. M. J. J. Bonvin, The HADDOCK2.2 Web Server: User-Friendly Integrative Modeling of Biomolecular Complexes, J Mol Biol 428, 720-725 (2016).
60. P. von Hundelshausen, S. M. Agten, V. Eckardt, X. Blanchet, M. M. Schmitt, H. Ippel, C. Neideck, K. Bidzhekov, J. Leberzammer, K. Wichapong, A. Faussner, M. Drechsler, J. Grommes, J. P. van Geffen, H. Li, A. Ortega-Gomez, R. T. Megens, R. Naumann, I. Dijkgraaf, G. A. Nicolaes, Y. Doring, O. Soehnlein, E. Lutgens, J. W. Heemskerk, R. R. Koenen, K. H. Mayo, T. M. Hackeng, C. Weber, Chemokine interactome mapping enables tailored intervention in acute and chronic inflammation, Sci Transl Med 9, eaah6650 (2017).
61. K. Wichapong, J.-E. Alard, A. Ortega-Gomez, C. Weber, T. M. Hackeng, O. Soehnlein, G. A. Nicolaes, Structure-Based Design of Peptidic Inhibitors of the Interaction between CC Chemokine Ligand 5 (CCL5) and Human Neutrophil Peptides 1 (HNP1), J Med Chem 59, 4289-4301 (2016).
62. P. V. Konarev, V. V. Volkov, A. V. Sokolova, M. H. J. Koch, D. I. Svergun, PRIMUS: a Windows PC-based system for small-angle scattering data analysis, J Appl Crystallogr 36, 1277-1282 (2003).
63. M. V. Petoukhov, D. Franke, A. V. Shkumatov, G. Tria, A. G. Kikhney, M. Gajda, C. Gorba, H. D. T. Mertens, P. V. Konarev, D. I. Svergun, New developments in the ATSAS program package for small-angle scattering data analysis, J Appl Crystallogr 45, 342-350 (2012).
64. F. Delaglio, S. Grzesiek, G. W. Vuister, G. Zhu, J. Pfeifer, A. Bax, NMRPipe: A multidimensional spectral processing system based on UNIX pipes, J Biomol NMR 6, 277-293 (1995).
65. B. A. Johnson, R. A. Blevins, NMR View: A computer program for the visualization and analysis of NMR data, J Biomol NMR 4, 603-614 (1994).
66. S. Giebe, N. Cockcroft, K. Hewitt, M. Brux, A. Hofmann, H. Morawietz, C. Brunssen, Cigarette smoke extract counteracts atheroprotective effects of high laminar flow on endothelial function, Redox Biol 12, 776-786 (2017).
67. C. Brunssen, S. Korten, M. Brux, S. Seifert, J. Roesler, S. Bornstein, H. Morawietz, W. Goettsch, COUP-TFII is regulated by high glucose in endothelial cells., Horm Metab Res 42, 81-87 (2010).
68. C. Mahl, V. Egea, R. T. Megens, T. Pitsch, D. Santovito, C. Weber, C. Ries, RECK (reversion-inducing cysteine-rich protein with Kazal motifs) regulates migration, differentiation and Wnt/β-catenin signaling in human mesenchymal stem cells, Cell MolLife Sci 73, 1489-1501 (2016).
69. M. W. Pfaffl, G. W. Horgan, L. Dempfle, Relative expression software tool (REST) for group-wise comparison and statistical analysis of relative expression results in real-time PCR., Nucleic Acids Res 30, e36 (2002).
70. J. Schindelin, I. Arganda-Carreras, E. Frise, V. Kaynig, M. Longair, T. Pietzsch, S. Preibisch, C. Rueden, S. Saalfeld, B. Schmid, J.-Y. Tinevez, D. White, V. Hartenstein, K. Eliceiri, P. Tomancak, A. Cardona, Fiji: an open-source platform for biological-image analysis, Nat Methods 9, 676-682 (2012).
71. L. Natarelli, C. Geißler, G. Csaba, Y. Wei, M. Zhu, A. di Francesco, P. Hartmann, R. Zimmer, A. Schober, miR-103 promotes endothelial maladaptation by targeting IncWDR59, Nat Commun 9, 2645 (2018).
72. D. Huang, B. T. Sherman, R. A. Lempicki, Systematic and integrative analysis of large gene lists using DAVID bioinformatics resources, Nat Protoc 4, 44 (2009).
73. M. Krzywinski, J. Schein, I. Birol, J. Connors, R. Gascoyne, D. Horsman, S. J. Jones, M. A. Marra, Circos: An information aesthetic for comparative genomics, Genome Res 19, 1639-1645 (2009).
74. C. A. Klattenhoff, J. C. Scheuermann, L. E. Surface, R. K. Bradley, P. A. Fields, M. L. Steinhauser, H. Ding, V. L. Butty, L. Torrey, S. Haas, R. Abo, M. Tabebordbar, R. T. Lee, C. B. Burge, L. A. Boyer, Braveheart, a Long Noncoding RNA Required for Cardiovascular Lineage Commitment, Cell 152, 570-583 (2013).
75. M. Popenda, M. Szachniuk, M. Antczak, K. J. Purzycka, P. Lukasiak, N. Bartol, J. Blazewicz, R. W. Adamiak, Automated 3D structure composition for large RNAs, Nucleic Acids Res 40, e112-e112 (2012).
76. C. Pop, Y.-R. Chen, B. Smith, K. Bose, B. Bobay, A. Tripathy, S. Franzen, C. A. Clark, Removal of the Pro-Domain Does Not Affect the Conformation of the Procaspase-3 Dimer, Biochemistry 40, 14224-14235 (2001).
77. J. Pfaff, J. Hennig, F. Herzog, R. Aebersold, M. Sattler, D. Niessing, G. Meister, Structural features of Argonaute-GW182 protein interactions, Proc National Acad Sci 110, E3770-E3779 (2013).
78. D. J. Klionsky, K. Abdelmohsen, A. Abe, M. Abedin, H. Abeliovich, A. Arozena, H. et al., Guidelines for the use and interpretation of assays for monitoring autophagy (3rd edition), Autophagy 12, 1-222 (2016).

## Claims

1. A nucleic acid molecule comprising or consisting of
(a) the nucleic acid sequence of SEQ ID NO: 1,
(b) a nucleic acid sequence being at least 80%, preferably at least 90% and most preferably at least 95% identical to SEQ ID NO: 1,
(c) a fragment of the nucleic acid sequence of (a) or (b), wherein said fragment comprises at least 15 nucleotides, preferably at least 17 nucleotides and most preferably at least 19 nucleotides,
(d) the nucleic acid sequence or any one of (a) to (c), wherein U is replaced by T,
(e) a vector expressing the nucleic acid sequence of any one of (a) to (d), and/or
(f) a host comprising the vector of (e)
for use in the prevention and/or treatment of disease-related apoptosis of endothelial cells.

2. The nucleic acid molecule for of claim 1, wherein the vector expresses the nucleic acid sequence of any one of (a) to (d) under the control of an endothelial cell-specific promoter.

3. The nucleic acid molecule for use of claim 1 or 2, wherein the vector is an adeno-associated vector (AAV).

4. The nucleic acid molecule for use of claim 3, wherein the AVV is AVV9, AVV1 or AAV5.

5. The nucleic acid molecule for use of claim 4, wherein the AAV is AAV9.

6. The nucleic acid molecule for use of any one of claims 1 to 5, wherein the disease is a disease with involvement of macro- or microvascular endothelial apoptosis, and is preferably selected from the group consisting of atherosclerosis, allograph or microvascular vasculopathy, pulmonary hypertension, ischemia-reperfusion injury, congestive heart failure, sepsis, microvascular complications of diabetes, systemic sclerosis, inflammatory vasculitis, and thrombotic thrombocytopenic purpura.

7. The nucleic acid molecule for use of any one of claims 1 to 6, wherein the nucleic acid molecule is formulated for delivery to the cell nucleus.

8. The nucleic acid molecule for use of any of one of claims 1 to 7, wherein the nucleic acid molecule is
(i) complexed with a nuclear localization signal (NLS) or a (poly)peptide comprising a NLS, wherein the NLS preferably comprises or consists of PKKKRKV or KKKX_{5- 20}RK,
(ii) complexed with a polymer, wherein the polymer preferably comprises of consists of prepolyamidoamine (PAMAM) dendrimers or polyethyleneimine (PEI).
(iii) comprised in a nanoparticle, preferably a PEGylated-polylysine/lipid/DNA nanoparticle,
(iv) complexed with a transcription factor or a (poly)peptide comprising transcription factor-binding site, wherein the transcription factor is preferably an endothelial cell-specific transcription factor,
(v) complexed with a transcription factor-binding site or a (poly)peptide comprising transcription factor-binding site, wherein the transcription factor-binding site is preferably an endothelial cell-specific transcription factor-binding site,
(vi) complexed with a small molecule ligand of importin, wherein the small molecule ligand is preferably biotin-streptavidin conjugate, and/or
(vii) formulated together with a small molecule that reversibly disrupts the nuclear pore complex, wherein the small molecule is preferably trans-cyclohexane-1,2-diol.

9. The nucleic acid molecule for use of any one of claims 1 to 8, wherein in addition
(i) a polypeptide comprising or consisting of the amino acid sequence of SEQ ID NO: 2 or an amino acid sequence being at least 80% identical thereto, or
(ii) a second nucleic acid molecule expressing the polypeptide of (i)
is used in the prevention and/or treatment of disease-related apoptosis.

10. The nucleic acid molecule for use of claim 9, wherein the second nucleic acid molecule is a vector, preferably an AAV, more preferably AAV9, AAV, AAV5, and most preferably AAV9.

11. The nucleic acid molecule for use of claim 9 or 10, wherein the second nucleic acid molecule is expressed under the control of an endothelial cell-specific promoter.

12. The nucleic acid molecule for use any one of claims 1 to 10, wherein in addition
(i') a second polypeptide comprising or consisting of the amino acid sequence of SEQ ID NO: 3 or an amino acid sequence being at least 80% identical thereto, or
(ii") a third nucleic acid molecule expressing the polypeptide of (i')
is used in the prevention and/or treatment of disease-related apoptosis.

13. The nucleic acid molecule for use of claim 12, wherein the third nucleic acid molecule is a vector, preferably an AAV, more preferably AAV9, AAV1 or AAV5 and most preferably AAV9.

14. The nucleic acid molecule for use of claim 12 or 13, wherein the second nucleic acid molecule is expressed under the control of an endothelial cell-specific promoter.


SEQUENCE LISTING

<110> Ludwig-Maximilians-Universität München

<120> Inhibition of caspase-3 by the microRNA 126-5p

<130> AD1236 EP

<150> EP20166120.4
<151> 2020-03-27

<160> 7

<170> BiSSAP 1.3

<210> 1
<211> 21
<212> RNA
<213> Homo sapiens

<220>
<223> miRNA 126-5p

<400> 1
cauuauuacu uuugguacgc g                                                21


<210> 2
<211> 520
<212> PRT
<213> Homo sapiens

<220>
<223> human Mex3a protein, UniProtKB - A1L020

<400> 2
Met Pro Ser Leu Val Val Ser Gly Ile Met Glu Arg Asn Gly Gly Phe
1               5                   10                  15
Gly Glu Leu Gly Cys Phe Gly Gly Ser Ala Lys Asp Arg Gly Leu Leu
            20                  25                  30
Glu Asp Glu Arg Ala Leu Gln Leu Ala Leu Asp Gln Leu Cys Leu Leu
        35                  40                  45
Gly Leu Gly Glu Pro Pro Ala Pro Thr Ala Gly Glu Asp Gly Gly Gly
    50                  55                  60
Gly Gly Gly Gly Ala Pro Ala Gln Pro Ala Ala Pro Pro Gln Pro Ala
65                  70                  75                  80
Pro Pro Pro Pro Pro Ala Ala Pro Pro Ala Ala Pro Thr Ala Ala Pro
                85                  90                  95
Ala Ala Gln Thr Pro Gln Pro Pro Thr Ala Pro Lys Gly Ala Ser Asp
            100                 105                 110
Ala Lys Leu Cys Ala Leu Tyr Lys Glu Ala Glu Leu Arg Leu Lys Gly
        115                 120                 125
Ser Ser Asn Thr Thr Glu Cys Val Pro Val Pro Thr Ser Glu His Val
    130                 135                 140
Ala Glu Ile Val Gly Arg Gln Gly Cys Lys Ile Lys Ala Leu Arg Ala
145                 150                 155                 160
Lys Thr Asn Thr Tyr Ile Lys Thr Pro Val Arg Gly Glu Glu Pro Val
                165                 170                 175
Phe Met Val Thr Gly Arg Arg Glu Asp Val Ala Thr Ala Arg Arg Glu
            180                 185                 190
Ile Ile Ser Ala Ala Glu His Phe Ser Met Ile Arg Ala Ser Arg Asn
        195                 200                 205
Lys Ser Gly Ala Ala Phe Gly Val Ala Pro Ala Leu Pro Gly Gln Val

210                 215                 220
Thr Ile Arg Val Arg Val Pro Tyr Arg Val Val Gly Leu Val Val Gly
225                 230                 235                 240
Pro Lys Gly Ala Thr Ile Lys Arg Ile Gln Gln Gln Thr Asn Thr Tyr
                245                 250                 255
Ile Ile Thr Pro Ser Arg Asp Arg Asp Pro Val Phe Glu Ile Thr Gly
            260                 265                 270
Ala Pro Gly Asn Val Glu Arg Ala Arg Glu Glu Ile Glu Thr His Ile
        275                 280                 285
Ala Val Arg Thr Gly Lys Ile Leu Glu Tyr Asn Asn Glu Asn Asp Phe
    290                 295                 300
Leu Ala Gly Ser Pro Asp Ala Ala Ile Asp Ser Arg Tyr Ser Asp Ala
305                 310                 315                 320
Trp Arg Val His Gln Pro Gly Cys Lys Pro Leu Ser Thr Phe Arg Gln
                325                 330                 335
Asn Ser Leu Gly Cys Ile Gly Glu Cys Gly Val Asp Ser Gly Phe Glu
            340                 345                 350
Ala Pro Arg Leu Gly Glu Gln Gly Gly Asp Phe Gly Tyr Gly Gly Tyr
        355                 360                 365
Leu Phe Pro Gly Tyr Gly Val Gly Lys Gln Asp Val Tyr Tyr Gly Val
    370                 375                 380
Ala Glu Thr Ser Pro Pro Leu Trp Ala Gly Gln Glu Asn Ala Thr Pro
385                 390                 395                 400
Thr Ser Val Leu Phe Ser Ser Ala Ser Ser Ser Ser Ser Ser Ser Ala
                405                 410                 415
Lys Ala Arg Ala Gly Pro Pro Gly Ala His Arg Ser Pro Ala Thr Ser
            420                 425                 430
Ala Gly Pro Glu Leu Ala Gly Leu Pro Arg Arg Pro Pro Gly Glu Pro
        435                 440                 445
Leu Gln Gly Phe Ser Lys Leu Gly Gly Gly Gly Leu Arg Ser Pro Gly
    450                 455                 460
Gly Gly Arg Asp Cys Met Val Cys Phe Glu Ser Glu Val Thr Ala Ala
465                 470                 475                 480
Leu Val Pro Cys Gly His Asn Leu Phe Cys Met Glu Cys Ala Val Arg
                485                 490                 495
Ile Cys Glu Arg Thr Asp Pro Glu Cys Pro Val Cys His Ile Thr Ala
            500                 505                 510
Thr Gln Ala Ile Arg Ile Phe Ser
        515                 520

<210> 3
<211> 859
<212> PRT
<213> Homo sapiens

<220>
<223> Human AGO2 (protein argonaute-2), UniProtKB - Q9UKV8

<400> 3
Met Tyr Ser Gly Ala Gly Pro Ala Leu Ala Pro Pro Ala Pro Pro Pro
1               5                   10                  15
Pro Ile Gln Gly Tyr Ala Phe Lys Pro Pro Pro Arg Pro Asp Phe Gly
            20                  25                  30
Thr Ser Gly Arg Thr Ile Lys Leu Gln Ala Asn Phe Phe Glu Met Asp
        35                  40                  45
Ile Pro Lys Ile Asp Ile Tyr His Tyr Glu Leu Asp Ile Lys Pro Glu
    50                  55                  60
Lys Cys Pro Arg Arg Val Asn Arg Glu Ile Val Glu His Met Val Gln
65                  70                  75                  80
His Phe Lys Thr Gln Ile Phe Gly Asp Arg Lys Pro Val Phe Asp Gly
                85                  90                  95
Arg Lys Asn Leu Tyr Thr Ala Met Pro Leu Pro Ile Gly Arg Asp Lys
            100                 105                 110

Val Glu Leu Glu Val Thr Leu Pro Gly Glu Gly Lys Asp Arg Ile Phe
        115                 120                 125
Lys Val Ser Ile Lys Trp Val Ser Cys Val Ser Leu Gln Ala Leu His
    130                 135                 140
Asp Ala Leu Ser Gly Arg Leu Pro Ser Val Pro Phe Glu Thr Ile Gln
145                 150                 155                 160
Ala Leu Asp Val Val Met Arg His Leu Pro Ser Met Arg Tyr Thr Pro
                165                 170                 175
Val Gly Arg Ser Phe Phe Thr Ala Ser Glu Gly Cys Ser Asn Pro Leu
            180                 185                 190
Gly Gly Gly Arg Glu Val Trp Phe Gly Phe His Gln Ser Val Arg Pro
        195                 200                 205
Ser Leu Trp Lys Met Met Leu Asn Ile Asp Val Ser Ala Thr Ala Phe
    210                 215                 220
Tyr Lys Ala Gln Pro Val Ile Glu Phe Val Cys Glu Val Leu Asp Phe
225                 230                 235                 240
Lys Ser Ile Glu Glu Gln Gln Lys Pro Leu Thr Asp Ser Gln Arg Val
                245                 250                 255
Lys Phe Thr Lys Glu Ile Lys Gly Leu Lys Val Glu Ile Thr His Cys
            260                 265                 270
Gly Gln Met Lys Arg Lys Tyr Arg Val Cys Asn Val Thr Arg Arg Pro
        275                 280                 285
Ala Ser His Gln Thr Phe Pro Leu Gln Gln Glu Ser Gly Gln Thr Val
    290                 295                 300
Glu Cys Thr Val Ala Gln Tyr Phe Lys Asp Arg His Lys Leu Val Leu
305                 310                 315                 320
Arg Tyr Pro His Leu Pro Cys Leu Gln Val Gly Gln Glu Gln Lys His
                325                 330                 335
Thr Tyr Leu Pro Leu Glu Val Cys Asn Ile Val Ala Gly Gln Arg Cys
            340                 345                 350
Ile Lys Lys Leu Thr Asp Asn Gln Thr Ser Thr Met Ile Arg Ala Thr
        355                 360                 365
Ala Arg Ser Ala Pro Asp Arg Gln Glu Glu Ile Ser Lys Leu Met Arg
    370                 375                 380
Ser Ala Ser Phe Asn Thr Asp Pro Tyr Val Arg Glu Phe Gly Ile Met
385                 390                 395                 400
Val Lys Asp Glu Met Thr Asp Val Thr Gly Arg Val Leu Gln Pro Pro
                405                 410                 415
Ser Ile Leu Tyr Gly Gly Arg Asn Lys Ala Ile Ala Thr Pro Val Gln
            420                 425                 430
Gly Val Trp Asp Met Arg Asn Lys Gln Phe His Thr Gly Ile Glu Ile
        435                 440                 445
Lys Val Trp Ala Ile Ala Cys Phe Ala Pro Gln Arg Gln Cys Thr Glu
    450                 455                 460
Val His Leu Lys Ser Phe Thr Glu Gln Leu Arg Lys Ile Ser Arg Asp
465                 470                 475                 480
Ala Gly Met Pro Ile Gln Gly Gln Pro Cys Phe Cys Lys Tyr Ala Gln
                485                 490                 495
Gly Ala Asp Ser Val Glu Pro Met Phe Arg His Leu Lys Asn Thr Tyr
            500                 505                 510
Ala Gly Leu Gln Leu Val Val Val Ile Leu Pro Gly Lys Thr Pro Val
        515                 520                 525
Tyr Ala Glu Val Lys Arg Val Gly Asp Thr Val Leu Gly Met Ala Thr
    530                 535                 540
Gln Cys Val Gln Met Lys Asn Val Gln Arg Thr Thr Pro Gln Thr Leu
545                 550                 555                 560
Ser Asn Leu Cys Leu Lys Ile Asn Val Lys Leu Gly Gly Val Asn Asn
                565                 570                 575
Ile Leu Leu Pro Gln Gly Arg Pro Pro Val Phe Gln Gln Pro Val Ile
            580                 585                 590
Phe Leu Gly Ala Asp Val Thr His Pro Pro Ala Gly Asp Gly Lys Lys
        595                 600                 605
Pro Ser Ile Ala Ala Val Val Gly Ser Met Asp Ala His Pro Asn Arg

610                 615                 620
Tyr Cys Ala Thr Val Arg Val Gln Gln His Arg Gln Glu Ile Ile Gln
625                 630                 635                 640
Asp Leu Ala Ala Met Val Arg Glu Leu Leu Ile Gln Phe Tyr Lys Ser
                645                 650                 655
Thr Arg Phe Lys Pro Thr Arg Ile Ile Phe Tyr Arg Asp Gly Val Ser
            660                 665                 670
Glu Gly Gln Phe Gln Gln Val Leu His His Glu Leu Leu Ala Ile Arg
        675                 680                 685
Glu Ala Cys Ile Lys Leu Glu Lys Asp Tyr Gln Pro Gly Ile Thr Phe
    690                 695                 700
Ile Val Val Gln Lys Arg His His Thr Arg Leu Phe Cys Thr Asp Lys
705                 710                 715                 720
Asn Glu Arg Val Gly Lys Ser Gly Asn Ile Pro Ala Gly Thr Thr Val
                725                 730                 735
Asp Thr Lys Ile Thr His Pro Thr Glu Phe Asp Phe Tyr Leu Cys Ser
            740                 745                 750
His Ala Gly Ile Gln Gly Thr Ser Arg Pro Ser His Tyr His Val Leu
        755                 760                 765
Trp Asp Asp Asn Arg Phe Ser Ser Asp Glu Leu Gln Ile Leu Thr Tyr
    770                 775                 780
Gln Leu Cys His Thr Tyr Val Arg Cys Thr Arg Ser Val Ser Ile Pro
785                 790                 795                 800
Ala Pro Ala Tyr Tyr Ala His Leu Val Ala Phe Arg Ala Arg Tyr His
                805                 810                 815
Leu Val Asp Lys Glu His Asp Ser Ala Glu Gly Ser His Thr Ser Gly
            820                 825                 830
Gln Ser Asn Gly Arg Asp His Gln Ala Leu Ala Lys Ala Val Gln Val
        835                 840                 845
His Gln Asp Thr Leu Arg Thr Met Tyr Phe Ala
    850                 855

<210> 4
<211> 7
<212> PRT
<213> Artificial Sequence

<220>
<223> AVV displayed peptide

<220>
<221> VARIANT
<222> 2
<223> Ile

<400> 4
Ser Leu Arg Ser Pro Pro Ser
1               5

<210> 5
<211> 7
<212> PRT
<213> Artificial Sequence

<220>
<223> AVV displayed peptide

<400> 5
Asn Leu His Ser Pro Pro Ala
1               5

<210> 6
<211> 7

<212> PRT
<213> Artificial Sequence

<220>
<223> Nuclear localization sequence (NLS)

<400> 6
Pro Lys Lys Lys Arg Lys Val
1               5

<210> 7
<211> 10
<212> PRT
<213> Artificial Sequence

<220>
<223> Nuclear localization sequence (NLS)

<220>
<221> VARIANT
<222> 4,5,6,7,8
<223> Ala, Cys, Asp, Glu, Phe, Gly, His, Ile, Lys, Leu, Met, Asn, Pro,
      Gln, Arg, Ser, Thr, Val, Trp, Tyr

<400> 7
Lys Lys Lys Xaa Xaa Xaa Xaa Xaa Arg Lys
1               5                   10